# EUROPEAN PATENT APPLICATION

(11) **EP 4 144 362 A1**
(43) Date of publication of application: **08.03.2023**
(21) Application number: 21195161.1
(22) Date of filing: 06.09.2021
(51) Int. Cl.: A61K 38/22, A61K 38/26, A61P 3/04, A61P 3/10, A61P 25/28

(54) **COMPOSITIONS COMPRISING SHORT-ACTING HORMONES FOR TREATING OR PREVENTING OBESITY AND PUMPS COMPRISING SAID COMPOSITION**

(71) Applicant: Adocia, 69003 Lyon (FR)
(72) Inventor: The designation of the inventor has not yet been filed
(74) Representative: Tripoz, Inès

(57) **Abstract**

The invention relates to therapies for treating obesity and overweight.

The invention is about compositions comprising at least 2 short-acting peptides chosen in at least two of the following groups, GLP-1 receptor agonist (abbreviated RA), Amylin RA and Glucagon RA, schemes of administration of such compositions and devices comprising such a composition.

## Description

The invention relates to therapies for treating obesity and overweight.

The invention is about compositions comprising at least 2 short-acting peptides chosen in at least two of the following groups, GLP-1 receptor agonist (abbreviated RA), Amylin RA and Glucagon RA, schemes of administration of such compositions and devices comprising such a composition.

In recent times, obesity has spread rapidly in the world, with the US and China being the most affected population. Today, 650 million of adults (13% of adults) and about 124 million of children older than 5 are obese in the world. In the US, 42.8% of adults and 18.5% of children are obese.

It has major health implications that can cause premature death due the increased risk of developing several diseases including osteoarthritis, non-alcoholic fatty liver disease, type II diabetes, cerebrovascular disease, sleep apnea and asthma.

A weight loss as small as 5 % is thought to significantly reduce the incidence of at least some severe complications.

As seen above, obesity has a high prevalence worldwide and induces a high risk of associated disease. However, few treatment options are available, including pharmacotherapy.

To treat this disease, therapies have focused on reducing food intake and increasing energy expenditure in patients.

A first proposed therapy consists in a surgical solution for reducing the gastric volume. The gastric volume reduction procedures are generally based on the provision of vertical and/or horizontal gastroplasty in which sutures, staples or other fixations are used to join opposing surfaces of the stomach in order to create a reduced volume pouch and thereby reduce caloric intake.

However, such surgical solution is irreversible, strongly invasive, and may lead to heavy side effects and long-term risks.

A second proposed therapy consists in implantable devices.

One implantable solution is based on the provision of a gastric banding. Such banding creates an upper chamber above the band to produce a sensation of satiety after consuming only a small volume of food. However, the provision of gastric banding does not address all mechanisms associated with obesity, for example with regard to patients continuously eating small volumes of high caloric food or consuming high caloric liquid foods.

Another implantable solution is based on the placement of an intra-gastric balloon within the stomach. However, some studies showed modest weight loss, and often a diminution of the effects of these balloons after three to four weeks.

The most promising therapies relies on pharmacotherapy. Recently, use of long acting hormones have yielded viable options to induce weight loss in obese patients.

The use of hormones with long duration of action is a well known paradigm because the necessity of frequent injections reduces patients long term adherence to the treatment. Thus a treatment with long-acting drugs, in particular with once-weekly injections, is the only proposed way today to convince patients to take such a treatment and also to continue this treatment over time.

Hormone therapies in development today, such as Semaglutide and Cagrilintide (from Novo Nordisk), Tirzepatide (from Eli Lilly) or Glucagon-GLP-1 dual agonists (from Boehringer Ingelheim and Zealand), are some examples of this strategy.

Furthermore, clinical results published with these classes of hormones have shown a significant efficacy, leading to the development of more products of these classes.

However, these classes of hormones induce side effects such as nausea, vomiting or diarrheas.

These side effects are a major drawback as by design, long acting drugs remain in circulation for a long duration (e.g. more than a week) without the possibility to end their side effects quickly when they occur. In this case, the patient has no other choice than to wait for the drug concentration to drop below the level inducing unwanted side effects. Thus the side effect lasts for a long time, such as one or several days.

Another issue with this long acting strategy is that the patient cannot decide to take a short break for social events, such as a family meal, or a day off treatment to release pressure, which could contribute to a better acceptation of the treatment in the long term and limit drop-out rates.

Another drawback of these long acting hormone treatments is that they cannot be modulated with a daily titration schedule to optimize efficacy and tolerance. It is well known that the concentration of the long acting hormones changes during the week with a difference of more than 30% between the peak and valley. Concentrations may then be too low at times to induce the best effect and too high at other times to provide the best tolerance.

All these drawbacks are significantly limiting their long term treatment adoption by patients and doctors, as demonstrated by the significant percentage of patient drop-out, in particular due to side effects. For example it was reported that with a once-a-week long acting GLP-1 RA treatment, 46.7% of type 2 diabetic patients stopped after less then one year.

An aim of the present invention is to provide a composition, a therapeutical scheme and a device to help patients efficiently lose weight by giving them the possibility to control the dosing in order to remedy to at least one of the aforementioned drawbacks. More particularly, an aim of the present invention is to provide a device allowing a continuous delivery of the composition while limiting unease and discomfort sensations, thus improving compliance, and/or improving the performance of the treatment.

The invention proposes a way to solve at least part of the the problems caused by the current treatment of overweight, obesity and related disease. This goes against the path used by the main actors of the field as it involves short-acting active principles while the main players of the field believe that the best solution involves long-acting active principles.

The therapeutical scheme according to the invention offers to patients suffering from overweight, obesity or related disease a way to control the hormones side effects by a patient driven control of the treatment administration. Thus the patient can temporarily stop or diminish the delivery of their treatment until the side-effect(s) disappear(s) and then resume or increase the dose delivery of the treatment, without compromising the long-term efficacy of the treatment or inducing treatment discontinuation.

The therapeutical scheme comprising compositions according to the invention may lead to:
- a better control of undesirable side effects by the patient and improvement of the compliance,
- a better shaping of the treatment and a better efficiency per molecule used.

In summary, an aim of the invention is to offer patient:
- to confer the advantages of long-acting hormones in that it maintains an efficient plasmatic concentrations of the hormones over time, and
- to limit the undesirable side effects related to the use of long-acting hormones, as the side effects can be managed, controlled or even stopped, relatively quickly by the patients, and
- to decrease, or even stop, momentarily the treatment for a while allowing him to take a break, thus leading to an improved compliance in the long term.

Thus the invention allows the patient to self adjust his treatment, on the contrary to the current long acting hormones treatments where the patient is passive, and can only choose to discontinue their treatment or carry on as is.

These advantages may be reached with the use of a device, such as a simple pump, in particular a subcutaneous infusion pump, for example an insulin pump, with a composition as disclosed below.

The invention relates to a composition comprising at least two short-acting peptides chosen in at least two of the following groups, short-acting GLP-1 RA, short-acting amylin RA and short-acting glucagon RA.

It also relates to a composition comprising at least two short-acting peptides chosen in at least two of the following groups, short-acting GLP-1 RA, short-acting amylin RA and short-acting glucagon RA for use as a medicament.

It also relates to a composition comprising at least two short-acting peptides chosen in at least two of the following groups, short-acting GLP-1 RA, short-acting amylin RA and short-acting glucagon RA for use in a method of treating overweight, obesity, and related disease.

It also relates to a composition comprising at least two short-acting peptides chosen in at least two of the following groups, short-acting GLP-1 RA, short-acting amylin RA and short-acting glucagon RA for use in a method of preventing overweight, obesity, and related diseases.

By "preventing or treating overweight, obesity and related diseases" is meant preventing or treating obesity, NASH, NAFLD, overweight, reducing body weight and/or food intake, or inducing energy expenditure or satiety.

In another embodiment, the composition is for use in a method for preventing or treating neurodegenerative diseases.

In another embodiment, the composition is used for preventing or treating Type 2 Diabetes.

It further relates to a composition comprising at least two short-acting peptides chosen in at least two of the following groups, short-acting GLP-1 RA, short-acting amylin RA and short-acting glucagon RA for use in a method of preventing or treating overweight, obesity, and related diseases and/or neurodegenerative diseases and /or Type 2 Diabetes, characterised in that the composition is administered in a therapeutical scheme that comprises modulation of the delivered dose, such as increasing and decreasing the delivery, or even stop and restart periods.

It further relates to a composition comprising at least two short-acting peptides chosen in at least two of the following groups, short-acting GLP-1 RA, short-acting amylin RA and short-acting glucagon RA for use in a method of preventing or treating overweight, obesity, and related diseases and/or neurodegenerative diseases and /or Type 2 Diabetes, characterised in that the composition is administered in a therapeutical scheme that comprises modulation of the delivered dose, such as increasing and decreasing the delivery, or even stop and restart periods.

It further relates to a composition comprising at least two short-acting peptides chosen in at least two of the following groups, short-acting GLP-1 RA, short-acting amylin RA and short-acting glucagon RA for use in a method of preventing or treating overweight, obesity, and related diseases and/or neurodegenerative diseases and /or Type 2 Diabetes, characterised in that the composition is administered in a therapeutical scheme that further comprises modulation periods that allow a modulation of the daily dose, for example from day to day.

It further relates to a composition comprising at least two short-acting peptides chosen in at least two of the following groups, short-acting GLP-1 RA, short-acting amylin RA and short-acting glucagon RA, for use in a method of preventing or treating overweight, obesity, and related diseases and/or neurodegenerative diseases and /or Type 2 Diabetes, characterised in that the composition is administered in a therapeutical scheme that further comprises modulation periods that allow a modulation of the rate of delivery intraday.

By intraday is meant a period going from 0h00 to 24h00.

It further relates to a composition comprising at least two short-acting peptides chosen in at least two of the following groups, short-acting GLP-1 RA, short-acting amylin RA and short-acting glucagon RA for use in a method of preventing or treating overweight, obesity, and related diseases and/or neurodegenerative diseases and /or Type 2 Diabetes, characterised in that the composition is administered in a therapeutical scheme that further comprises modulation periods that allow a decrease of the daily dose until it is temporarily discontinued.

By "temporarily" is meant periods such as 1h, 2h, 3h, 6h, 8h, 12h, 24h, 2 days, 4 days or even one week, 2 weeks or a month.

It further relates to a composition comprising at least two short-acting peptides chosen in at least two of the following groups, GLP-1 RA, amylin RA and Glucagon RA for use in a method of preventing or treating overweight, obesity, and related diseases and/or neurodegenerative diseases and /or Type 2 Diabetes, characterised in that the composition is administered in a therapeutical scheme that comprises stop and restart times.

Stop and restart time may be the same as defined for the term "temporarily".

It further relates to a composition comprising at least two short-acting peptides chosen in at least two of the following groups, GLP-1 RA, amylin RA and Glucagon RA for use in a method of preventing or treating overweight, obesity, and related diseases and/or neurodegenerative diseases and /or Type 2 Diabetes, characterised in that the composition is administered in a therapeutical scheme that comprises modulation of the delivered dose.

It further relates to a composition comprising at least two short-acting peptides chosen in at least two of the following groups, GLP-1 RA, amylin RA and Glucagon RA for use in a method of preventing or treating overweight, obesity, and related diseases and/or neurodegenerative diseases and /or Type 2 Diabetes, characterised in that the composition is administered in a therapeutical scheme that comprises modulation of the delivered dose during the day without changing the total daily dose.

It further relates to a composition comprising at least two short-acting peptides chosen in at least two of the following groups, GLP-1 RA, amylin RA and Glucagon RA for use in a method of preventing or treating overweight, obesity, and related diseases and/or neurodegenerative diseases and /or Type 2 Diabetes, characterised in that the molar ratio of short-acting GLP-1 RA and short-acting glucagon RA in the composition is such that it leads to a molar ratio of short-acting GLP-1 RA and short-acting glucagon RA at steady state is comprised betweeen 0.1 to 15.

It also relates to a composition comprising at least two short-acting peptides chosen in at least two of the following groups, GLP-1 RA, amylin RA and Glucagon RA for use in a method of preventing or treating overweight, obesity, and related diseases and/or neurodegenerative diseases and /or Type 2 Diabetes, characterised in that amylin RA is administered at a daily dose comprised from 100 to 3000 µg/day.

It also relates to a composition comprising at least two short-acting peptides chosen in at least two of the following groups, GLP-1 RA, amylin RA and Glucagon RA for use in a method of preventing or treating overweight, obesity, and related diseases and/or neurodegenerative diseases and /or Type 2 Diabetes, characterised in that amylin RA is administered at a daily dose comprised from 150 to 2000 µg/day.

It also relates to a composition comprising at least two short-acting peptides chosen in at least two of the following groups, GLP-1 RA, amylin RA and Glucagon RA for use in a method of preventing or treating overweight, obesity, and related diseases and/or neurodegenerative diseases and /or Type 2 Diabetes, characterised in that amylin RA is administered at a daily dose comprised from 500 to 2000 µg/day.

It also relates to a composition comprising at least two short-acting peptides chosen in at least two of the following groups, GLP-1 RA, amylin RA and Glucagon RA for use in a method of preventing or treating overweight, obesity, and related diseases and/or neurodegenerative diseases and /or Type 2 Diabetes, characterised in that amylin RA is pramlintide administered at a daily dose comprised from 150 to 2000 µg/day.

It also relates to a composition comprising at least two short-acting peptides chosen in at least two of the following groups, GLP-1 RA, amylin RA and Glucagon RA for use in a method of preventing or treating overweight, obesity, and related diseases and/or neurodegenerative diseases and /or Type 2 Diabetes, characterised in that amylin RA is pramlintide administered at a daily dose comprised from 500 to 2000 µg/day.

It also relates to a composition comprising at least two short-acting peptides chosen in at least two of the following groups, GLP-1 RA, amylin RA and Glucagon RA for use in a method of preventing or treating overweight, obesity, and related diseases and/or neurodegenerative diseases and /or Type 2 Diabetes, characterised in that GLP-1 RA is exenatide administered at a daily dose comprised from 10 to 250 µg/day.

It also relates to a composition comprising at least two short-acting peptides chosen in at least two of the following groups, GLP-1 RA, amylin RA and Glucagon RA for use in a method of preventing or treating overweight, obesity, and related diseases and/or neurodegenerative diseases and /or Type 2 Diabetes, characterised in that GLP-1 RA is exenatide administered at a daily dose comprised from 60 to 180 µg/day.

It also relates to a composition comprising at least two short-acting peptides chosen in at least two of the following groups, GLP-1 RA, amylin RA and Glucagon RA for use in a method of preventing or treating overweight, obesity, and related diseases and/or neurodegenerative diseases and /or Type 2 Diabetes, characterised in that GLP-1 RA is lixisenatide administered at a daily dose comprised from 20 to 600 µg/day.

It also relates to a composition comprising at least two short-acting peptides chosen in at least two of the following groups, GLP-1 RA, amylin RA and Glucagon RA for use in a method of preventing or treating overweight, obesity, and related diseases and/or neurodegenerative diseases and /or Type 2 Diabetes, characterised in that GLP-1 RA is lixisenatide administered at a daily dose comprised from 120 to 360 µg/day.

It also relates to a composition comprising at least two short-acting peptides chosen in at least two of the following groups, GLP-1 RA, amylin RA and Glucagon RA for use in a method of preventing or treating overweight, obesity, and related diseases and/or neurodegenerative diseases and /or Type 2 Diabetes, characterised in that Glucagon RA is human glucagon administered at a daily dose of above 500 µg.

It also relates to a composition comprising at least two short-acting peptides chosen in at least two of the following groups, GLP-1 RA, amylin RA and Glucagon RA for use in a method of preventing or treating overweight, obesity, and related diseases and/or neurodegenerative diseases and /or Type 2 Diabetes, characterised in in that Glucagon RA is dasiglucagon administered at a daily dose of above 250 µg.

The invention also relates to a method of preventing or treating overweight, obesity, and related diseases and/or neurodegenerative diseases and /or Type 2 Diabetes, wherein a composition comprising at least two short-acting peptides chosen in at least two of the following groups, short-acting GLP-1 RA, short-acting amylin RA and short-acting glucagon RA is administered in a therapeutical scheme that comprises modulation of the delivered dose, such as increasing and decreasing the delivery, or even stop and restart periods.

It further relates to a method of preventing or treating overweight, obesity, and related diseases and/or neurodegenerative diseases and /or Type 2 Diabetes, wherein a composition comprising at least two short-acting peptides chosen in at least two of the following groups, short-acting GLP-1 RA, short-acting amylin RA and short-acting glucagon RA is administered in a therapeutical scheme that comprises modulation periods that allow a modulation of the daily dose, for example from day to day.

It further relates to a method of preventing or treating overweight, obesity, and related diseases and/or neurodegenerative diseases and /or Type 2 Diabetes, wherein a composition comprising at least two short-acting peptides chosen in at least two of the following groups, short-acting GLP-1 RA, short-acting amylin RA and short-acting glucagon RA, is administered in a therapeutical scheme that further comprises modulation periods that allow a modulation of the rate of delivery intraday.

By intraday is meant a period going from 0h00 to 24h00.

It further relates to a method of preventing or treating overweight, obesity, and related diseases and/or neurodegenerative diseases and /or Type 2 Diabetes, wherein a composition comprising at least two short-acting peptides chosen in at least two of the following groups, short-acting GLP-1 RA, short-acting amylin RA and short-acting glucagon RA is administered in a therapeutical scheme comprising modulation periods that allow a decrease of the daily dose until it is temporarily discontinued.

It further relates to a method of preventing or treating overweight, obesity, and related diseases and/or neurodegenerative diseases and /or Type 2 Diabetes, wherein a composition comprising at least two short-acting peptides chosen in at least two of the following groups, short-acting GLP-1 RA, short-acting amylin RA and short-acting glucagon RA is administered in a therapeutical scheme comprising stop and restart periods.

It further relates to a method of preventing or treating overweight, obesity, and related diseases and/or neurodegenerative diseases and /or Type 2 Diabetes,wherein a composition comprising at least two short-acting peptides chosen in at least two of the following groups, short-acting GLP-1 RA, short-acting amylin RA and short-acting glucagon RA is administered in a therapeutical scheme comprising modulation of the delivered dose.

It further relates to a method of preventing or treating overweight, obesity, and related diseases and/or neurodegenerative diseases and /or Type 2 Diabetes, wherein a composition comprising at least two short-acting peptides chosen in at least two of the following groups, short-acting GLP-1 RA, short-acting amylin RA and short-acting glucagon RA is administered in a therapeutical scheme comprising modulation of the delivered dose during the day without changing the total daily dose.

It further relates to a method of preventing or treating overweight, obesity, and related diseases and/or neurodegenerative diseases and /or Type 2 Diabetes, wherein a composition comprising at least two short-acting peptides chosen in at least two of the following groups, short-acting GLP-1 RA, short-acting amylin RA and short-acting glucagon RA, the molar ratio of short-acting GLP-1 RA and short-acting glucagon RA in the composition being such that it leads to a molar ratio of short-acting GLP-1 RA and short-acting glucagon RA at steady state is comprised betweeen 0.1 to 15.

It also relates to a method of preventing or treating overweight, obesity, and related diseases and/or neurodegenerative diseases and /or Type 2 Diabetes, wherein a composition comprising at least two short-acting peptides chosen in at least two of the following groups, short-acting GLP-1 RA, short-acting amylin RA and short-acting glucagon RA, characterised in that amylin RA is administered at a daily dose comprised from 100 to 3000 µg/day.

It also relates to a method of preventing or treating overweight, obesity, and related diseases and/or neurodegenerative diseases and /or Type 2 Diabetes, wherein a composition comprising at least two short-acting peptides chosen in at least two of the following groups, short-acting GLP-1 RA, short-acting amylin RA and short-acting glucagon RA, characterised in that amylin RA is administered at a daily dose comprised from 150 to 2000 µg/day.

It also relates to a method of preventing or treating overweight, obesity, and related diseases and/or neurodegenerative diseases and /or Type 2 Diabetes, wherein a composition comprising at least two short-acting peptides chosen in at least two of the following groups, short-acting GLP-1 RA, short-acting amylin RA and short-acting glucagon RA, characterised in that amylin RA is administered at a daily dose comprised from 500 to 2000 µg/day.

It also relates to a method of preventing or treating overweight, obesity, and related diseases and/or neurodegenerative diseases and /or Type 2 Diabetes, wherein a composition comprising at least two short-acting peptides chosen in at least two of the following groups, short-acting GLP-1 RA, short-acting amylin RA and short-acting glucagon RA, characterised in that amylin RA is pramlintide administered at a daily dose comprised from 150 to 2000 µg/day.

It also relates to a method of preventing or treating overweight, obesity, and related diseases and/or neurodegenerative diseases and /or Type 2 Diabetes, wherein a composition comprising at least two short-acting peptides chosen in at least two of the following groups, short-acting GLP-1 RA, short-acting amylin RA and short-acting glucagon RA, characterised in that amylin RA is pramlintide administered at a daily dose comprised from 500 to 2000 µg/day.

It also relates to a method of preventing or treating overweight, obesity, and related diseases and/or neurodegenerative diseases and /or Type 2 Diabetes, wherein a composition comprising at least two short-acting peptides chosen in at least two of the following groups, short-acting GLP-1 RA, short-acting amylin RA and short-acting glucagon RA, characterised in that GLP-1 RA is exenatide administered at a daily dose comprised from 10 to 250 µg/day.

It also relates to a method of preventing or treating overweight, obesity, and related diseases and/or neurodegenerative diseases and /or Type 2 Diabetes, wherein a composition comprising at least two short-acting peptides chosen in at least two of the following groups, short-acting GLP-1 RA, short-acting amylin RA and short-acting glucagon RA, characterised in that GLP-1 RA is exenatide administered at a daily dose comprised from 60 to 180 µg/day.

It also relates to a method of preventing or treating overweight, obesity, and related diseases and/or neurodegenerative diseases and /or Type 2 Diabetes, wherein a composition comprising at least two short-acting peptides chosen in at least two of the following groups, short-acting GLP-1 RA, short-acting amylin RA and short-acting glucagon RA, characterised in that GLP-1 RA is lixisenatide administered at a daily dose comprised from 20 to 600 µg/day.

It also relates to a method of preventing or treating overweight, obesity, and related diseases and/or neurodegenerative diseases and /or Type 2 Diabetes, wherein a composition comprising at least two short-acting peptides chosen in at least two of the following groups, short-acting GLP-1 RA, short-acting amylin RA and short-acting glucagon RA, characterised in that GLP-1 RA is lixisenatide administered at a daily dose comprised from 120 to 360 µg/day.

It also relates to a method of preventing or treating overweight, obesity, and related diseases and/or neurodegenerative diseases and /or Type 2 Diabetes, wherein a composition comprising at least two short-acting peptides chosen in at least two of the following groups, short-acting GLP-1 RA, short-acting amylin RA and short-acting glucagon RA, characterised in that Glucagon RA is human glucagon administered at a daily dose of above 500 µg.

It also relates to a method of treating overweight, obesity, and related disease, wherein a composition comprising at least two short-acting peptides chosen in at least two of the following groups, short-acting GLP-1 RA, short-acting amylin RA and short-acting glucagon RA, characterised in in that Glucagon RA is dasiglucagon administered at a daily dose of above 250 µg.

The invention also relates to a device comprising:
- a composition comprising at least two short-acting peptides chosen in at least two of the following groups, short-acting GLP-1 RA, short-acting Amylin RA and short-acting Glucagon RA,
- an administering apparatus for delivering the composition to a patient, said administering apparatus including:
- a reservoir containing the composition,
- a drive mechanism for pumping the composition contained in the reservoir,
- a control unit (24) for controlling the drive mechanism according to an injection profile defining at least one rate for the administration of the composition to the patient,
- an injection set configured to be attached to an infusion site on the patient for the subcutaneous administering of the composition to the patient.

According to an embodiment the device allows the delivery of the composition according to the schemes of administration as defined in the instant specification.

The invention also relates to a composition for use for prevention or treatment of overweight, obesity, and related diseases.

The invention also relates to a device comprising a composition for use for prevention or treatment of overweight, obesity, and related diseases.

The invention also relates to a method for prevention or treatment of overweight, obesity, and related disease.

The invention relates to an administering device , said device comprises:
- a composition comprising at least two short-acting peptides chosen in at least two of the following groups, GLP-1 RA, Amylin RA and Glucagon RA, for use as a medicament.
- an administering apparatus for delivering the composition to a patient, said administering apparatus including:
- a reservoir containing the composition,
- a drive mechanism for pumping the composition contained in the reservoir,
- a control unit for controlling the drive mechanism according to an injection profile defining at least one rate for the administering of the composition to the patient,
- an injection set configured to be attached to an infusion site on the patient for the subcutaneous administering of the composition to the patient

Preferred, but non-limiting, aspects of the administering device according to the invention are the following:
- device wherein the administering apparatus further comprises an actuator, such as a button, for deactivating the administering apparatus for a predetermined period of time.
- device which further comprises a supervisory terminal for controlling the administering apparatus, said supervisory terminal being configured for adjusting the injection profile according to at least one parameter associated to the patient.
- device wherein the supervisory terminal includes a Graphical User Interface (GUI) for the input by the patient of the at least one parameter associated to the patient.
- device wherein the at least one parameter associated to the patient includes a level of comfort representative of a sensation of nausea felt by the patient, the supervisory terminal being configured for decreasing the at least one rate of the injection profile if said level of comfort does not satisfy a comfort threshold.
- device wherein the at least one parameter associated to the patient includes a level of activity representing whether the patient is:
   ∘ in an active state or
   ∘ in a passive state,
   the supervisory terminal being configured for decreasing the at least one rate of the injection profile if said level of activity corresponds to a passive state of the patient.
- device wherein the administering apparatus includes at least one sensor, such as a gyroscope and/or an accelerometer, for measuring signals representative of motions of the patient and/or of the orientation of the patient, said signals being transmitted to the control unit and/or the supervisory terminal for detecting whether the patient is in an active state or in a passive state.
- device wherein the at least one parameter associated to the patient includes an information representative of an event occurring in the life of the patient, the supervisory terminal being configured for decreasing the at least one rate of the injection profile for a duration corresponding to the duration of said event.

The present invention may be more completely understood in consideration of the following detailed description of various embodiments in connection with the accompanying drawings, in which:
- Figure 1 is a schematic perspective view of a device for administering composition 1,
- Figure 2 is a block diagram representing an example of an administering apparatus of the device,
- Figure 3 is a schematic view illustrating an example of injection profile for the administration of a composition using the administering apparatus,
- Figure 4 is a schematic view illustrating another example of injection profile for the administration of the composition using the administering apparatus.

Different examples of the device according to the invention will now be described with reference to the figures. In these different figures, equivalent elements are designated by the same numerical reference.

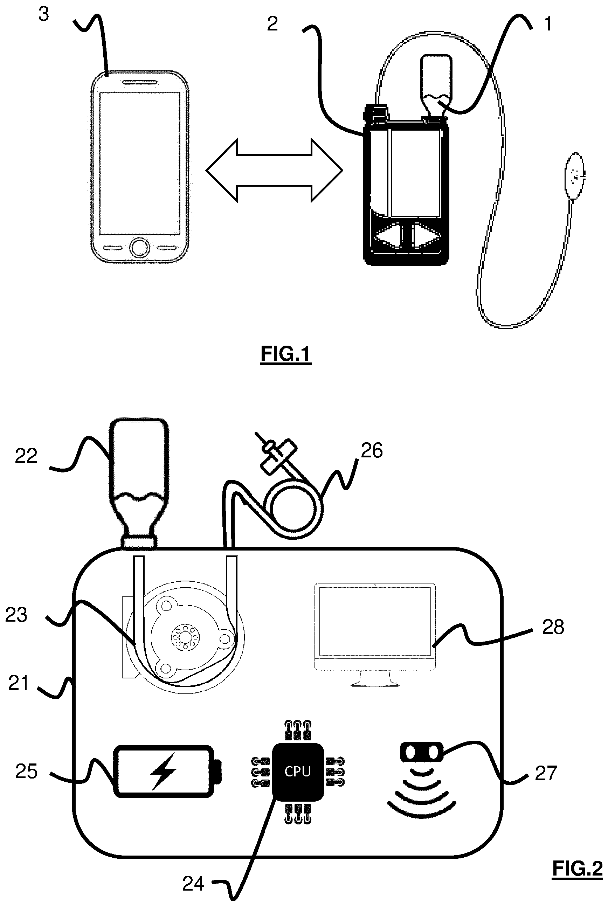

Referring to figure 1, the device comprises:
- a composition 1 such as disclosed above,
- an administering apparatus 2 for delivering the composition 1 to a patient, and
- a supervisory terminal 3 for controlling the administering apparatus 2.

According to an embodiment, the device is configured for a subcutaneous delivery of the composition 1 to a patient.

Preferably, the composition 1 is administered regularly or continuously under the skin of the patient according to an injection profile which can be predetermined, or can be adjustable according to user parameters, as will be described in more details below.

As disclosed in the following description, the device for use in a method for preventing or treating overweight, obesity, and related diseases offers the advantages of:
- continuous administration of composition 1,
- precise dosing, and
- programmable delivery schedules.

This results in a closer control and an improved feeling of wellness.

In particular, the device for use in a method for preventing or treating overweight, obesity, and related diseases allows administering an amount of composition 1 that is:
- above an efficiency threshold and
- below a discomfort threshold.

This provides the patient with an efficient administration while limiting discomfort sensations.

More precisely the invention relates to a composition comprising two or three short-acting peptides, each chosen from a different group as defined above.

In an embodiment the composition comprises only one compound per group.

In an embodiment the composition does not comprise any other API.

By "active pharmaceutical ingredient" or "API", is meant any substance or combination of substances used in a finished pharmaceutical product (FPP), intended to furnish pharmacological activity or to otherwise have direct effect in the diagnosis, cure, mitigation, treatment or prevention of disease, or to have direct effect in restoring, correcting or modifying physiological functions in human beings."

As used herein, the term "short-acting peptide" refers to a hormone or a peptide that has an apparent half-life of elimination after subcutaneous injection in humans of at least less than 8 hours, in particular less than 5 hours, preferably less than 4 hours or even less than 3 hours.

As used herein, the term "long-acting peptide" refers to a peptide that has an apparent half-life of elimination after subcutaneous injection in humans of more than 8 hours.

As used herein, "receptor agonist" means a compound, such as a synthetic peptide, that activates a target receptor and elicits at least one in vivo or in vitro effect elicited by a native agonist for that receptor.

GLP-1 receptor agonist (GLP-1 RA), also known as incretin mimetics, are insulin secretagogues. Among short-acting GLP-1 RA can be cited exenatide and lixisenatide, which are also known for diminishing the food intake and for slowing down the gastric emptying.

Throughout the present specification "natural amino acid" is an amino acid (with the usual three letter codes & one letter codes in parenthesis) selected from the group consisting of: Glycine (Gly & G), proline (Pro & P), alanine (Ala & A), valine (Val & V), leucine (Leu & L), isoleucine (Ile & I), methionine (Met & M), cysteine (Cys & C), phenylalanine (Phe & F), tyrosine (Tyr & Y ), tryptophan (Trp & W), histidine (His & H), lysine (Lys & K), arginine (Arg & R), glutamine (Gln & Q), asparagine (Asn & N), glutamic acid (Glu & E), aspartic acid (Asp & D), serine (Ser & S) and threonine (Thr & T). If, due to typing errors, there are deviations from the commonly used codes, the commonly used codes apply. The amino acids present in the polypeptides of the present invention are, preferably, amino acids which can be coded for by a nucleic acid.

Less common or non-naturally occurring amino acids may be used, unless they are referred to by their full name (e.g. sarcosine, ornithine, etc.), the three- or four-character codes may be employed for residues thereof, including Orn (ornithine, i.e. 2,5-diaminopentanoic acid), Aib (a-aminoisobutyric acid), Dab (2,4-diaminobutanoic acid), Dap (2,3-diaminopropanoic acid), Har (homoarginine), γ-Glu (γ-glutamic acid), Gaba (γ-aminobutanoic acid), β-Ala (i.e. 3-aminopropanoic acid) and 8Ado (8-amino-3,6- dioxaoctanoic acid).

The term "basic amino acid" as employed in the context of the present invention refers to an amino acid containing one or more additional basic groups. Examples thereof include Lys, DLys, Orn, DOrn, Dap, DDap, Dab, DDab, His, DHis, Arg and DArg. Further examples thereof include guanidino derivatives of such basic amino acids, e.g. 2-amino-4-guanidino-1-butyric acid (Guanidino analog of L-Dab) or 2-amino-3-guanidino-1 -propionic acid (Guanidino analog of L-Dap).

Among other than natural amino acids may be cited:
- Ala(Me): N-methylalanine,
- Gly(Me): N-methylglycine, also called sarcosine (Sar),
- Ile(Me): N-methylisoleucine,
- Ser(Me): N-methylserine,
- Tyr(Me): N-methyltyrosine,
- Apr: 4-aminoproline, e.g. (2S,4R)-4-aminoproline, also called (4R)-4-amino-L-proline,
- Aze: azetidine-2-carboxylic acid, e.g. (2S)-azetidine-2-carboxylic acid,
- Bep: 4-benzylproline, e.g. (2S,4R)-4-benzylproline, also called (4R)-4-benzyl-L-proline,
- Hyp: 4-hydroxyproline, e.g. (2S,4R)-4-hydroxyproline, also called (4R)-4-hydroxyL-proline,
- Php: 4-phenylproline, e.g. (2S,4S)-4-phenylproline, also called (4S)-4-phenyl-L-proline
- Pip: (2S)-piperidine-2-carboxylic acid, also called (S)-pipecolic acid,
- Aad: 2-aminoadipic acid, e.g. (2S)-2-aminoadipic acid [also (2S)-2-aminohexanedioic,
- hLys: 2-amino-7-amino-heptanoic acid, also known as homo-lysine, e.g. (2S)-2-amino-7-amino-heptanoic acid.

As used herein,"4Pal" means 3-(4- Pyridyl)-L- alanine.

As used herein,"aMeF(2F)" means alpha-methyl 2-fluoro-L- phenylalanine.

As used herein,"aMeY" and"aMeL" mean alpha-methyl-L-tyrosine and alpha-methyl-L- leucine, respectively.

As used herein,"e" and"D-Glu" mean D- glutamic acid.

As used herein,"D-Tyr"and"y" each mean D-tyrosine.

As used herein, "D-Ala" and"a" each mean D-alanine.

As used herein,"aMeF" means alpha-methyl-F and alpha-methyl-Phe.

As used herein"Iva" means L-isovaline.

As used herein," Ac3c" means 1-Aminocyclopropanecarboxylic acid.

As used herein, "Ac4c" means 1-Amino-1-cyclobutane carboxylic acid.

As used herein, "Ac5c" means 1-Amino-1-cyclopentane carboxylic acid.

Unless otherwise indicated, reference is made to the L-isomeric forms of the amino acids in question. Where appropriate, the D-isomeric form of an amino acid is indicated in the conventional manner by the prefix "D" before the conventional three-letter code (e.g. DAsp, DPhe). In some case the d isoform of Glu is written "e" in the sequence.

If the amylin RA contains either more than 37 amino acid residues or less than 37 amino acid residues then the skilled person can still align that sequence with the sequence of pramlintide (SEQ ID NO: 2) to determine the placement number of the corresponding, respective amino acid residue. A suitable alignment program is "needle", which is a Needleman-Wunsch alignment. The algorithm for this alignment program is described in Needleman, S.B. and Wunsch, CD., (1970), Journal of Molecular Biology, 48: 443-453.

In the numbering sequence of SEQ ID NO: X, and according to established practice in the art, the amino acid residue at the N-terminal is assigned no. 1 and subsequent amino acid residues are numbered consecutively, ending at the C-terminal.

In an embodiment, the short-acting peptide is chosen among short-acting GLP-1 RA, short-acting amylin RA or short-acting glucagon RA.

In an embodiment the composition comprises one short-acting GLP-1 RA and one short-acting amylin RA.

In an embodiment the composition comprises one short-acting GLP-1 RA and one short-acting glucagon RA.

In an embodiment the composition comprises one short-acting amylin RA and one short-acting glucagon RA.

In an embodiment the composition comprises one short-acting GLP-1 RA, one short-acting amylin RA and one short-acting glucagon RA.

Among short-acting amylin RA can be cited pramlintide and salmon calcitonin.

Among short-acting glucagon RA can be cited human glucagon and dasiglucagon.

In an embodiment, the composition comprises at least one short-acting amylin receptor agonist.

By "short-acting" amylin RA is meant an amylin RA that has an apparent half-life of elimination after subcutaneous injection in humans of less than 3 hours, in particular less than 2 hours, preferably less than 1.5 hours or even less than 1 hour.

An agonist of the amylin receptor, or amylin RA, refers to a compound which imitates one or more characteristics of the activity of amylin.

Amylin derivatives are described in the article Yan et al., PNAS, vol. 103, no. 7, p. 2046-2051, 2006.

Native human amylin is a 37-amino acid peptide having the formula Hy-KC()NTATC()ATQRLANFLVHSSNNFGAILSSTNVGSNTY-NH₂ (SEQ ID NO: 1) wherein Hy- at the N-terminus designates a hydrogen atom, corresponding to the presence of a free amino group on the N-terminal amino acid residue [i.e. the lysine (K) residue at sequence position number 1 in SEQ ID NO: 1]; wherein -NH₂ at the C-terminus indicates that the C-terminal carboxyl group is in the amide form; and wherein the parentheses () associated with the two cysteine (C, Cys) residues at sequence positions 2 and 7 indicate the presence of an intramolecular disulfide bridge between the two Cys residues in question.

In an embodiment, the composition is characterized in that the amylin RA is an amylin analog.

As used herein, "analog" means a compound, such as a synthetic peptide, that activates a target receptor and elicits at least one in vivo or in vitro effect elicited by a native agonist for that receptor.

In an embodiment, the amylin RA is chosen in the group consisting of pramlintide and salmon calcitonin.

In an embodiment, the composition is characterized in that the amylin RA is pramlintide (Symlin^{®}) marketed by the company ASTRAZENECA AB.

Pramlintide is an amylin receptor agonist and amylin analog which has been developed by the company Amylin to compensate the lack of physical stability of human amylin. This product, marketed under the name of Symlin^{®}, was approved in 2005 by the FDA for the treatment of type 1 and type 2 diabetes, as a complement to insulin therapy.

Pramlintide is a peptide having the sequence: KCNTATCATQRLANFLVHSSNNFGPILPPTNVGSNTY (SEQ ID n°2)

According to the invention, the short acting amylin RA can also be chosen amongst the peptides according to Formula 1:

**R₁-Z-R₂** Formula 1

wherein
- R₁ is chosen in the group consisting of hydrogen, C₁₋₄ acyl, benzoyl or C₁₋₄ alkyl;
- R₂ is OH or NHR₃, wherein R₃ is hydrogen or C₁₋₃-alkyl; and
- Z is an amino acid sequence according to formula A1 or B1 or C1 or D1 or E1, and, Formula A1 is the following amino acid sequence as set forth in (SEQ ID NO:3): Xaa1-Cys-Xaa3-Thr-Ala-Thr-Cys-Ala-Thr-Gln-Arg-Leu-Ala-Xaa14-Phe-Leu-Xaa17-Xaa18-Ser-Ser-Xaa21-Xaa22-Phe-Gly-Xaa25-Xaa26-Xaa27-Xaa28-Xaa29-Thr-Xaa31-Val-Gly-Xaa34-Xaa35-Thr-Xaa37 (SEQ ID NO:3) wherein Xaa represents an amino acid and
   wherein
- Xaa1 is absent or independently selected from Ala, Cys, Glu, Gly, His, Arg, Ser and Lys;
- Xaa3 is independently selected from Gly, His, Arg, Ser and Asn;
- Xaa14 is independently selected from Asp, Glu, His, Asn, Arg, Gly, Ala, Ser, Lys, Thr and Cys;
- Xaa17 is independently selected from His, Arg, Lys and Val;
- Xaa18 is independently selected from Arg, Lys and His;
- Xaa21 is independently selected from Ala, Lys, Gln, Ser, Asn and Pro;
- Xaa22 is independently selected from Glu, Gln, Ser, Thr and Asn;
- Xaa25 is independently selected from Pro and Ala;
- Xaa26 is independently selected from Pro, Arg and Ile;
- Xaa27 is independently selected from Pro and Leu;
- Xaa28 is independently selected from Pro and Ser;
- Xaa29 is independently selected from Pro and Ser;
- Xaa31 is independently selected from Ser, Glu, Asp, Pro and Asn;
- Xaa34 is independently selected from Pro, His, Lys, Arg and Ser;
- Xaa35 is independently selected from His, Arg, Lys, Asp, Glu, Gln and Asn;
- Xaa37 is independently selected from Pro and Tyr;
- and
   Formula B1 is the following amino acid sequence as set forth in (SEQ ID NO:4): X(-1)-X1-X2-X3-X4-X5-X6-X7-X8-X9-X10-X11-X12-X13-X14-X15-X16-X17-X18-X19-X20-X21-X22-X23-X24-X25-X26-X27-X28-X29-X30-X31-X32-X33-X34-X35-X36 (SEQ ID NO: 4)
wherein X represents an amino acid and
wherein
- X(-1) is absent or E,
- X1 is absent or selected from the group consisting of E or A,
- X2 is selected from the group consisting of L, A or P,
- X3 is selected from the group consisting of S or P,
- X4 is selected from the group consisting of E, P, K, Q or G,
- X5 is selected from the group consisting of L, V, or I,
- X6 is S, T or H,
- X7 is T,
- X8 is selected from the group consisting of L or A,
- X9 is selected from the group consisting of A, V, I, S or T,
- X10 is selected from the group consisting of L, A, I, H or V,
- X11 is G,
- X12 is selected from the group consisting of R, H or K,
- X13 is L,
- X14 is selected from the group consisting of S, T or E,
- X15 is selected from the group consisting of A, Q, E, e or T,
- X16 is selected from the group consisting of R, E, K or Q,
- X17 is selected from the group consisting of L or I,
- X18 is selected from the group consisting of H or A,
- X19 is selected from the group consisting of E, R or K,
- X20 is selected from the group consisting of L, I or V,
- X21 is selected from the group consisting of A, Q, S, E or T,
- X22 is T,
- X23 is selected from the group consisting of T, Y or L,
- X24 is P,
- X25 is selected from the group consisting of R, P, H or K,
- X26 is T,
- X27 is selected from the group consisting of E, Q, G or K,
- X28 is selected from the group consisting of T or P,
- X29 is G,
- X30 is selected from the group consisting of P, S or T,
- X31 is selected from the group consisting of E, Q, G, A, P or K,
- X32 is selected from the group consisting of T, S, H, P or A,
- X33 is selected from the group consisting of P, Y, H, F, L, S, G or A,
- X34 is absent or G,
- X35 is absent or T, and
- X36 is absent or Y;
and
Formula C1 is the following amino acid sequence as set forth in (SEQ ID NO:5): X0-X1-Cys()-X3-Thr-Ala-Thr-Cys()-Ala-Thr-X10-Arg-X12-Ala-X14-Phe-X16-X17-X18-X19-X20-X21-X22-X23-X24-X25-X26-X27-X28-X29-X30-X31-Val-Gly-Ser-X35-Thr-X37 (SEQ ID NO: 5) wherein X represents an amino acid and wherein
- X0 is a basic amino acid or is absent;
- X1 is a basic amino acid or is selected from the group consisting of Ala and Trp;
- X3 is selected from the group consisting of Asn, Gly, Ser, Pro, Asp and Glu;
- X10 is selected from the group consisting of Gln, Gly, Ala, Ser, Leu, Thr, Pro, Asp and Glu;
- X12 is selected from the group consisting of Leu and DLeu;
- X14 is selected from the group consisting of Glu, DGlu, Asp and DAsp;
- X16 is selected from the group consisting of Leu and DLeu, or is absent;
- X17 is selected from the group consisting of His, DHis, Val, DVal, Arg, DArg, Lys, DLys, Orn, DOrn, Dap and Dab, or is absent;
- X18 is selected from the group consisting of His, DHis, Arg, DArg, Dap and Dab, or is absent;
- X19 is selected from the group consisting of Ser and DSer, or is absent;
- X20 is selected from the group consisting of Ser and DSer, or is absent;
- X21 is selected from the group consisting of Asn, DAsn, Glu, DGlu, Asp, DAsp, Ser and DSer, or is absent;
- X22 is selected from the group consisting of Asn, DAsn, Glu, DGlu, Asp, DAsp, Ser and DSer, or is absent;
- X23 is selected from the group consisting of Phe and DPhe, or is absent;
- X24 is selected from the group consisting of Gly(Me), Ala(Me), DAla(Me), Ile(Me), DIle(Me), Ala, DAla, Pro and DPro, or is absent;
- X25 is selected from the group consisting of Ala, DAla, Pro and DPro, or is absent;
- X26 is selected from the group consisting of Gly(Me), Ile(Me), DIle(Me), Ile and DIle, or is absent;
- X27 is selected from the group consisting of Leu and DLeu, or is absent;
- X28 is selected from the group consisting of Ser, DSer, Pro, DPro, Ser(Me) and DSer(Me), or is absent;
- X29 is selected from the group consisting of Ser, DSer, Pro, DPro, Ser(Me) and DSer(Me), or is absent;
- X30 is selected from the group consisting of Thr and DThr, or is absent;
- X31 is selected from the group consisting of Asn, DAsn, Glu, DGlu, Asp and DAsp, or is absent;
- X35 is selected from the group consisting of His, DHis, Asn, DAsn, Glu, DGlu, Asp and DAsp, or is absent;
- X37 is selected from the group consisting of Tyr, DTyr, Pro, DPro, Hyp, Phe, Ser, Gly, Ala, Val, Ile, Leu, Thr, Tyr(Me), Gly(Me), Ala(Me), Ile(Me), Aze, Pip, Apr, Php and Bep, or is absent;
and wherein the parentheses () associated with the two cysteine (C, Cys) residues at sequence positions 2 and 7 indicate the presence of an intramolecular disulfide bridge between the two Cys residues in question;
and,
Formula D1 is the following amino acid sequence as set forth in (SEQ ID NO:6): Arg-Cys-X3-Thr-Ala-Thr-Cys-Ala-Thr-X10-Arg-Leu-Ala-X14-Phe-Leu-X17-Arg-X19-X20-Phe-Gly(Me)-Ala-Ile(Me)-Leu-Ser-Ser-Thr-X31-Val-Gly-Ser-X35-Thr-X37 (SEQ ID NO: 6) wherein X represents an amino acid and wherein
- X3 is selected from the group consisting of Asn, Gly, Pro and Gln;
- X10 is selected from the group consisting of Gln, Asp and Glu;
- X14 is selected from the group consisting of Asp, His, Asn and Aad;
- X17 is selected from the group consisting of His, Asn, Gln, Glu, Thr, Val, Lys and Aad;
- X19-X20 is selected from the group consisting of Ser-Ser, Val-Val, Ser-Val and Val-Ser, or is absent;
- X31 is selected from the group consisting of Asp, Glu and Asn;
- X35 is selected from the group consisting of Asp, Glu, Asn, Ser, Phe, Orn, Aad, Gly and Thr; and
- X37 is selected from the group consisting of Pro, Apr and Hyp;
and wherein the compound has at least one residue selected from:
- X3 is Gln;
- X14 is His, Asn or Aad;
- X17 is Asn, Gln, Glu, Thr or Aad;
- X19-X20 is Val-Ser or Ser-Val; and
- X35 is Ser, Phe, Orn, Aad, Gly or Thr;
and,
Formula E1 is the following amino acid sequence as set forth in (SEQ ID NO:7): X1-X2-X3-X4-X5-X6-X7-Ala-Thr-X10-Arg-Leu-Ala-X14-Phe-Leu-X17-Arg-X19-X20-Phe- Gly(Me)-Ala-Ile(Me)-X27-Ser-Ser-Thr-Glu-X32-Gly-Ser-X35-Thr-X37 (SEQ ID NO: 7) wherein X represents an amino acid and
wherein
- X1 is selected from the group consisting of Arg, Lys and Glu;
- X3 is selected from the group consisting of Gly, Gln and Pro;
- X4 is selected from the group consisting of Thr and Glu;
- X5 is selected from the group consisting of Ala and Leu;
- X6 is selected from the group consisting of Thr and Ser;
- X10 is selected from the group consisting of Glu and Gln;
- X14 is selected from the group consisting of Aad, His, Asp, Asn and Arg;
- X17 is selected from the group consisting of Gln, His and Thr;
- X19-X20 is selected from Ser-Ser, Thr-Thr, Ala-Thr, Ala-Ala, Gly-Thr, Gly-Gly and Ala-Asn or is absent;
- X27 is selected from the group consisting of Leu and Pro;
- X32 is selected from the group consisting of Val and Thr;
- X35 is selected from the group consisting of Asn and Ser;
- X37 is selected from the group consisting of Hyp and Pro; and
   X2 and X7 are amino acid residues whose side chains together form a lactam bridge; or a pharmaceutically acceptable salt or solvate thereof.

The sequence numbering corresponds to the amino acids sequence of amylin.

The sequence D1 and E1 contains a two amino acid deletion corresponding to the two residues Asn21 and Asn22 of human amylin. Thus, for ease of comparison with the amylin sequence, the Phe residue immediately C-terminal (downstream) of position X20 is designated as position 23, since it aligns with Phe23 of human amylin

In an embodiment, the short acting amylin RA chosen amongst the peptides according to formula 1 is not native human amylin.

In an embodiment, the short acting amylin RA chosen amongst the peptides according to formula 1 is not pramlintide.

Short acting amylin RA is chosen amongst the short acting amylin RAs that are disclosed in WO12168430, WO12168431, WO12168432, WO13156594, WO16034604, WO15040182, WO16146739, and corresponds to amylin RA having only a peptidic sequence.

In particular the short acting amylin RA consists of a peptidic sequence wherein the C-terminal carboxyl group is in the amide form.

In other word the short acting amylin RA does not comprise a graft comprising an acyl group wherein the acyl group may be of the RCO-type where the R group is an alkyl comprising at least at least 4 carbon atoms and may bear a carboxylic function.

In an embodiment R₁ is hydrogen.

In another embodiment R₂ is OH.

In another embodiment R₂ is NH₂.

In an embodiment R₁ is hydrogen and R₂ is OH

In an embodiment R₁ is hydrogen and R₂ is NH₂.

According to an embodiment the short acting amylin RA can also be chosen amongst the peptides according to Formula 1:

**R₁-Z-R₂** Formula 1

wherein
- R₁ is hydrogen;
- R₂ is NHR₃, wherein R₃ is hydrogen or C₁₋₃-alkyl; and
- Z is an amino acid sequence according to formula A1 or B1 or C1 or D1 or E1.

According to an embodiment the short acting amylin RA can also be chosen amongst the peptides according to Formula 1 :

**R₁-Z-R₂** Formula 1

wherein
- R₁ is hydrogen;
- R₂ is NHR₃, wherein R₃ is hydrogen, and
- Z is an amino acid sequence according to formula A1 or B1 or C1 or D1 or E1.

The Formula 1 with Z according to Formula A1, B1, C1, D1 and E1 covers compounds disclosed in, respectively,
- WO12168430, WO12168431, WO12168432 and WO13156594,
- WO16034604
- WO15040182
- WO16146739
- WO18046719.

The applications WO12168430, WO12168431, WO12168432 and WO13156594, WO16034604, WO15040182, WO16146739, WO18046719 are incorporated by reference, in particular the peptidic sequences they disclose,

In particular the incorporation by reference of these document concerns the peptidic sequence Z as such which do not bear a graft comprising more than 6 carbon atoms.

In an embodiment the amylin RA is according to Formula 1 in which Z is an amino acid of sequence according to formula A1.

In an embodiment the amylin RA is according to Formula 1 in which R₁ is H and R₂ is NH₂, and Z is an amino acid of sequence according to formula A1.

In an embodiment the amylin RA is according to Formula 1 in which Z is an amino acid of sequence according to formula A1-1, said formula A1-1 being the following amino acid sequence as set forth in (SEQ ID NO:8): Xaa1-Cys-Xaa3-Thr-Ala-Thr-Cys-Ala-Thr-Gln-Arg-Leu-Ala-Xaa14-Phe-Leu-Xaa17-Xaa18-Ser-Ser-Xaa21-Xaa22-Phe-Gly-Pro-Xaa26-Leu-Pro-Pro-Thr-Xaa31-Val-Gly-Ser-Xaa35-Thr-Xaa37 (SEQ ID NO:8) wherein Xaa represents an amino acid and wherein
- Xaa1 is absent or independently selected from Ala, Cys, Glu, Gly, His, Arg, Ser and Lys;
- Xaa3 is independently selected from Gly, His, Arg, Ser and Asn;
- Xaa14 is Glu;
- Xaa17 is independently selected from His, Arg, Lys and Val;
- Xaa18 is independently selected from Arg, Lys and His;
- Xaa21 is independently selected from Ala, Lys, Gln, Ser and Asn;
- Xaa22 is independently selected from Glu, Gln, Ser, Thr and Asn;
- Xaa26 is independently selected from Pro, Arg and Ile;
- Xaa31 is independently selected from Ser, Glu, Asp and Asn;
- Xaa35 is independently selected from His, Arg, Lys, Asp and Glu; and
- Xaa37 is independently selected from Pro and Tyr.

In an embodiment the amylin RA is according to Formula 1 in which R₁ is H and R₂ is NH₂, and Z is an amino acid of sequence according to formula A1-1.

In an embodiment the amylin RA is according to Formula 1 in which Z is an amino acid of sequence according to formula A1-2, said formula A1-2 being the following amino acid sequence as set forth in (SEQ ID NO:9): Xaa1-Cys-Xaa3-Thr-Ala-Thr-Cys-Ala-Thr-Gln-Arg-Leu-Ala-Xaa14-Phe-Leu-Xaa17-Xaa18-Ser-Ser-Xaa21-Xaa22-Phe-Gly-Pro-Xaa26-Leu-Pro-Pro-Thr-Xaa31-Val-Gly-Ser-Xaa35-Thr-Xaa37 (SEQ ID NO:9) wherein Xaa represents an amino acid and wherein
- Xaa1 is absent or independently selected from Ala, Cys, Glu, Gly, His, Arg, Ser and Lys;
- Xaa3 is independently selected from Gly, His, Arg, Ser and Asn;
- Xaa14 is independently selected from Glu and Asn;
- Xaa17 is Arg;
- Xaa18 is independently selected from Arg, Lys and His;
- Xaa21 is independently selected from Ala, Lys, Gln, Ser and Asn;
- Xaa22 is independently selected from Glu, Gln, Ser, Thr and Asn;
- Xaa26 is independently selected from Pro, Arg and Ile;
- Xaa31 is independently selected from Ser, Glu, Asp and Asn;
- Xaa35 is independently selected from His, Arg, Lys, Asp and Glu; and
- Xaa37 is independently selected from Pro and Tyr.

In an embodiment the amylin RA is according to Formula 1 in which R₁ is H and R₂ is NH₂, and Z is an amino acid of sequence according to formula A1-2.

In an embodiment the amylin RA is according to Formula 1 in which Z is an amino acid of sequence according to formula A1-3, said formula A1-3 being the following amino acid sequence as set forth in (SEQ ID NO:10): Xaa1-Cys-Xaa3-Thr-Ala-Thr-Cys-Ala-Thr-Gln-Arg-Leu-Ala-Xaa14-Phe-Leu-Xaa17-Xaa18-Ser-Ser-Xaa21-Xaa22-Phe-Gly-Pro-Xaa26-Leu-Pro-Pro-Thr-Xaa31-Val-Gly-Ser-Xaa35-Thr-Xaa37 (SEQ ID NO:10) wherein Xaa represents an amino acid and wherein
- Xaa1 is absent or independently selected from Ala, Cys, Glu, Gly, His, Arg, Ser and Lys;
- Xaa3 is independently selected from Gly, His, Arg, Ser and Asn;
- Xaa14 is independently selected from Glu and Asn
- Xaa17 is independently selected from His, Arg, Lys and Val;
- Xaa18 is independently selected from Arg, Lys and His;
- Xaa21 is independently selected from Ala, Lys, Gln, Ser and Asn;
- Xaa22 is independently selected from Glu, Gln, Ser, Thr and Asn;
- Xaa26 is independently selected from Pro, Arg and Ile;
- Xaa31 is independently selected from Ser, Glu, Asp and Asn; and
- Xaa35 is independently selected from His, Arg, Lys, Asp and Glu;
- Xaa37 is independently selected from Pro and Tyr.

In an embodiment the amylin RA is according to Formula 1 in which Z is an amino acid of sequence according to formula A1-3, said formula A1-3 being the following amino acid sequence as set forth in (SEQ ID NO:10), Xaa37 being Pro.

In an embodiment the amylin RA is according to Formula 1 in which R₁ is H and R₂ is NH₂, and Z is an amino acid of sequence according to formula A1-3.

In an embodiment the amylin RA is according to Formula 1 in which Z is an amino acid of sequence according to formula A1-4, said formula A1-4 being the following amino acid sequence as set forth in (SEQ ID NO:11): Xaa1-Cys-Xaa3-Thr-Ala-Thr-Cys-Ala-Thr-Gln-Arg-Leu-Ala-Xaa14-Phe-Leu-Xaa17-Xaa18-Ser-Ser-Xaa21-Xaa22-Phe-Gly-Xaa25-Xaa26-Xaa27-Xaa28-Xaa29-Thr-Xaa31-Val-Gly-Xaa34-Xaa35-Thr-Xaa37 (SEQ ID NO:11) wherein Xaa represents an amino acid and
wherein
- Xaa1 is independently selected from Ala, Cys, Glu, Gly, His, Arg, Ser and Lys;
- Xaa3 is Asn;
- Xaa14 is independently selected from Asp, Glu, His, Asn, Arg, Gly, Ala, Ser, Lys, Thr and Cys;
- Xaa17 is independently selected from Arg and Val;
- Xaa18 is His;
- Xaa21 is Pro;
- Xaa22 is Asn;
- Xaa25 is independently selected from Pro and Ala;
- Xaa26 is independently selected from Pro and Ile;
- Xaa27 is independently selected from Pro and Leu;
- Xaa28 is independently selected from Pro and Ser;
- Xaa29 is independently selected from Pro and Ser;
- Xaa31 is independently selected from Pro and Asn;
- Xaa34 is independently selected from Pro, His, Lys, Arg and Ser;
- Xaa35 is independently selected from Asp, Arg, Glu, Lys, His and Asn; and
- Xaa37 is independently selected from Pro and Tyr.

In an embodiment the amylin RA is according to Formula 1 in which R₁ is H and R₂ is NH₂, and Z is an amino acid of sequence according to formula A1-4.

In an embodiment the amylin RA has a sequence corresponding to [Glu14,His17,His35]-pramlintide (Cpd 1).

In an embodiment the amylin RA has a sequence corresponding to [Glu14,Arg17,His35]-pramlintide (Cpd 2).

In an embodiment the amylin RA has a sequence corresponding to [Glu14,His17,Pro37]-pramlintide (Cpd 3).

In an embodiment the amylin RA has a sequence corresponding to [Arg1,Glu14,His17,Pro37]-pramlintide (Cpd 4); disclosed as Example 96 in WO 2012/168430 A2.

In an embodiment the amylin RA has a sequence corresponding to [Glu14,Arg17,Pro37]-pramlintide (Cpd 5).

In an embodiment the amylin RA is Cpd 5 and has the following sequence: Lys-Cys-Asn-Thr-Ala-Thr-Cys-Ala-Thr-Gln-Arg-Leu-Ala-Glu-Phe-Leu-Arg-His-Ser-Ser-Asn-Asn-Phe-Gly-Pro-Ile-Leu-Pro-Pro-Thr-Asn-Val-Gly-Ser-Asn-Thr-Pro-Nh₂. (SEQ ID NO: 12).

In an embodiment the amylin RA is according to Formula 1 in which Z is an amino acid of sequence according to formula B1.

In an embodiment the amylin RA corresponds to Formula 1 in which R₁ is H and R₂ is NH₂, and Z is an amino acid of sequence according to formula B1.

In an embodiment the amylin RA corresponds to Formula 1 in which Z is an amino acid of sequence according to formula B1-1, said formula B1-1 being the following amino acid sequence as set forth in (SEQ ID NO:13): X(-1)-X1-X2-X3-X4-X5-X6-X7-X8-X9-X10-X11-X12-X13-X14-X15-X16-X17-X18-X19-X20-X21-X22-X23-X24-X25-X26-X27-X28-X29-X30-X31-X32-X33-X34 (SEQ ID NO: 13),
wherein X represents an amino acid and
wherein
- X(-1) is absent or E,
- X1 is absent or selected from the group consisting of E or A,
- X2 is selected from the group consisting of L, A or P,
- X3 is selected from the group consisting of S or P
- X4 is selected from the group consisting of E, P, K, Q, or G
- X5 is selected from the group consisting of L, V, or I,
- X6 is S, T or H,
- X7 is T,
- X8 is selected from the group consisting of L or A,
- X9 is selected from the group consisting of A, V, I, S or T,
- X10 is selected from the group consisting of L, A, I, H or V,
- X11 is G,
- X12 is selected from the group consisting of R, H or K,
- X13 is L,
- X14 is selected from the group consisting of S, T or E,
- X15 is selected from the group consisting of A, Q, E, e or T,
- X16 is selected from the group consisting of R, E, K or Q,
- X17 is selected from the group consisting of L or I,
- X18 is selected from the group consisting of H or A,
- X19 is selected from the group consisting of E, R or K,
- X20 is selected from the group consisting of L, I or V,
- X21 is selected from the group consisting of A, Q, S, E or T,
- X22 is T,
- X23 is selected from the group consisting of T, Y or L,
- X24 is P,
- X25 is selected from the group consisting of R, P, H or K,
- X26 is T,
- X27 is selected from the group consisting of E, Q, G or K,
- X28 is selected from the group consisting of T or P,
- X29 is G,
- X30 is selected from the group consisting of P, S or T,
- X31 is selected from the group consisting of E, Q, G, A, P or K,
- X32 is selected from the group consisting of T, S, H, P or A and
- X33 is selected from the group consisting of P, Y, H, F, L, S, G or A and
- X34 is absent or G.

In an embodiment the amylin RA is according to Formula 1 in which R₁ is H and R₂ is NH₂, and Z is an amino acid of sequence according to formula B1-1.

In an embodiment the amylin RA is according to Formula 1 in which Z is an amino acid of sequence according to formula B1-2, said formula B1-2 being the following amino acid sequence as set forth in (SEQ ID NO:14): X1-X2-X3-X4-X5-X6-X7-X8-X9-X10-X11-X12-X13-X14-X15-X16-X17-X18-X19-X20-X21-X22-X23-X24-X25-X26-X27-X28-X29-X30-X31-X32-X33-X34-X35-X36 (SEQ ID NO: 14) wherein X represents an amino acid and
wherein
- X1 is absent or selected from the group consisting of E or A,
- X2 is selected from the group consisting of L, A or P,
- X3 is selected from the group consisting of S or P,
- X4 is selected from the group consisting of E, P, K, Q, or G,
- X5 is selected from the group consisting of L, V, or I,
- X6 is S, T or H,
- X7 is T,
- X8 is selected from the group consisting of L or A,
- X9 is selected from the group consisting of A, V, I, S or T,
- X10 is selected from the group consisting of L, A, I, H or V,
- X11 is G,
- X12 is selected from the group consisting of R, H or K,
- X13 is L,
- X14 is selected from the group consisting of S, T or E,
- X15 is selected from the group consisting of A, Q, E, e or T,
- X16 is selected from the group consisting of R, E, K or Q,
- X17 is selected from the group consisting of L or I,
- X18 is selected from the group consisting of H or A,
- X19 is selected from the group consisting of E, R or K,
- X20 is selected from the group consisting of L, I or V,
- X21 is selected from the group consisting of A, Q, S, E or T,
- X22 is T,
- X23 is selected from the group consisting of T, Y or L,
- X24 is P,
- X25 is selected from the group consisting of R, P, H or K,
- X26 is T,
- X27 is selected from the group consisting of E, Q, G or K,
- X28 is selected from the group consisting of T or P,
- X29 is G,
- X30 is selected from the group consisting of P, S or T,
- X31 is selected from the group consisting of E, Q, G, A, P or K,
- X32 is selected from the group consisting of T, S, H, P or A,
- X33 is selected from the group consisting of P, Y, H, F, L, S, G or A,
- X34 is absent or G,
- X35 is absent or T and
- X36 is absent or Y.

In an embodiment the amylin RA is according to Formula 1 in which R₁ is H and R₂ is NH₂, and Z is an amino acid of sequence according to formula B1-2.

In an embodiment the amylin RA is according to Formula 1 in which Z is an amino acid of sequence according to formula B1-3, said formula B1-3 being the following amino acid sequence as set forth in (SEQ ID NO:15): X1-X2-X3-X4-X5-X6-T-X8-X9-X10-G-X12-L-X14-X15-X16-X17-X18-X19-X20-X21-T-X23-P-X25-T-X27-X28-G-X30-X31-X32-X33 (SEQ ID NO: 15), wherein X represents an amino acid and
wherein
- X1 is absent or selected from the group consisting of E or A,
- X2 is selected from the group consisting of L, A or P,
- X3 is selected from the group consisting of S or P,
- X4 is selected from the group consisting of E, P, K, Q or G,
- X5 is selected from the group consisting of L, V, or I,
- X6 is selected from the group consisting of S, T or H,
- X8 is selected from the group consisting of L or A,
- X9 is selected from the group consisting of A, V, I, S or T,
- X10 is selected from the group consisting of L, A, I, H or V,
- X12 is selected from the group consisting of R, H or K,
- X14 is selected from the group consisting of S, T or E,
- X15 is selected from the group consisting of A, Q, E, e or T,
- X16 is selected from the group consisting of R, E, K or Q,
- X17 is selected from the group consisting of L or I,
- X18 is selected from the group consisting of H or A,
- X19 is selected from the group consisting of E, R or K,
- X20 is selected from the group consisting of L, I or V,
- X21 is selected from the group consisting of A, Q, S, E or T,
- X23 is selected from the group consisting of T, Y or L,
- X25 is selected from the group consisting of R, P, H or K,
- X27 is selected from the group consisting of E, Q, G or K,
- X28 is selected from the group consisting of T or P,
- X30 is selected from the group consisting of P, S or T,
- X31 is selected from the group consisting of E, Q, G, A, P or K,
- X32 is selected from the group consisting of T, S, H, P or A and
- X33 is selected from the group consisting of P, Y, H, F, L, S, G or A.

In an embodiment the amylin RA is according to Formula 1 in which R₁ is H and R₂ is NH2, and Z is an amino acid of sequence according to formula B1-3.

In an embodiment the amylin RA is mimylin. This amylin RA has the sequence EASELSTAALGRLSAELHELATLPRTETGPESP-NH₂ SEQ ID NO: 16 (Cpd 6).

In an embodiment the amylin RA is a peptide comprising at least 66% sequence identity to EASELSTAALGRLSAELHELATLPRTETGPESP SEQ ID NO: 17.

In an embodiment the amylin RA is a peptide comprising at least 66% sequence identity to EASELSTAALGRLSAELHELATLPRTETGPESP-NH₂ SEQ ID NO: 18.

In an embodiment the amylin RA is according to Formula 1 in which Z is an amino acid of sequence according to formula C1.

In an embodiment the amylin RA is according to Formula 1 in which R₁ is H and R₂ is NH₂, and Z is an amino acid of sequence according to formula C1.

In an embodiment the amylin RA is according to Formula 1 in which Z is an amino acid of sequence according to formula C1-1, said formula C1-1 being the following amino acid sequence as set forth in (SEQ ID NO:19): X0-X1-Cys()-X3-Thr-Ala-Thr-Cys()-Ala-Thr-X10-Arg-X12-Ala-X14-Phe-X16-X17-X18-X19-X20-X21-X22-X23-X24-X25-X26-X27-X28-X29-X30-X31-Val-Gly-Ser-X35-Thr-X37 (SEQ ID NO: 19) wherein X represents an amino acid and
wherein
- X0 is a basic amino acid or is absent;
- X1 is a basic amino acid or is selected from the group consisting of Ala and Trp;
- X3 is selected from the group consisting of Asn, Gly, Ser, Pro, Asp and Glu;
- X10 is selected from the group consisting of Gln, Gly, Ala, Ser, Leu, Thr, Pro, Asp and Glu;
- X12 is selected from the group consisting of Leu and DLeu;
- X14 is selected from the group consisting of Glu, DGlu, Asp and DAsp;
- X16 is selected from the group consisting of Leu and DLeu, or is absent;
- X17 is selected from the group consisting of His, DHis, Val, DVal, Arg, DArg, Lys, DLys, Orn, DOrn, Dap and Dab, or is absent;
- X18 is selected from the group consisting of His, DHis, Arg, DArg, Dap and Dab, or is absent;
- X19 is selected from the group consisting of Ser and DSer, or is absent;
- X20 is selected from the group consisting of Ser and DSer, or is absent;
- X21 is selected from the group consisting of Asn, DAsn, Glu, DGlu, Asp, DAsp, Ser and DSer, or is absent;
- X22 is selected from the group consisting of Asn, DAsn, Glu, DGlu, Asp, DAsp, Ser and DSer, or is absent;
- X23 is selected from the group consisting of Phe and DPhe, or is absent;
- X24 is selected from the group consisting of Gly(Me), Ala(Me), DAla(Me), Ile(Me), DIle(Me), Ala, DAla, Pro and DPro,or is absent;
- X25 is selected from the group consisting of Ala, DAla, Pro and DPro, or is absent;
- X26 is selected from the group consisting of Gly(Me), Ile(Me), DIle(Me), Ile and DIle, or is absent;
- X27 is selected from the group consisting of Leu and DLeu, or is absent;
- X28 is selected from the group consisting of Ser, DSer, Pro, DPro, Ser(Me) and DSer(Me), or is absent;
- X29 is selected from the group consisting of Ser, DSer, Pro, DPro, Ser(Me) and DSer(Me), or is absent;
- X30 is selected from the group consisting of Thr and DThr, or is absent;
- X31 is selected from the group consisting of Asn, DAsn, Glu, DGlu, Asp and DAsp, or is absent;
- X35 is selected from the group consisting of His, DHis, Asn, DAsn, Glu, DGlu, Asp and DAsp, or is absent;
- X37 is selected from the group consisting of Tyr, DTyr, Pro, DPro, Hyp, Phe, Ser, Gly, Ala, Val, Ile, Leu, Thr, Tyr(Me), Gly(Me), Ala(Me), Ile(Me), Aze, Pip, Apr, Php and Bep, or is absent;
and wherein the parentheses () associated with the two cysteine (C, Cys) residues at sequence positions 2 and 7 indicate the presence of an intramolecular disulfide bridge between the two Cys residues in question.

In an embodiment the amylin RA is according to Formula 1 in which R₁ is H and R₂ is NH₂, and Z is an amino acid of sequence according to formula C1-1.

In an embodiment the amylin RA is according to Formula 1 in which Z is an amino acid of sequence according to formula C1-2 said formula C1-2 being the following amino acid sequence as set forth in (SEQ ID NO:20): X1-Cys()-X3-Thr-Ala-Thr-Cys()-Ala-Thr-X10-Arg-X12-Ala-X14-Phe-X16-X17-X18-X19-X20-X21-X22-X23-X24-X25-X26-X27-X28-X29-X30-X31-Val-Gly-Ser-X35-Thr-X37 (SEQ ID NO: 20) wherein X represents an amino acid and;
wherein
- X1 is a basic amino acid or is selected from the group consisting of Ala and Trp;
- X3 is selected from the group consisting of Asn, Gly, Ser, Pro, Asp and Glu;
- X10 is selected from the group consisting of Gln, Gly, Ala, Ser, Leu, Thr, Pro, Asp and Glu;
- X12 is selected from the group consisting of Leu and DLeu;
- X14 is selected from the group consisting of Glu, DGlu, Asp and DAsp;
- X16 is selected from the group consisting of Leu and DLeu, or is absent;
- X17 is selected from the group consisting of His, DHis, Val, DVal, Arg, DArg, Lys, DLys, Orn, DOrn, Dap and Dab, or is absent;
- X18 is selected from the group consisting of His, DHis, Arg, DArg, Dap and Dab, or is absent;
- X19 is selected from the group consisting of Ser and DSer, or is absent;
- X20 is selected from the group consisting of Ser and DSer, or is absent;
- X21 is selected from the group consisting of Asn, DAsn, Glu, DGlu, Asp, DAsp, Ser and DSer, or is absent;
- X22 is selected from the group consisting of Asn, DAsn, Glu, DGlu, Asp, DAsp, Ser and DSer, or is absent;
- X23 is selected from the group consisting of Phe and DPhe, or is absent;
- X24 is selected from the group consisting of Gly(Me), Ala(Me), DAla(Me), Ile(Me), DIle(Me), Ala, DAla, Pro and DPro, or is absent;
- X25 is selected from the group consisting of Ala, DAla, Pro and DPro, or is absent;
- X26 is selected from the group consisting of Gly(Me), Ile(Me), DIle(Me), Ile and DIle, or is absent;
- X27 is selected from the group consisting of Leu and DLeu, or is absent;
- X28 is selected from the group consisting of Ser, DSer, Pro, DPro, Ser(Me) and DSer(Me), or is absent;
- X29 is selected from the group consisting of Ser, DSer, Pro, DPro, Ser(Me) and DSer(Me), or is absent;
- X30 is selected from the group consisting of Thr and DThr, or is absent;
- X31 is selected from the group consisting of Asn, DAsn, Glu, DGlu, Asp and DAsp, or is absent;
- X35 is selected from the group consisting of His, DHis, Asn, DAsn, Glu, DGlu, Asp and DAsp, or is absent;
- X37 is selected from the group consisting of Tyr, DTyr, Pro, DPro, Hyp, Phe, Ser, Gly, Ala, Val, Ile, Leu, Thr, Tyr(Me), Gly(Me), Ala(Me), Ile(Me), Aze, Pip, Apr, Php and Bep, or is absent;
and wherein the parentheses () associated with the two cysteine (C, Cys) residues at sequence positions 2 and 7 indicate the presence of an intramolecular disulfide bridge between the two Cys residues in question.

In an embodiment the amylin RA is according to Formula 1 in which R₁ is H and R₂ is NH₂, and Z is an amino acid of sequence according to formula C1-2.

In an embodiment in Formula C1, C1-1 and C1-2 at least one peptide bond in the peptide backbone of the amino acid sequence of Z is N-methylated; and that the side-chain of at least one amino acid residue that is other than the amino acid residue bearing the N-methyl group of the N-methylated peptide bond on its N-alpha atom and that is located at a sequence position from X19 to X37 of Z is different from the side-chain of the amino acid residue at the corresponding position in human amylin ; or a pharmaceutically acceptable salt or solvate thereof.

In an embodiment the amylin RA is according to Formula 1 in which Z is an amino acid of sequence according to formula C1-3 said formula C1-3 being the following amino acid sequence as set forth in (SEQ ID NO:21): X1-Cys()-X3-Thr-Ala-Thr-Cys()-Ala-Thr-X10-Arg-Leu-Ala-X14-Phe-X16-X17-X18-X19-X20-X21-X22-X23-X24-X25-X26-X27-X28-X29-X30-X31-Val-Gly-Ser-X35-Thr-X37; (SEQ ID NO: 21) wherein X represents an amino acid and
wherein
- X1 is selected from the group consisting of Trp, Lys and Arg;
- X3 is selected from the group consisting of Gly, Asn, Glu and Pro;
- X10 is selected from the group consisting of Gln, Asp and Glu;
- X14 is selected from the group consisting of Asp and Glu;
- X16 is Leu or is absent;
- X17 is His or is absent;
- X18 is selected from the group consisting of His and Arg, or is absent;
- X19 is selected from the group consisting of Ser and D-Ser, or is absent;
- X20 is Ser or is absent;
- X21 is selected from the group consisting of Asn and Ser, or is absent;
- X22 is selected from the group consisting of Asn and Ser, or is absent;
- X23 is selected from the group consisting of Phe and D-Phe, or is absent;
- X24 is Gly(Me) or is absent;
- X25 is Ala or is absent;
- X26 is Ile(Me) or is absent;
- X27 is Leu or is absent;
- X28 is selected from the group consisting of Ser, Ser(Me), D-Ser and D-Ser(Me), or is absent;
- X29 is selected from the group consisting of Ser and Ser(Me) or is absent;
- X30 is Thr or is absent;
- X31 is selected from the group consisting of Asp, Glu and Asn;
- X35 is selected from the group consisting of Asp, Glu and Asn; and
- X37 is selected from the group consisting of Tyr, Tyr(Me), Pro, Hyp, Apr, Aze, Pip, Php and Bep;
wherein the parentheses () associated with the two cysteine (C, Cys) residues at sequence positions 2 and 7 indicate the presence of an intramolecular disulfide bridge between the two Cys residues in question and wherein no more than three positions may be absent.

In an embodiment the amylin RA is according to Formula 1 in which R₁ is H and R₂ is NH₂, and Z is an amino acid of sequence according to formula C1-3.

In an embodiment in Formula C1-3, no more than three positions may be absent, with the proviso that:
(i) if two positions are absent, they are adjacent to one another or one of the absent positions is X25; and
(ii) if three positions are absent, two are adjacent to one another and the other is X25;
or a pharmaceutically acceptable salt or solvate thereof.

In an embodiment the amylin RA is according to Formula 1 in which Z is an amino acid of sequence according to formula C1-4 said formula C1-4 being the following amino acid sequence as set forth in (SEQ ID NO:22): X1-Cys()-X3-Thr-Ala-Thr-Cys()-Ala-Thr-X10-Arg-Leu-Ala-Glu-Phe-Leu-His-X18-Ser-Ser-X21-X22-X23-Gly(Me)-Ala-Ile(Me)-Leu-X28-Ser-Thr-X31-Val-Gly-Ser-X35-Thr-X37; (SEQ ID NO: 22) wherein X represents an amino acid and
wherein
- X1 is selected from the group consisting of Trp, Lys and Arg;
- X3 is selected from the group consisting of Gly, Asn and Pro;
- X10 is selected from the group consisting of Gln, Asp and Glu;
- X18 is selected from the group consisting of His and Arg;
- X21 is selected from the group consisting of Asn and Ser, or is absent;
- X22 is selected from the group consisting of Asn and Ser, or is absent;
- X23 is selected from the group consisting of Phe and D-Phe;
- X28 is selected from the group consisting of Ser and D-Ser;
- X31 is selected from the group consisting of Asp, Glu and Asn;
- X35 is selected from the group consisting of Asp, Glu and Asn; and
- X37 is selected from the group consisting of Pro and Hyp.

In an embodiment the amylin RA is according to Formula 1 in which R₁ is H and R₂ is NH₂, and Z is an amino acid of sequence according to formula C1-4.

In any of the above formulae C1, C1-1 to C₁₋₄ (except where stated otherwise), the following residues or combinations of residues may be employed:
- X16 is Leu and/or X30 is Thr, e.g. X16 is Leu and X30 is Gly.
- X3 is selected from Asn, Gly and Pro, e.g. X3 is Asn.
- X10 is Gln.
- X14 is Glu.
- X23 is D-Phe and/or X28 is D-Ser. For example, X23 is D-Phe and X28 is D-Ser.
- X21 and/or X22 are absent. For example, X21 and X22 are absent.
- X21 and/or X22 are Asn. For example, X21 and X22 are Asn.
- X21 and/or X22 are Ser. For example, X21 and X22 are Ser.
- X19 and/or X20 are absent. For example. X19 and X20 are absent. (Not applicable for formula C1-4)
- X19 and/or X20 are Ser. For example, X19 and X20 are Ser.
- X25 is absent. (Not applicable for formula C1-4).
- X31 and/or X35 are Glu. For example, X31 and X35 are Glu.
- X31 and/or X35 are Asp. For example, X31 and X35 are Asp.
- X31 and/or X35 are Asn. For example, X31 and X35 are Asn.

In certain embodiments of a compound or pharmaceutically acceptable salt or solvate according to formula C1 and C1-1 to C1-4:
- X0 is selected from the group consisting of Lys, His and Arg, or is absent;
- X1 is selected from the group consisting of Trp, Lys and Arg;
- X3 is selected from the group consisting of Gly, Asn, Glu and Pro;
- X10 is selected from the group consisting of Asp and Glu;
- X12 is Leu;
- X14 is Glu;
- X16 is Leu;
- X17 is His; X18 is Arg;
- X19 is Ser;
- X20 is Ser;
- X21 is selected from the group consisting of Asn and Ser, or is absent;
- X22 is selected from the group consisting of Asn and Ser, or is absent;
- X23 is Phe;
- X24 is Gly(Me);
- X25 is Ala, or is absent;
- X26 is Ile(Me);
- X27 is Leu;
- X28 is Ser;
- X29 is Ser;
- X30 is Thr;
- X31 is selected from the group consisting of Asp, Glu and Asn;
- X35 is selected from the group consisting of Asp, Glu and Asn;
- X37 is selected from the group consisting of Tyr, Pro, Hyp and Apr.

In an embodiment the amylin RA is according to Formula 1 in which Z is an amino acid of sequence according to formula C1-5, said formula C1-5 being the following amino acid sequence as set forth in (SEQ ID NO:23): X1-Cys()-Asn-Thr-Ala-Thr-Cys()-Ala-Thr-Gln-Arg-Leu-Ala-X14-Phe-X16-X17-X18-X19-X20-X21-X22-X23-X24-X25-X26-X27-X28-X29-X30-X31-Val-Gly-Ser-X35-Thr-X37 (SEQ ID NO: 23) wherein X represents an amino acid and
wherein
- X1 is a basic amino acid;
- X14 is selected from the group consisting of Glu, DGlu, Asp and DAsp;
- X16 is selected from the group consisting of Leu and DLeu, or is absent;
- X17 is selected from the group consisting of His, DHis, Val, DVal, Arg, DArg, Dap and Dab, or is absent;
- X18 is selected from the group consisting of His, DHis, Arg, DArg, Dap and Dab, or is absent;
- X19 is selected from the group consisting of Ser and DSer, or is absent;
- X20 is selected from the group consisting of Ser and DSer, or is absent;
- X21 is selected from the group consisting of Asn, DAsn, Glu, DGlu, Asp, DAsp, Ser and DSer, or is absent;
- X22 is selected from the group consisting of Asn, DAsn, Glu, DGlu, Asp, DAsp, Ser and DSer, or is absent;
- X23 is selected from the group consisting of Phe and DPhe, or is absent;
- X24 is selected from the group consisting of Pro, DPro, Ala, DAla, Ala(Me), DAla(Me), Gly(Me), Ile(Me) and DIle(Me), or is absent;
- X25 is selected from the group consisting of Ala, DAla, Pro and DPro, or is absent; X26 is selected from the group consisting of Gly(Me), Ile(Me), DIle(Me) and DIle, or is absent;
- X27 is selected from the group consisting of Leu and DLeu, or is absent;
- X28 is selected from the group consisting of Pro, DPro, Ser, DSer, Ser(Me) and DSer(Me), or is absent;
- X29 is selected from the group consisting of Pro, DPro, Ser, DSer, Ser(Me) and DSer(Me), or is absent;
- X30 is selected from the group consisting of Thr and DThr, or is absent;
- X31 is selected from the group consisting of Asn, DAsn, Glu, DGlu, Asp and DAsp, or is absent;
- X35 is selected from the group consisting of His, DHis, Asn, DAsn, Glu, DGlu, Asp and DAsp, or is absent; and
- X37 is selected from the group consisting of Tyr, DTyr, Pro and DPro, or is absent.

In an embodiment the amylin RA is according to Formula 1 in which R₁ is H and R₂ is NH₂, and Z is an amino acid of sequence according to formula C1-5.

In an embodiment, in Formula C1-5 the peptide bond formed by at least one amino acid residue in sequence positions X19 to X37 of the amino acid sequence of Z is *N*-methylated; and that at least one other of the amino acid residues in sequence positions X19 to X37 of Z is different from the amino acid residue at the corresponding position in the sequence of human amylin; and wherein at most 5 amino acid residues at positions X17 to X31, X35 and X37 are absent; or a pharmaceutically acceptable salt or solvate thereof.

In some embodiments in Formula C1-5 among this latter group of embodiments, X1 is a basic amino acid selected from the group consisting of Lys, DLys, Orn, DOrn, Dap, DDap, Dab, DDab, His, DHis, Arg and DArg.

In some embodiments in Formula C1-5 among this latter group of embodiments, X24 is Gly(Me).

In some embodiments in Formula C1-5 among this latter group of embodiments, X26 is Ile(Me).

In some embodiments in Formula C1-5 among this latter group of embodiments, X24 is Gly(Me) and X26 is Ile(Me).

In some embodiments in Formula C1-5 among this latter group of embodiments, X25 is Ala or is absent.

In some embodiments in Formula C1-5 among this latter group of embodiments, X14 is Glu.

In an embodiment in Formulas C1 and C1-5 the peptide bond formed by at least one amino acid residue in sequence positions X19 to X37 of the amino acid sequence of Z is N-methylated; and at least one other of the amino acid residues in sequence positions X19 to X37 of Z is different from the amino acid residue at the corresponding position in the sequence of human amylin; and wherein at most 5 amino acid residues at positions X17 to X31 , X35 and X37 are absent.

In an embodiment in Formula C1 and C1-1 to C1-5 there is no more than 5 positions which are absent.

In an embodiment in Formula C1 and C1-1 to C1-5 there is no more than 4 positions which are absent.

In an embodiment in Formula C1 and C1-1 to C1-5 there is no more than 3 positions which are absent.

In an embodiment, the amylin RA has the following sequence RC()NTATC()ATQRLAEFLHRSSF-Gly(Me)-A-Ile(Me)-LSSTEVGSETP-NH₂ (SEQ ID No: 24) (Cpd 7).

This sequence is the peptidic sequence of Compound 82 from WO2015040182.

In an embodiment, the amylin RA has the following sequence RC()NTATC()ATQRLAEFLHRSSNNF-Gly(Me)-A-Ile(Me)-LSSTNVGSNTP-NH₂ (SEQ ID NO: 25) (Cpd 8).

This sequence is the peptidic sequence of Compound 18 from WO2015040182.

In an embodiment the amylin RA is according to Formula 1 in which Z is an amino acid of sequence according to formula D1.

In an embodiment the amylin RA is according to Formula 1 in which R₁ is H and R₂ is NH₂, and Z is an amino acid of sequence according to formula D1.

In an embodiment the amylin RA is according to Formula 1 in which Z is an amino acid of sequence according to formula D1-1, said formula D1-1 being the following amino acid sequence as set forth in (SEQ ID NO:26): Arg-Cys-X3-Thr-Ala-Thr-Cys-Ala-Thr-X10-Arg-Leu-Ala-X14-Phe-Leu-X17-Arg-X19-X20-Phe-Gly(Me)-Ala-Ile(Me)-Leu-Ser-Ser-Thr-X31-Val-Gly-Ser-X35-Thr-X37 (SEQ ID NO: 26) wherein X represents an amino acid and
wherein
- X3 is selected from the group consisting of Asn, Gly, Pro and Gln;
- X10 is selected from the group consisting of Gln, Asp and Glu;
- X14 is selected from the group consisting of Asp, His, Asn and Aad;
- X17 is selected from the group consisting of His, Asn, Gln, Glu, Thr, Val, Lys and Aad;
- X19-X20 is selected from the group consisting of Ser-Ser, Val-Val, Ser-Val and Val-Ser, or is absent;
- X31 is selected from the group consisting of Asp, Glu and Asn;
- X35 is selected from the group consisting of Asp, Glu, Asn, Ser, Phe, Orn, Aad, Gly and Thr; and
- X37 is selected from the group consisting of Pro, Apr and Hyp;
or a pharmaceutically acceptable salt or solvate thereof.

In an embodiment the amylin RA corresponds to Formula 1 in which R₁ is H and R₂ is NH₂, and Z is an amino acid of sequence according to formula D1-1.

According to an embodiment the peptide according to Formula D1-1 has at least one residue selected from:
- X3 is Gln;
- X17 is Asn, Gln, Glu, Thr or Aad;
- X19-X20 is Val-Ser or Ser-Val; and
- X35 is Ser, Phe, Orn, Aad, Gly or Thr;
or a pharmaceutically acceptable salt or solvate thereof.

In some embodiments according to formula D1-1 it may be desirable that: X31 is Glu; X19-X20 is Ser-Ser or is absent; and/or X37 is Hyp or Pro.

It may be desirable that the amylin analogue contains at least one of His14, Asn14, Aad14, Gln17 and Thr17.

If X14 is Asp, then it may be desirable that X17 is Asn, Gln, Glu, Thr or Aad.

X17 is Gln may be particularly preferred. In some circumstances, it may be desirable that X35 is not a hydrophobic residue, e.g. Phe. Such residues may increase tendency towards fibrillation in some formulations.

In an embodiment the amylin RA is according to Formula 1 in which Z is an amino acid of sequence according to formula D1-2 said formula D1-2 being the following amino acid sequence as set forth in (SEQ ID NO:27): Arg-Cys-X3-Thr-Ala-Thr-Cys-Ala-Thr-X10-Arg-Leu-Ala-X14-Phe-Leu-X17-Arg-X19-X20-Phe-Gly(Me)-Ala-Ile(Me)-Leu-Ser-Ser-Thr-Glu-Val-Gly-Ser-X35-Thr-X37 ( (SEQ ID NO: 27) wherein X represents an amino acid and
wherein
- X3 is selected from the group consisting of Asn, Gly and Gln;
- X10 is selected from the group consisting of Gln, Asp and Glu;
- X14 is selected from the group consisting of Asp, His and Aad;
- X17 is selected from the group consisting of His, Asn, Gln, Glu, Lys and Aad;
- X19-X20 is Ser-Ser or is absent;
- X35 is selected from the group consisting of Asp, Glu, Asn, Ser, Orn, Aad, Gly and Thr; and
- X37 is selected from the group consisting of Pro and Hyp;
and wherein the compound has at least one residue selected from:
- X3 is Gln;
- X14 is His or Aad;
- X17 is Asn, Gln, Glu or Aad; and
- X35 is Ser, Phe, Orn, Aad, Gly or Thr.

In an embodiment the amylin RA is according to Formula 1 in which R₁ is H and R₂ is NH₂, and Z is an amino acid of sequence according to formula D1-2.

In some embodiments according to formula D1-2, X17 may be selected from His and Gln.

In an embodiment the amylin RA is according to Formula 1 in which Z is an amino acid of sequence according to formula D1-3, said formula D1-3 being the following amino acid sequence as set forth in (SEQ ID NO:28): Arg-Cys-X3-Thr-Ala-Thr-Cys-Ala-Thr-X10-Arg-Leu-Ala-X14-Phe-Leu-X17-Arg-X19-X20-Phe-Gly(Me)-Ala-Ile(Me)-Leu-Ser-Ser-Thr-Glu-Val-Gly-Ser-X35-Thr-X37; (SEQ ID NO: 28)
wherein
- X3 is selected from the group consisting of Asn, Gly and Gln;
- X10 is selected from the group consisting of Gln, Asp and Glu;
- X14 is selected from the group consisting of Asp, His and Aad;
- X17 is selected from the group consisting of His and Gln;
- X19-X20 is Ser-Ser or is absent;
- X35 is selected from the group consisting of Asp, Glu, Asn, Aad and Gly; and
- X37 is selected from the group consisting of Pro and Hyp;
and wherein the compound has at least one residue selected from:
- X3 is Gln;
- X14 is His or Aad;
- X17 is Gln; and
- X35 is Aad.

In an embodiment the amylin RA is according to Formula 1 in which R₁ is H and R₂ is NH₂, and Z is an amino acid of sequence according to formula D1-3.

In particular the short-acting amylin RA according to Formula D1-1 to D1-3 corresponds to the amino acid sequences disclosed in WO2016146739, in particular from pages 9-12, which are incorporated by reference.

In an embodiment, the short acting amylin RA is a peptide having a sequence corresponding to the peptidic sequence of Compound 6, 7, 9, 11, 13, 15, 28, 34, 36, 49, 55, 57, 59, 71 or 79 disclosed in WO2016146739, which is incorporated by reference.

In an embodiment this short acting amylin RA is according to Formula 1 with R₁ being H and R₂ being NH₂.

In an embodiment the short acting amylin RA has the following sequence, R-C()-GTAT-C()-ATERLAHFLQRSSF-Gly(Me)-A-Ile(Me)-LSSTEVGSNT-Hyp-NH₂ (SEQ ID NO: 29) (Cpd 9).

This sequence is the peptidic sequence of Compound 7 of WO2016146739.

In an embodiment the amylin RA is according to Formula 1 in which Z is an amino acid of sequence according to formula E1.

In an embodiment of Formula E1, one of the residues at positions X2 and X7 is a residue with a side chain comprising a carboxylic acid group and the other is a residue with a side chain comprising an amine group, wherein a lactam (cyclic amide) is formed between the carboxylic acid and amine groups. The amine may be a primary or secondary amine, but is typically a primary amine. Suitable amino acids whose side chains can participate in a lactam bridge include Asp, Glu and Aad (having side chains comprising carboxylic acid groups) and Dap, Dab, Orn, Lys and hLys (having side chains comprising amine groups). Any of the amino acids selected from Asp, Glu and Aad may in principle form a lactam bridge with any of the amino acids selected from the group consisting of Dap, Dab, Orn, Lys and hLys.

In an embodiment in Formula E1, X2 is selected from Asp, Glu and Aad and X7 is selected from Dap, Dab, Orn, Lys and hLys.

In an embodiment the amylin RA corresponds to Formula 1 in which R₁ is H and R₂ is NH₂, and Z is an amino acid of sequence according to formula E1.

In an embodiment the amylin RA corresponds to Formula 1 in which Z is an amino acid of sequence according to formula E1-1 said formula E1-1 being the following amino acid sequence as set forth in (SEQ ID NO:30): X1-X2-X3-X4-X5-X6-X7-Ala-Thr-X10-Arg-Leu-Ala-X14-Phe-Leu-X17-Arg-X19-X20-Phe- Gly(Me)-Ala-Ile(Me)-X27-Ser-Ser-Thr-Glu-X32-Gly-Ser-X35-Thr-X37 (SEQ ID NO: 30) wherein X represents an amino acid and
wherein
- X1 is selected from the group consisting of Arg, Lys and Glu;
- X3 is selected from the group consisting of Gly, Gln and Pro;
- X4 is selected from the group consisting of Thr and Glu;
- X5 is selected from the group consisting of Ala and Leu;
- X6 is selected from the group consisting of Thr and Ser;
- X10 is selected from the group consisting of Glu and Gln;
- X14 is selected from the group consisting of Aad, His, Asp, Asn and Arg;
- X17 is selected from the group consisting of Gln, His and Thr;
- X19-X20 is selected from Ser-Ser, Thr-Thr, Ala-Thr, Ala-Ala, Gly-Thr, Gly-Gly and Ala-Asn or is absent;
- X27 is selected from the group consisting of Leu and Pro;
- X32 is selected from the group consisting of Val and Thr;
- X35 is selected from the group consisting of Asn and Ser;
- X37 is selected from the group consisting of Hyp and Pro;
   and
- X2 and X7 are amino acid residues whose side chains together form a lactam bridge; or a pharmaceutically acceptable salt or solvate thereof.

In an embodiment the amylin RA corresponds to Formula 1 in which R₁ is H and R₂ is NH₂, and Z is an amino acid of sequence according to formula E1-1.

In some embodiments, X1 is selected from Arg and Lys.

In some embodiments, X3 is Gly, X4 is Thr, X5 is Ala and/or X6 is Thr, e.g. X3 is Gly, X4 is Thr, X5 is Ala and X6 is Thr.

In some embodiments, X14 is selected from His, Asp and Aad.

In some embodiments, X17 is Gln.

In some embodiments, X19-X20 is selected from Ser-Ser and Thr-Thr, or is absent, e.g. Ser-Ser.

In some embodiments, X32 is Val, X35 is Asn and/or X37 is Hyp.

In an embodiment the amylin RA corresponds to Formula 1 in which Z is an amino acid of sequence according to formula E1-2 said formula E1-2 being the following amino acid sequence as set forth in (SEQ ID NO: 31): X1-X2-Gly-Thr-Ala-Thr-X7-Ala-Thr-X10-Arg-Leu-Ala-X14-Phe-Leu-Gln-Arg-X19-X20-Phe-Gly(Me)-Ala-Ile(Me)-X27-Ser-Ser-Thr-Glu-Val-Gly-Ser-Asn-Thr-Hyp (SEQ ID NO: 31) wherein X represents an amino acid and
wherein
- X1 is selected from the group consisting of Arg and Lys;
- X10 is selected from the group consisting of Glu and Gln;
- X14 is selected from the group consisting of Aad, Asp and His;
- X19-X20 is selected from Ser-Ser and Thr-Thr or is absent;
- X27 is selected from the group consisting of Leu and Pro; and
- X2 and X7 are amino acid residues whose side chains together form a lactam bridge.

In an embodiment the amylin RA corresponds to Formula 1 in which R₁ is H and R₂ is NH₂, and Z is an amino acid of sequence according to formula E1-2.

In some embodiments, X14 is Aad, X19-X20 is Ser-Ser and X27 is Leu.

In particular the specific pairing of short-acting amylin RA according to Formula E1-1 to E1-2 are disclosed in WO2018046719, in particular from pages 8-9, which are incorporated by reference

In particular the short-acting amylin RA according to Formula E1-1 to E1-2 corresponds to the amino acid sequences disclosed in WO2018046719, in particular from pages 9-13, which are incorporated by reference.

In an embodiment the short acting amylin RA has the following sequence, RD()GTATK()ATQRLADFLQRSSF-Gly(Me)-A-Ile(Me)-LSSTE-VGSNT-Hyp-NH₂ (SEQ ID NO: 32) (Cpd 10) corresponding to the peptidic sequence of Compd. 41 of WO18046719.

In an embodiment the short acting amylin RA has the following sequence, RD()GTATK()ATERLA-Aad-FLQRSSF-Gly(Me)-A-Ile(Me)-LSSTEVGSNT-Hyp-NH₂

(SEQ ID NO: 33) (Cpd 11) corresponding to the peptidic sequence of Compd. 35 of WO18046719.

In an embodiment the short acting amylin RA has the following sequence, RD()GTATK()ATERLA-Aad-FLQRTTF-Gly(Me)-A-Ile(Me)-LSSTEVGSNT-Hyp-NH₂ (SEQ ID NO: 34) (Cpd 12) corresponding to the peptidic sequence of Compd. 38 of WO18046719.

In an embodiment the short acting amylin RA has the following sequence, RD()GTATK()ATERLAHFLQRSSF-Gly(Me)-A-Ile(Me)-LSSTE-VGSNT-Hyp-NH₂ (SEQ ID NO: 35) (Cpd 13) corresponding to the peptidic sequence of Compd. 18 of WO18046719.

In an embodiment the short acting amylin RA has the following sequence, KD()GTATK()ATQRLA-Aad-FLQRSSF-Gly(Me)-A-Ile(Me)-LSSTEVGSNT-Hyp-NH₂ (SEQ ID NO: 36) (Cpd 14) corresponding to the peptidic sequence of Compd. 39 of WO18046719.

In an embodiment, the short acting amylin RA corresponds to Formula I with R₁ = H, R₂ = NH₂ and Z is the peptide sequence of ZP8396.

According to an embodiment, the short acting amylin RA has an EC50 in a human amylin receptor potency assay, such as that disclosed in WO2012168430, of about 1800pM or less.

According to an embodiment, the short acting amylin RA has an EC50 in a human calcitonin receptor potency assay, such as that disclosed in WO2012168430, of about 1800 pM or less.

According to an embodiment, the short acting amylin RA has a solubility at pH 4 of about 100 µM or more in a solubility assay, such as that disclosed in WO2012168430.

According to an embodiment, the short acting amylin RA has a solubility at pH 7 of about 100 µM or more in a solubility assay, such as that disclosed in WO2012168430.

According to an embodiment, the short acting amylin RA has a physical stability of about 25 hours or greater in a fibrillogenesis assay (also called a fibrillation assay), such as that disclosed in WO2012168430.

According to an embodiment, the short acting amylin RA has a physical stability of about 20 hours or greater in a fibrillogenesis assay (also called a fibrillation assay).

According to an embodiment, the short acting amylin RA has a physical stability of about 15 hours or greater in a fibrillogenesis assay (also called a fibrillation assay).

According to an embodiment, the short acting amylin RA has a physical stability of about 10 hours or greater in a fibrillogenesis assay (also called a fibrillation assay).

According to an embodiment, the short acting amylin RA has an IC50 in a human amylin receptor potency assay, such as that disclosed in WO2013156594, of about 1200 pM or less.

In an embodiment, the concentration of amilyn RA is ranging from 150 to 10 000 µg/mL.

In an embodiment, the concentration of amilyn RA is ranging from 300 to 9 000 µg/mL.

In an embodiment, the concentration of amilyn RA is ranging from 500 to 8 000 µg/mL.

In an embodiment, the concentration of amilyn RA is ranging from 1000 to 7 000 µg/mL.

In an embodiment, the concentration of amilyn RA is ranging from 1000 to 5 000 µg/mL.

In an embodiment, the concentration of amilyn RA is ranging from 1000 to 3 000 µg/mL.

In an embodiment the amylin RA is a peptide according to Formula 1.

In an embodiment, the concentration of amilyn RA according to Formula 1 is ranging from 150 to 10 000 µg/mL.

In an embodiment, the concentration of amilyn RA according to Formula 1 is ranging from 300 to 9 000 µg/mL.

In an embodiment, the concentration of amilyn RA according to Formula 1 is ranging from 500 to 8 000 µg/mL.

In an embodiment, the concentration of amilyn RA according to Formula 1 is ranging from 1000 to 7 000 µg/mL.

In an embodiment, the concentration of amilyn RA according to Formula 1 is ranging from 1000 to 5 000 µg/mL.

In an embodiment, the concentration of amilyn RA according to Formula 1 is ranging from 1000 to 3 000 µg/mL.

In an embodiment the amylin RA is pramlintide.

In an embodiment, the concentration of pramlintide is ranging from 150 to 10 000 µg/mL.

In an embodiment, the concentration of pramlintide is ranging from 300 to 9 000 µg/mL.

In an embodiment, the concentration of pramlintide is ranging from 500 to 8 000 µg/mL.

In an embodiment, the concentration of pramlintide is ranging from 1000 to 7 000 µg/mL.

In an embodiment, the concentration of pramlintide is ranging from 1000 to 5 000 µg/mL.

In an embodiment, the concentration of pramlintide is ranging from 1000 to 3 000 µg/mL

In an embodiment, the composition allows the delivery of a daily dose of amilyn RA ranging from 100 to 3000 µg/day

In an embodiment, the composition allows the delivery of a daily dose of amilyn RA ranging from 150 to 2500 µg/day.

In an embodiment, the composition allows the delivery of a daily dose of amilyn RA ranging from 250 to 2000 µg/day.

In an embodiment, the composition allows the delivery of a daily dose of amilyn RA ranging from 300 to 2000 µg/day.

In an embodiment, the composition allows the delivery of a daily dose of amilyn RA ranging from 350 to 2000 µg/day.

In an embodiment, the composition allows the delivery of a daily dose of amilyn RA ranging from 400 to 2000 µg/day.

In an embodiment, the composition allows the delivery of a daily dose of amilyn RA ranging from 500 to 2000 µg/day.

In an embodiment, the composition allows the delivery of a daily dose of amilyn RA according to Formula 1 ranging from 150 to 2500 µg/day.

In an embodiment, the composition allows the delivery of a daily dose of amilyn RA according to Formula 1 ranging from 250 to 2000 µg/day.

In an embodiment, the composition allows the delivery of a daily dose of amilyn RA according to Formula 1 ranging from 300 to 2000 µg/day.

In an embodiment, the composition allows the delivery of a daily dose of amilyn RA according to Formula 1 ranging from 350 to 2000 µg/day.

In an embodiment, the composition allows the delivery of a daily dose of amilyn RA according to Formula 1 ranging from 400 to 2000 µg/day.

In an embodiment, the composition allows the delivery of a daily dose of amilyn RA according to Formula 1 ranging from 500 to 2000 µg/day.

In an embodiment, the composition allows the delivery of a daily dose of pramlintide ranging from 150 to 2500 µg/day.

In an embodiment, the composition allows the delivery of a daily dose of pramlintide ranging from 250 to 2000 µg/day.

In an embodiment, the composition allows the delivery of a daily dose of pramlintide ranging from 300 to 2000 µg/day.

In an embodiment, the composition allows the delivery of a daily dose of pramlintide ranging from 350 to 2000 µg/day.

In an embodiment, the composition allows the delivery of a daily dose of pramlintide ranging from 400 to 2000 µg/day.

In an embodiment, the composition allows the delivery of a daily dose of pramlintide ranging from 500 to 2000 µg/day.

In an embodiment, the composition comprises at least one short-acting GLP-1 receptor agonist.

By "short-acting" GLP-1 RA is meant a GLP-1 RA that has an apparent half-life of elimination after subcutaneous injection in humans of less than 8 hours, in particular less than 5 hours, preferably less than 4 hours or even less than 3 hours.

In an embodiment, the GLP-1 RA is selected from the group consisting of exenatide (Byetta^{®}, ASTRA-ZENECA), lixisenatide (Lyxumia^{®}, SANOFI), the analogs or derivatives thereof and pharmaceutically acceptable salts thereof.

Exenatide is a peptide having the sequence: HGEGTFTSDL SKQMEEEAVR LFIEWLKNGG PSSGAPPPS (SEQ ID NO: 37)

Lixisenatide is a peptide having the sequence: HGEGTFTSDL SKQMEEEAVR LFIEWLKNGG PSSGAPPSKK KKKK-[NH2] (SEQ ID NO: 38)

In one embodiment, the composition comprises at least one short-acting GLP-1 receptor agonist at a concentration ranging from 20 to 3500 µg/ml.

In an embodiment, the composition allows the delivery of a daily dose of short-acting GLP-1 RA ranging from 10 to 600 µg/day.

In one embodiment, the composition comprises exenatide at a concentration ranging from 20 to 1000 µg/ml.

In one embodiment, the composition comprises exenatide at a concentration ranging from 40 to 500 µg/ml

In one embodiment, the composition comprises exenatide at a concentration ranging from 60 to 400 µg/ml.

In one embodiment, the composition comprises exenatide at a concentration ranging from 80 to 300 µg/ml.

In one embodiment, the composition comprises exenatide at a concentration ranging from 90 to 250 µg/ml.

In one embodiment, the composition comprises exenatide at a concentration ranging from 100 to 200 µg/ml.

In one embodiment, the composition comprises exenatide at a concentration ranging from 100 to 1500 µg/ml.

In one embodiment, the composition comprises exenatide at a concentration ranging from 250 to 1200 µg/ml.

In one embodiment, the composition comprises lixisenatide at a concentration ranging from 50 to 2000 µg/ml.

In one embodiment, the composition comprises lixisenatide at a concentration ranging from 100 to 1500 µg/ml.

In one embodiment, the composition comprises lixisenatide at a concentration ranging from 150 to 1000 µg/ml.

In one embodiment, the composition comprises lixisenatide at a concentration ranging from 200 to 750 µg/ml.

In one embodiment, the composition comprises lixisenatide at a concentration ranging from 225 to 650 µg/ml.

In one embodiment, the composition comprises lixisenatide at a concentration ranging from 250 to 500 µg/ml.

In one embodiment, the composition comprises lixisenatide at a concentration ranging from 250 to 3500 µg/ml.

In one embodiment, the composition comprises lixisenatide at a concentration ranging from 500 to 2500 µg/ml.

In an embodiment, the composition allows the delivery of a daily dose of exenatide ranging from 10 to 250 µg/day.

In an embodiment, the composition allows the delivery of a daily dose of exenatide ranging from 20 to 220 µg/day.

In an embodiment, the composition allows the delivery of a daily dose of exenatide ranging from 30 to 210 µg/day.

In an embodiment, the composition allows the delivery of a daily dose of exenatide ranging from 40 to 200 µg/day.

In an embodiment, the composition allows the delivery of a daily dose of exenatide ranging from 50 to 190 µg/day.

In an embodiment, the composition allows the delivery of a daily dose of exenatide ranging from 60 to 180 µg/day.

In an embodiment, the compositions allow the delivery of a daily dose of lixisenatide ranging from 20 to 600 µg/day.

In an embodiment, the compositions allow the delivery of a daily dose of lixisenatide ranging from 40 to 550 µg/day.

In an embodiment, the compositions allow the delivery of a daily dose of lixisenatide ranging from 60 to 500 µg/day.

In an embodiment, the compositions allow the delivery of a daily dose of lixisenatide ranging from 80 to 450 µg/day.

In an embodiment, the compositions allow the delivery of a daily dose of lixisenatide ranging from 100 to 400 µg/day.

In an embodiment, the compositions allow the delivery of a daily dose of lixisenatide ranging from 120 to 360 µg/day.

The invention also relates to a composition comprising pharmaceutical active ingredients (API) chosen in the group consisting of a short acting amylin RA and exenatide.

The invention also relates to a composition comprising pharmaceutical active ingredients (API) chosen in the group consisting of a short acting amylin RA and lixisenatide.

The invention also relates to a composition comprising pharmaceutical active ingredients (API) chosen in the group consisting of pramlintide and exenatide.

The invention also relates to a composition comprising pharmaceutical active ingredients (API) chosen in the group consisting of pramlintide and lixisenatide.

The invention also relates to a composition comprising pharmaceutical active ingredients (API) chosen in the group consisting of a short acting amylin RA chosen amongst the peptides according to formula 1 and exenatide.

The invention also relates to a composition comprising pharmaceutical active ingredients (API) chosen in the group consisting of a short acting amylin RA chosen amongst the peptides according to formula 1 and lixisenatide.

In an embodiment, the composition comprises a short acting amylin RA according to formula 1 wherein Z is according to formula A1, in particular with R₁ being H and R₂ being NH₂, and a short acting GLP-1 RA, such as exenatide.

In an embodiment, the composition comprises a short acting amylin RA according to formula 1 wherein Z is according to formula B1, in particular with R₁ being H and R₂ being NH₂, and a short acting GLP-1 RA, such as exenatide.

In an embodiment, the composition comprises a short acting amylin RA according to formula 1 wherein Z is according to formula C1, in particular with R₁ being H and R₂ being NH₂, and a short acting GLP-1 RA, such as exenatide.

In an embodiment, the composition comprises a short acting amylin RA according to formula 1 wherein Z is according to formula D1, in particular with R₁ being H and R₂ being NH₂, and a short acting GLP-1 RA, such as exenatide.

In an embodiment, the composition comprises a short acting amylin RA according to formula 1 wherein Z is according to formula E1, in particular with R₁ being H and R₂ being NH₂, and a short acting GLP-1 RA, such as exenatide.

In an embodiment, the composition comprises a short acting amylin RA according to formula 1 wherein Z is according to formula A1, in particular with R₁ being H and R₂ being NH₂, and a short acting GLP-1 RA, such as lixisenatide.

In an embodiment, the composition comprises a short acting amylin RA according to formula 1 wherein Z is according to formula B1, in particular with R₁ being H and R₂ being NH₂, and a short acting GLP-1 RA, such as lixisenatide.

In an embodiment, the composition comprises a short acting amylin RA according to formula 1 wherein Z is according to formula C1, in particular with R₁ being H and R₂ being NH₂, and a short acting GLP-1 RA, such as lixisenatide.

In an embodiment, the composition comprises a short acting amylin RA according to formula 1 wherein Z is according to formula D1, in particular with R₁ being H and R₂ being NH₂, and a short acting GLP-1 RA, such as lixisenatide.

In an embodiment, the composition comprises a short acting amylin RA according to formula 1 wherein Z is according to formula E1, in particular with R₁ being H and R₂ being NH₂, and a short acting GLP-1 RA, such as lixisenatide.

In an embodiment, the composition comprises a short acting amylin RA according to formula 1 wherein Z is according to formula A1-1, in particular with R₁ being H and R₂ being NH₂, and a short acting GLP-1 RA, such as exenatide.

In an embodiment, the composition comprises a short acting amylin RA according to formula 1 wherein Z is according to formula A1-2, in particular with R₁ being H and R₂ being NH₂, and a short acting GLP-1 RA, such as exenatide.

In an embodiment, the composition comprises a short acting amylin RA according to formula 1 wherein Z is according to formula A1-3, in particular with R₁ being H and R₂ being NH₂, and a short acting GLP-1 RA, such as exenatide.

In an embodiment, the composition comprises a short acting amylin RA according to formula 1 wherein Z is according to formula A1-4, in particular with R₁ being H and R₂ being NH₂, and a short acting GLP-1 RA, such as exenatide.

In an embodiment, the composition comprises a short acting amylin RA according to formula 1 wherein Z is according to formula B1-1, in particular with R₁ being H and R₂ being NH₂, and a short acting GLP-1 RA, such as exenatide.

In an embodiment, the composition comprises a short acting amylin RA according to formula 1 wherein Z is according to formula B1-2, in particular with R₁ being H and R₂ being NH₂, and a short acting GLP-1 RA, such as exenatide.

In an embodiment, the composition comprises a short acting amylin RA according to formula 1 wherein Z is according to formula B1-3, in particular with R₁ being H and R₂ being NH₂, and a short acting GLP-1 RA, such as exenatide.

In an embodiment, the composition comprises a short acting amylin RA according to formula 1 wherein Z is according to formula C1-1, in particular with R₁ being H and R₂ being NH₂, and a short acting GLP-1 RA, such as exenatide.

In an embodiment, the composition comprises a short acting amylin RA according to formula 1 wherein Z is according to formula C1-2, in particular with R₁ being H and R₂ being NH₂, and a short acting GLP-1 RA, such as exenatide.

In an embodiment, the composition comprises a short acting amylin RA according to formula 1 wherein Z is according to formula C1-3, in particular with R₁ being H and R2 being NH₂, and a short acting GLP-1 RA, such as exenatide.

In an embodiment, the composition comprises a short acting amylin RA according to formula 1 wherein Z is according to formula C1-4, in particular with R1 being H and R₂ being NH₂, and a short acting GLP-1 RA, such as exenatide.

In an embodiment, the composition comprises a short acting amylin RA according to formula 1 wherein Z is according to formula C1-5, in particular with R₁ being H and R₂ being NH₂, and a short acting GLP-1 RA, such as exenatide.

In an embodiment, the composition comprises a short acting amylin RA according to formula 1 wherein Z is according to formula D1-1, in particular with R₁ being H and R₂ being NH₂, and a short acting GLP-1 RA, such as exenatide.

In an embodiment, the composition comprises a short acting amylin RA according to formula 1 wherein Z is according to formula D1-2, in particular with R₁ being H and R₂ being NH₂, and a short acting GLP-1 RA, such as exenatide.

In an embodiment, the composition comprises a short acting amylin RA according to formula 1 wherein Z is according to formula D1-3, in particular with R₁ being H and R₂ being NH₂, and a short acting GLP-1 RA, such as exenatide.

In an embodiment, the composition comprises a short acting amylin RA according to formula 1 wherein Z is according to formula E1-1, in particular with R₁ being H and R₂ being NH₂, and a short acting GLP-1 RA, such as exenatide.

In an embodiment, the composition comprises a short acting amylin RA according to formula 1 wherein Z is according to formula E1-2, in particular with R₁ being H and R₂ being NH₂, and a short acting GLP-1 RA, such as exenatide.

In an embodiment, the composition comprises a short acting amylin RA according to formula 1 wherein Z is according to formula A1-1, in particular with R₁ being H and R₂ being NH₂, and a short acting GLP-1 RA, such as lixisenatide.

In an embodiment, the composition comprises a short acting amylin RA according to formula 1 wherein Z is according to formula A1-2, in particular with R₁ being H and R₂ being NH₂, and a short acting GLP-1 RA, such as lixisenatide.

In an embodiment, the composition comprises a short acting amylin RA according to formula 1 wherein Z is according to formula A1-3, in particular with R₁ being H and R₂ being NH₂, and a short acting GLP-1 RA, such as lixisenatide.

In an embodiment, the composition comprises a short acting amylin RA according to formula 1 wherein Z is according to formula A1-4, in particular with R₁ being H and R₂ being NH₂, and a short acting GLP-1 RA, such as lixisenatide.

In an embodiment, the composition comprises a short acting amylin RA according to formula 1 wherein Z is according to formula B1-1, in particular with R₁ being H and R₂ being NH₂, and a short acting GLP-1 RA, such as lixisenatide.

In an embodiment, the composition comprises a short acting amylin RA according to formula 1 wherein Z is according to formula B1-2, in particular with R₁ being H and R₂ being NH₂, and a short acting GLP-1 RA, such as lixisenatide.

In an embodiment, the composition comprises a short acting amylin RA according to formula 1 wherein Z is according to formula B1-3, in particular with R₁ being H and R₂ being NH₂, and a short acting GLP-1 RA, such as lixisenatide.

In an embodiment, the composition comprises a short acting amylin RA according to formula 1 wherein Z is according to formula C1-1, in particular with R₁ being H and R₂ being NH₂, and a short acting GLP-1 RA, such as lixisenatide.

In an embodiment, the composition comprises a short acting amylin RA according to formula 1 wherein Z is according to formula C1-2, in particular with R₁ being H and R₂ being NH₂, and a short acting GLP-1 RA, such as lixisenatide.

In an embodiment, the composition comprises a short acting amylin RA according to formula 1 wherein Z is according to formula C1-3, in particular with R₁ being H and R₂ being NH₂, and a short acting GLP-1 RA, such as lixisenatide.

In an embodiment, the composition comprises a short acting amylin RA according to formula 1 wherein Z is according to formula C1-4, in particular with R₁ being H and R₂ being NH₂, and a short acting GLP-1 RA such as lixisenatide.

In an embodiment, the composition comprises a short acting amylin RA according to formula 1 wherein Z is according to formula C1-5, in particular with R₁ being H and R₂ being NH₂, and a short acting GLP-1 RA, such as lixisenatide.

In an embodiment, the composition comprises a short acting amylin RA according to formula 1 wherein Z is according to formula D1-1, in particular with R₁ being H and R₂ being NH₂, and a short acting GLP-1 RA, such as lixisenatide.

In an embodiment, the composition comprises a short acting amylin RA according to formula 1 wherein Z is according to formula D1-2, in particular with R₁ being H and R₂ being NH₂, and a short acting GLP-1 RA, such as lixisenatide.

In an embodiment, the composition comprises a short acting amylin RA according to formula 1 wherein Z is according to formula D1-3, in particular with R₁ being H and R₂ being NH2, and a short acting GLP-1 RA, such as lixisenatide.

In an embodiment, the composition comprises a short acting amylin RA according to formula 1 wherein Z is according to formula E1-1, in particular with R₁ being H and R₂ being NH₂, and a short acting GLP-1 RA, such as lixisenatide.

In an embodiment, the composition comprises a short acting amylin RA according to formula 1 wherein Z is according to formula E1-2, in particular with R₁ being H and R₂ being NH₂, and a short acting GLP-1 RA, such as lixisenatide.

In an embodiment the composition comprises a short acting amylin RA that has a sequence corresponding to [Glu14,His17,His35]-pramlintide (Cpd 1) and exenatide.

In an embodiment the composition comprises a short acting amylin RA that has a sequence corresponding to [Glu14,His17,His35]-pramlintide (Cpd 1) and lixisenatide.

In an embodiment the composition comprises a short acting amylin RA that has a sequence corresponding to [Glu14,Arg17,His35]-pramlintide (Cpd 2) and exenatide.

In an embodiment the composition comprises a short acting amylin RA that has a sequence corresponding to [Glu14,Arg17,His35]-pramlintide (Cpd 2) and lixisenatide.

In an embodiment the composition comprises a short acting amylin RA that has a sequence corresponding to [Glu14,His17,Pro37]-pramlintide (Cpd 3) and exenatide.

In an embodiment the composition comprises a short acting amylin RA that has a sequence corresponding to [Glu14,His17,Pro37]-pramlintide (Cpd 3) and lixisenatide.

In an embodiment the composition comprises a short acting amylin RA that has a sequence corresponding to [Arg1,Glu14,His17,Pro37]-pramlintide (Cpd 4) and exenatide.

In an embodiment the composition comprises a short acting amylin RA that has a sequence corresponding to [Arg1,Glu14,His17,Pro37]-pramlintide (Cpd 4) and lixisenatide.

In an embodiment the composition comprises a short acting amylin RA that has a sequence corresponding to [Glu14,Arg17,Pro37]-pramlintide (Cpd 5) and exenatide.

In an embodiment the composition comprises a short acting amylin RA that has a sequence corresponding to [Glu14,Arg17,Pro37]-pramlintide (Cpd 5) and lixisenatide.

In an embodiment the composition comprises a short acting amylin RA has the sequence EASELSTAALGRLSAELHELATLPRTETGPESP-NH₂ (SEQ ID NO: 16) (Cpd 6) and exenatide.

In an embodiment the composition comprises a short acting amylin RA has the sequence EASELSTAALGRLSAELHELATLPRTETGPESP-NH₂ (SEQ ID NO: 16) (Cpd 6) and lixisenatide.

In an embodiment the composition comprises a short acting amylin RA that has the following sequence RC()NTATC()ATQRLAEFLHRSSF-Gly(Me)-A-Ile(Me)-LSSTEVGSETP-NH₂ (SEQ ID NO: 24) (Cpd 7) and exenatide.

In an embodiment the composition comprises a short acting amylin RA that has the following sequence RC()NTATC()ATQRLAEFLHRSSF-Gly(Me)-A-Ile(Me)-LSSTEVGSETP-NH₂ (SEQ ID NO: 24) (Cpd 7) and lixisenatide.

In an embodiment the composition comprises a short acting amylin RA that has the following sequence R-C()NTATC()ATQRLAEFLHRSSNNF-Gly(Me)-A-Ile(Me)-LSSTNVGSNTP -NH₂ (SEQ ID NO: 25) (Cpd 8) and exenatide.

In an embodiment the composition comprises a short acting amylin RA that has the following sequence R-C()NTATC()ATQRLAEFLHRSSNNF-Gly(Me)-A-Ile(Me)-LSSTNVGSNTP -NH₂ (SEQ ID NO: 25) (Cpd 8) and lixisenatide.

In an embodiment the composition comprises a short acting amylin RA that has the following sequence R-C()-GTAT-C()-ATERLAHFLQRSSF-Gly(Me)-A-Ile(Me)-LSSTEVGSNT-Hyp-NH₂ (SEQ ID NO: 29) (Cpd 9) and exenatide.

In an embodiment the composition comprises a short acting amylin RA that has the following sequence, RD()GTATK()ATQRLADFLQRSSF-Gly(Me)-A-Ile(Me)-LSSTE-VGSNT-Hyp-NH₂ (SEQ ID NO: 32) (Cpd 10) and exenatide.

In an embodiment the composition comprises a short acting amylin RA that has the following sequence, RD()GTATK()ATERLA-Aad-FLQRSSF-Gly(Me)-A-Ile(Me)-LSSTEVGSNT-Hyp-NH2 (SEQ ID NO: 33) (Cpd 11) and exenatide.

In an embodiment the composition comprises a short acting amylin RA that has the following sequence, RD()GTATK()ATERLA-Aad-FLQRTTF-Gly(Me)-A-Ile(Me)-LSSTEVGSNT-Hyp-NH₂ (SEQ ID NO: 34) (Cpd 12) and exenatide.

In an embodiment the composition comprises a short acting amylin RA that has the following sequence, RD()GTATK()ATERLAHFLQRSSF-Gly(Me)-AIle(Me)-LSSTE-VGSNT-Hyp-NH₂ (SEQ ID NO: 35) (Cpd 13) and exenatide.

In an embodiment the composition comprises a short acting amylin RA that has the following sequence, KD()GTATK()ATQRLA-Aad-FLQRSSF-Gly(Me)-A-Ile(Me)-LSSTEVGSNT-Hyp-NH₂ (SEQ ID NO: 36) (Cpd 14) and exenatide.

In an embodiment the composition comprises a short acting amylin RA that has the following sequence R-C()-GTAT-C()-ATERLAHFLQRSSF-Gly(Me)-A-Ile(Me)-LSSTE-VGSNT-Hyp-NH₂ (SEQ ID NO: 29) (Cpd 9) and lixisenatide.

In an embodiment the composition comprises a short acting amylin RA that has the following sequence, RD()GTATK()ATQRLADFLQRSSF-Gly(Me)-A-Ile(Me)-LSSTEVGSNT-Hyp-NH₂ (SEQ ID NO: 32) (Cpd 10) and lixisenatide.

In an embodiment the composition comprises a short acting amylin RA that has the following sequence, RD()GTATK()ATERLA-Aad-FLQRSSF-Gly(Me)-A-Ile(Me)-LSSTEVGSNT-Hyp-NH₂ (SEQ ID NO: 33) (Cpd 11) and lixisenatide.

In an embodiment the composition comprises a short acting amylin RA that has the following sequence, RD()GTATK()ATERLA-Aad-FLQRTTF-Gly(Me)-A-Ile(Me)-LSSTE-VGSNT-Hyp-NH2 (SEQ ID NO: 34) (Cpd 12) and lixisenatide.

In an embodiment the composition comprises a short acting amylin RA that has the following sequence, RD()GTATK()ATERLAHFLQRSSF-Gly(Me)-AIle(Me)-LSSTEVGSNTHyp-NH₂ (SEQ ID NO: 35) (Cpd 13) and lixisenatide.

In an embodiment the composition comprises a short acting amylin RA that has the following sequence, KD()GTATK()ATQRLA-Aad-FLQRSSF-Gly(Me)-A-Ile(Me)-LSSTEVGSNT-Hyp-NH₂ (SEQ ID NO: 36) (Cpd 14) and lixisenatide.

In an embodiment, the composition comprises at least one short-acting glucagon receptor agonist. By "short-acting" glucagon RA is meant glucagon RA having an apparent half-life of elimination after subcutaneous injection in humans of less than 3 hours, in particular less than 2 hours, preferably less than 1.5 hours or even less than 1 hour.

The glucagon receptor agonist may be a glucagon analog.

As used herein, "analog" means a peptide wherein one or more amino acid residues of the basic human glucagon peptide have been substituted by another amino acid residue and/or wherein one or more amino acid residues of the peptide have been deleted and/or wherein one or more amino acid residues have been added to the peptide. Such addition can take place either at the N-terminal end or at the C-terminal end or both.

The glucagon analogs may present up to 8, in particular up to 7, substitutions, deletions or additions compared to the sequence of human glucagon. The substitutions may be done with standard amino acid residues or by non-standard amino-acid residues, such as synthetic amino acid residues, among which can be cited Aib (2-aminoisobutyric acid).

In an embodiment, the glucagon analog present up to 8, in particular up to 7, substitutions compared to the sequence of human glucagon.

In one embodiment the glucagon RA is chosen in the group of human glucagon and dasiglucagon (Dasiglucagon^{®}, Zealand Pharma).

Human glucagon is a peptide having the sequence: HSQGTFTSDY SKYLDSRRAQ DFVQWLMNT (SEQ ID NO: 39)

Dasiglucagon is a peptide having the sequence: HSQGTFTSDY SKYLDXARAE EFVKWLEST where X is Aib (SEQ ID NO: 40)

In one embodiment the glucagon RA is human glucagon.

In one embodiment the glucagon RA is dasiglucagon.

In one embodiment the concentration in glucagon RA is comprised from 10 to 15000 µg/mL.

In one embodiment the concentration in glucagon RA is comprised from 100 to 10000 µg/mL.

In one embodiment the concentration in glucagon RA is comprised from 1000 to 5000 µg/mL.

In one embodiment the human glucagon concentration is comprised from 30 to 12000 µg/mL.

In one embodiment the human glucagon concentration is comprised from 100 to 12000 µg/mL.

In one embodiment the human glucagon concentration is comprised from 1000 to 10000 µg/mL.

In one embodiment the human glucagon concentration is comprised from 1000 to 5000 µg/mL.

In one embodiment the human glucagon concentration is comprised from 2000 to 4000 µg/mL.

In one embodiment the dasiglucagon concentration is comprised from 10 to 5000 µg/mL.

In one embodiment the dasiglucagon concentration is comprised from 100 to 4000 µg/mL.

In one embodiment the dasiglucagon concentration is comprised from 200 to 3000 µg/mL.

In one embodiment the dasiglucagon concentration is comprised from 200 to 2000 µg/mL.

In one embodiment the dasiglucagon concentration is comprised from 300 to 1500 µg/mL.

In an embodiment, the composition allows the delivery of a daily dose of human glucagon ranging from 250 to 10000 µg/day.

In an embodiment, the composition allows the delivery of a daily dose of human glucagon ranging from 500 to 7500 µg/day.

In an embodiment, the composition allows the delivery of a daily dose of human glucagon ranging from 500 to 5000 µg/day.

In an embodiment, the composition allows the delivery of a daily dose of dasiglucagon ranging from 100 to 5000 µg/day.

In an embodiment, the composition allows the delivery of a daily dose of dasiglucagon ranging from 250 to 3000 µg/day.

In an embodiment, the composition allows the delivery of a daily dose of dasiglucagon ranging from 250 to 2000 µg/day.

In an embodiment, the composition comprises a molar ratio between glucagon RA and GLP-1 RA ranging from 1 to 60.

In an embodiment the molar ratio between glucagon RA and GLP-1 RA in the composition is ranging from 2 to 50.

In an embodiment the molar ratio between glucagon RA and GLP-1 RA in the composition is ranging from 2 to 25.

In an embodiment the molar ratio between human glucagon and exenatide in the composition is ranging from 3 to 35.

In an embodiment the molar ratio between human glucagon and exenatide in the composition is ranging from 4 to 25.

In an embodiment the molar ratio between human glucagon and exenatide in the composition is ranging from 5 to 20.

In an embodiment the molar ratio between human glucagon and exenatide in the composition is ranging from 8 to 60.

In an embodiment the molar ratio between human glucagon and exenatide in the composition is ranging from 10 to 55.

In an embodiment the molar ratio between human glucagon and exenatide in the composition is ranging from 15 to 40.

In an embodiment the molar ratio between human glucagon and lixisenatide in the composition is ranging from 2 to 40.

In an embodiment the molar ratio between human glucagon and lixisenatide in the composition is ranging from 3 to 30.

In an embodiment the molar ratio between human glucagon and lixisenatide in the composition is ranging from 4 to 20.

In an embodiment the molar ratio between dasiglucagon and exenatide in the composition is ranging from 0.5 to 15.

In an embodiment the molar ratio between dasiglucagon and exenatide in the composition is ranging from 1 to 10.

In an embodiment the molar ratio between dasiglucagon and exenatide in the composition is ranging from 1.2 to 8.

In an embodiment the molar ratio between dasiglucagon and exenatide in the composition is ranging from 1 to 40.

In an embodiment the molar ratio between dasiglucagon and exenatide in the composition is ranging from 2 to 25.

In an embodiment the molar ratio between dasiglucagon and exenatide in the composition is ranging from 3 to 16.

In an embodiment the molar ratio between dasiglucagon and lixisenatide in the composition is ranging from 0.3 to 15.

In an embodiment the molar ratio between dasiglucagon and lixisenatide in the composition is ranging from 0.5 to 10.

In an embodiment the molar ratio between dasiglucagon and lixisenatide in the composition is ranging from 1 to 7.

In an embodiment, the administration of the composition leads, at steady state, to a molar ratio of exenatide and human glucagon ranging from 0.3 to 15.

In an embodiment, the administration of the composition leads, at steady state, to a molar ratio of exenatide and human glucagon ranging from 0.5 to 8.

In an embodiment, the administration of the composition leads, at steady state, to a molar ratio of exenatide and human glucagon ranging from 0.8 to 4.

In an embodiment, the administration of the composition leads, at steady state, to a molar ratio of exenatide and dasiglucagon ranging from 0.3 to 15.

In an embodiment, the administration of the composition leads, at steady state, to a molar ratio of exenatide and dasiglucagon ranging from 0.5 to 8.

In an embodiment, the administration of the composition leads, at steady state, to a molar ratio of exenatide and dasiglucagon ranging from 0.8 to 4.

In an embodiment, the administration of the composition leads, at steady state, to a molar ratio of lixisenatide and human glucagon ranging from 0.1 to 2.

In an embodiment, the administration of the composition leads, at steady state, to a molar ratio of lixisenatide and human glucagon ranging from 0.25 to 1.5.

In an embodiment, the administration of the composition leads, at steady state, to a molar ratio of lixisenatide and human glucagon ranging from 0.3 to 1.3.

In an embodiment, the administration of the composition leads, at steady state, to a molar ratio of lixisenatide and dasiglucagon ranging from 0.1 to 2.

In an embodiment, the administration of the composition leads, at steady state, to a molar ratio of lixisenatide and dasiglucagon ranging from 0.25 to 1.5.

In an embodiment, the administration of the composition leads, at steady state, to a molar ratio of lixisenatide and dasiglucagon ranging from 0.3 to 1.3.

The invention also relates to a composition comprising pharmaceutical active ingredients (API) chosen in the group consisting of pramlintide and human glucagon.

The invention also relates to a composition comprising pharmaceutical active ingredients (API) chosen in the group consisting of pramlintide and dasiglucagon.

The invention also relates to a composition comprising pharmaceutical active ingredients (API) chosen in the group consisting of a short acting amylin RA chosen amongst the peptides according to formula 1 and human glucagon.

The invention also relates to a composition comprising pharmaceutical active ingredients (API) chosen in the group consisting of a short acting amylin RA chosen amongst the peptides according to formula 1 and dasiglucagon.

In an embodiment, the composition comprises a short acting amylin RA according to formula 1 wherein Z is according to formula A1, in particular with R₁ being H and R₂ being NH₂, and a short acting GLP-1 RA, such as human glucagon.

In an embodiment, the composition comprises a short acting amylin RA according to formula 1 wherein Z is according to formula B1, in particular with R₁ being H and R₂ being NH₂, and a short acting GLP-1 RA, such as human glucagon.

In an embodiment, the composition comprises a short acting amylin RA according to formula 1 wherein Z is according to formula C1, in particular with R₁ being H and R₂ being NH₂, and a short acting GLP-1 RA, such as human glucagon.

In an embodiment, the composition comprises a short acting amylin RA according to formula 1 wherein Z is according to formula D1, in particular with R₁ being H and R₂ being NH₂, and a short acting GLP-1 RA, such as human glucagon.

In an embodiment, the composition comprises a short acting amylin RA according to formula 1 wherein Z is according to formula E1, in particular with R₁ being H and R₂ being NH₂, and a short acting GLP-1 RA, such as human glucagon.

In an embodiment, the composition comprises a short acting amylin RA according to formula 1 wherein Z is according to formula A1, in particular with R₁ being H and R₂ being NH₂, and a short acting GLP-1 RA, such as dasiglucagon.

In an embodiment, the composition comprises a short acting amylin RA according to formula 1 wherein Z is according to formula B1, in particular with R₁ being H and R₂ being NH₂, and a short acting GLP-1 RA, such as dasiglucagon.

In an embodiment, the composition comprises a short acting amylin RA according to formula 1 wherein Z is according to formula C1, in particular with R₁ being H and R₂ being NH₂, and a short acting GLP-1 RA, such as dasiglucagon.

In an embodiment, the composition comprises a short acting amylin RA according to formula 1 wherein Z is according to formula D1, in particular with R₁ being H and R₂ being NH₂, and a short acting GLP-1 RA, such as dasiglucagon.

In an embodiment, the composition comprises a short acting amylin RA according to formula 1 wherein Z is according to formula E1, in particular with R₁ being H and R₂ being NH₂, and a short acting GLP-1 RA, such as dasiglucagon.

In an embodiment, the composition comprises a short acting amylin RA according to formula 1 wherein Z is according to formula A1-1, in particular with R₁ being H and R₂ being NH₂, and a short acting GLP-1 RA, such as human glucagon.

In an embodiment, the composition comprises a short acting amylin RA according to formula 1 wherein Z is according to formula A1-2, in particular with R₁ being H and R₂ being NH₂, and a short acting GLP-1 RA, such as human glucagon.

In an embodiment, the composition comprises a short acting amylin RA according to formula 1 wherein Z is according to formula A1-3, in particular with R₁ being H and R₂ being NH₂, and a short acting GLP-1 RA, such as human glucagon.

In an embodiment, the composition comprises a short acting amylin RA according to formula 1 wherein Z is according to formula A1-4, in particular with R₁ being H and R₂ being NH₂, and a short acting GLP-1 RA, such as human glucagon.

In an embodiment, the composition comprises a short acting amylin RA according to formula 1 wherein Z is according to formula B1-1, in particular with R₁ being H and R₂ being NH₂, and a short acting GLP-1 RA, such as human glucagon.

In an embodiment, the composition comprises a short acting amylin RA according to formula 1 wherein Z is according to formula B1-2, in particular with R₁ being H and R₂ being NH₂, and a short acting GLP-1 RA, such as human glucagon.

In an embodiment, the composition comprises a short acting amylin RA according to formula 1 wherein Z is according to formula B1-3, in particular with R₁ being H and R₂ being NH₂, and a short acting GLP-1 RA, such as human glucagon.

In an embodiment, the composition comprises a short acting amylin RA according to formula 1 wherein Z is according to formula C1-1, in particular with R₁ being H and R₂ being NH₂, and a short acting GLP-1 RA, such as human glucagon.

In an embodiment, the composition comprises a short acting amylin RA according to formula 1 wherein Z is according to formula C1-2, in particular with R₁ being H and R₂ being NH₂, and a short acting GLP-1 RA, such as human glucagon.

In an embodiment, the composition comprises a short acting amylin RA according to formula 1 wherein Z is according to formula C1-3, in particular with R₁ being H and R2 being NH₂, and a short acting GLP-1 RA, such as human glucagon.

In an embodiment, the composition comprises a short acting amylin RA according to formula 1 wherein Z is according to formula C1-4, in particular with R1 being H and R₂ being NH₂, and a short acting GLP-1 RA, such as human glucagon.

In an embodiment, the composition comprises a short acting amylin RA according to formula 1 wherein Z is according to formula C1-5, in particular with R₁ being H and R₂ being NH₂, and a short acting GLP-1 RA, such as human glucagon.

In an embodiment, the composition comprises a short acting amylin RA according to formula 1 wherein Z is according to formula D1-1, in particular with R₁ being H and R₂ being NH₂, and a short acting GLP-1 RA, such as human glucagon.

In an embodiment, the composition comprises a short acting amylin RA according to formula 1 wherein Z is according to formula D1-2, in particular with R₁ being H and R₂ being NH₂, and a short acting GLP-1 RA, such as human glucagon.

In an embodiment, the composition comprises a short acting amylin RA according to formula 1 wherein Z is according to formula D1-3, in particular with R₁ being H and R₂ being NH₂, and a short acting GLP-1 RA, such as human glucagon.

In an embodiment, the composition comprises a short acting amylin RA according to formula 1 wherein Z is according to formula E1-1, in particular with R₁ being H and R₂ being NH₂, and a short acting GLP-1 RA, such as human glucagon.

In an embodiment, the composition comprises a short acting amylin RA according to formula 1 wherein Z is according to formula E1-2, in particular with R₁ being H and R₂ being NH₂, and a short acting GLP-1 RA, such as human glucagon.

In an embodiment, the composition comprises a short acting amylin RA according to formula 1 wherein Z is according to formula A1-1, in particular with R₁ being H and R₂ being NH₂, and a short acting GLP-1 RA, such as dasiglucagon.

In an embodiment, the composition comprises a short acting amylin RA according to formula 1 wherein Z is according to formula A1-2, in particular with R₁ being H and R₂ being NH₂, and a short acting GLP-1 RA, such as dasiglucagon.

In an embodiment, the composition comprises a short acting amylin RA according to formula 1 wherein Z is according to formula A1-3, in particular with R₁ being H and R₂ being NH₂, and a short acting GLP-1 RA, such as dasiglucagon.

In an embodiment, the composition comprises a short acting amylin RA according to formula 1 wherein Z is according to formula A1-4, in particular with R₁ being H and R₂ being NH₂, and a short acting GLP-1 RA, such as dasiglucagon.

In an embodiment, the composition comprises a short acting amylin RA according to formula 1 wherein Z is according to formula B1-1, in particular with R₁ being H and R₂ being NH₂, and a short acting GLP-1 RA, such as dasiglucagon.

In an embodiment, the composition comprises a short acting amylin RA according to formula 1 wherein Z is according to formula B1-2, in particular with R₁ being H and R₂ being NH₂, and a short acting GLP-1 RA, such as dasiglucagon.

In an embodiment, the composition comprises a short acting amylin RA according to formula 1 wherein Z is according to formula B1-3, in particular with R₁ being H and R₂ being NH₂, and a short acting GLP-1 RA, such as dasiglucagon.

In an embodiment, the composition comprises a short acting amylin RA according to formula 1 wherein Z is according to formula C1-1, in particular with R₁ being H and R₂ being NH₂, and a short acting GLP-1 RA, such as dasiglucagon.

In an embodiment, the composition comprises a short acting amylin RA according to formula 1 wherein Z is according to formula C1-2, in particular with R₁ being H and R₂ being NH₂, and a short acting GLP-1 RA, such as dasiglucagon.

In an embodiment, the composition comprises a short acting amylin RA according to formula 1 wherein Z is according to formula C1-3, in particular with R₁ being H and R₂ being NH₂, and a short acting GLP-1 RA, such as dasiglucagon.

In an embodiment, the composition comprises a short acting amylin RA according to formula 1 wherein Z is according to formula C1-4, in particular with R₁ being H and R₂ being NH₂, and a short acting GLP-1 RA such as dasiglucagon.

In an embodiment, the composition comprises a short acting amylin RA according to formula 1 wherein Z is according to formula C1-5, in particular with R₁ being H and R₂ being NH₂, and a short acting GLP-1 RA, such as dasiglucagon.

In an embodiment, the composition comprises a short acting amylin RA according to formula 1 wherein Z is according to formula D1-1, in particular with R₁ being H and R₂ being NH₂, and a short acting GLP-1 RA, such as dasiglucagon.

In an embodiment, the composition comprises a short acting amylin RA according to formula 1 wherein Z is according to formula D1-2, in particular with R₁ being H and R₂ being NH₂, and a short acting GLP-1 RA, such as dasiglucagon.

In an embodiment, the composition comprises a short acting amylin RA according to formula 1 wherein Z is according to formula D1-3, in particular with R₁ being H and R₂ being NH2, and a short acting GLP-1 RA, such as dasiglucagon.

In an embodiment, the composition comprises a short acting amylin RA according to formula 1 wherein Z is according to formula E1-1, in particular with R₁ being H and R₂ being NH₂, and a short acting GLP-1 RA, such as dasiglucagon.

In an embodiment, the composition comprises a short acting amylin RA according to formula 1 wherein Z is according to formula E1-2, in particular with R₁ being H and R₂ being NH₂, and a short acting GLP-1 RA, such as dasiglucagon.

In an embodiment the composition comprises a short acting amylin RA that has a sequence corresponding to [Glu14,His17,His35]-pramlintide (Cpd 1) and human glucagon.

In an embodiment the composition comprises a short acting amylin RA that has a sequence corresponding to [Glu14,His17,His35]-pramlintide (Cpd 1) and dasiglucagon.

In an embodiment the composition comprises a short acting amylin RA that has a sequence corresponding to [Glu14,Arg17,His35]-pramlintide (Cpd 2) and human glucagon.

In an embodiment the composition comprises a short acting amylin RA that has a sequence corresponding to [Glu14,Arg17,His35]-pramlintide (Cpd 2) and dasiglucagon.

In an embodiment the composition comprises a short acting amylin RA that has a sequence corresponding to [Glu14,His17,Pro37]-pramlintide (Cpd 3) and human glucagon.

In an embodiment the composition comprises a short acting amylin RA that has a sequence corresponding to [Glu14,His17,Pro37]-pramlintide (Cpd 3) and dasiglucagon.

In an embodiment the composition comprises a short acting amylin RA that has a sequence corresponding to [Arg1,Glu14,His17,Pro37]-pramlintide (Cpd 4) and human glucagon.

In an embodiment the composition comprises a short acting amylin RA that has a sequence corresponding to [Arg1,Glu14,His17,Pro37]-pramlintide (Cpd 4) and dasiglucagon.

In an embodiment the composition comprises a short acting amylin RA that has a sequence corresponding to [Glu14,Arg17,Pro37]-pramlintide (Cpd 5) and human glucagon.

In an embodiment the composition comprises a short acting amylin RA that has a sequence corresponding to [Glu14,Arg17,Pro37]-pramlintide (Cpd 5) and dasiglucagon.

In an embodiment the composition comprises a short acting amylin RA has the sequence EASELSTAALGRLSAELHELATLPRTETGPESP-NH₂ (SEQ ID NO: 16) (Cpd 6) and human glucagon.

In an embodiment the composition comprises a short acting amylin RA has the sequence EASELSTAALGRLSAELHELATLPRTETGPESP-NH₂ (SEQ ID NO: 16) (Cpd 6) and dasiglucagon.

In an embodiment the composition comprises a short acting amylin RA that has the following sequence RC()NTATC()ATQRLAEFLHRSSF-Gly(Me)-A-Ile(Me)-LSSTEVGSETP-NH₂ (SEQ ID NO: 24) (Cpd 7) and human glucagon.

In an embodiment the composition comprises a short acting amylin RA that has the following sequence RC()NTATC()ATQRLAEFLHRSSF-Gly(Me)-A-Ile(Me)-LSSTEVGSETP-NH₂ (SEQ ID NO: 24) (Cpd 7) and dasiglucagon.

In an embodiment the composition comprises a short acting amylin RA that has the following sequence R-C()NTATC()ATQRLAEFLHRSSNNF-Gly(Me)-A-Ile(Me)-LSSTNVGSNTP -NH₂ (SEQ ID NO: 25) (Cpd 8) and human glucagon.

In an embodiment the composition comprises a short acting amylin RA that has the following sequence R-C()NTATC()ATQRLAEFLHRSSNNF-Gly(Me)-A-Ile(Me)-LSSTNVGSNTP-NH₂ (SEQ ID NO: 25) (Cpd 8) and dasiglucagon.

In an embodiment the composition comprises a short acting amylin RA that has the following sequence R-C()-GTAT-C()-ATERLAHFLQRSSF-Gly(Me)-A-Ile(Me)-LSSTEVGSNT-Hyp-NH₂ (SEQ ID NO: 29) (Cpd 9) and human glucagon.

In an embodiment the composition comprises a short acting amylin RA that has the following sequence, RD()GTATK()ATQRLADFLQRSSF-Gly(Me)-A-Ile(Me)-LSSTE-VGSNT-Hyp-NH₂ (SEQ ID NO: 32) (Cpd 10) and human glucagon.

In an embodiment the composition comprises a short acting amylin RA that has the following sequence, RD()GTATK()ATERLA-Aad-FLQRSSF-Gly(Me)-A-Ile(Me)-LSSTEVGSNT-Hyp-NH2 (SEQ ID NO: 33) (Cpd 11) and human glucagon.

In an embodiment the composition comprises a short acting amylin RA that has the following sequence, RD()GTATK()ATERLA-Aad-FLQRTTF-Gly(Me)-A-Ile(Me)-LSSTEVGSNT-Hyp-NH₂ (SEQ ID NO: 34) (Cpd 12) and human glucagon.

In an embodiment the composition comprises a short acting amylin RA that has the following sequence, RD()GTATK()ATERLAHFLQRSSF-Gly(Me)-AIle(Me)-LSSTE-VGSNT-Hyp-NH₂ (SEQ ID NO: 35) (Cpd 13) and human glucagon.

In an embodiment the composition comprises a short acting amylin RA that has the following sequence, KD()GTATK()ATQRLA-Aad-FLQRSSF-Gly(Me)-A-Ile(Me)-LSSTEVGSNTHyp-NH₂ (SEQ ID NO: 36) (Cpd 14) and human glucagon.

In an embodiment the composition comprises a short acting amylin RA that has the following sequence R-C()-GTAT-C()-ATERLAHFLQRSSF-Gly(Me)-A-Ile(Me)-LSSTE-VGSNT-Hyp-NH₂ (SEQ ID NO: 29) (Cpd 9) and dasiglucagon.

In an embodiment the composition comprises a short acting amylin RA that has the following sequence, RD()GTATK()ATQRLADFLQRSSF-Gly(Me)-A-Ile(Me)-LSSTEVGSNT-Hyp-NH₂ (SEQ ID NO: 32) (Cpd 10) and dasiglucagon.

In an embodiment the composition comprises a short acting amylin RA that has the following sequence, RD()GTATK()ATERLA-Aad-FLQRSSF-Gly(Me)-A-Ile(Me)-LSSTEVGSNT-Hyp-NH₂ (SEQ ID NO: 33) (Cpd 11) and dasiglucagon.

In an embodiment the composition comprises a short acting amylin RA that has the following sequence, RD()GTATK()ATERLA-Aad-FLQRTTF-Gly(Me)-A-Ile(Me)-LSSTE-VGSNT-Hyp-NH2 (SEQ ID NO: 34) (Cpd 12) and dasiglucagon.

In an embodiment the composition comprises a short acting amylin RA that has the following sequence, RD()GTATK()ATERLAHFLQRSSF-Gly(Me)-AIle(Me)-LSSTEVGSNTHyp-NH₂ (SEQ ID NO: 35) (Cpd 13) and dasiglucagon.

In an embodiment the composition comprises a short acting amylin RA that has the following sequence, KD()GTATK()ATQRLA-Aad-FLQRSSF-Gly(Me)-A-Ile(Me)-LSSTEVGSNTHyp-NH₂ (SEQ ID NO: 36) (Cpd 14) and dasiglucagon.

In an embodiment, the composition comprises a short acting amylin RA according to formula 1 wherein Z is according to formula A1, in particular with R₁ being H and R₂ being NH₂, a short acting GLP-1 RA, such as exenatide and a short acting glucagon RA, such as human glucagon.

In an embodiment, the composition comprises a short acting amylin RA according to formula 1 wherein Z is according to formula B1, in particular with R₁ being H and R₂ being NH₂, a short acting GLP-1 RA, such as exenatide and a short acting glucagon RA, such as human glucagon.

In an embodiment, the composition comprises a short acting amylin RA according to formula 1 wherein Z is according to formula C1, in particular with R₁ being H and R₂ being NH₂, a short acting GLP-1 RA, such as exenatide and a short acting glucagon RA, such as human glucagon.

In an embodiment, the composition comprises a short acting amylin RA according to formula 1 wherein Z is according to formula D1, in particular with R₁ being H and R₂ being NH₂, a short acting GLP-1 RA, such as exenatide and a short acting glucagon RA such as human glucagon.

In an embodiment, the composition comprises a short acting amylin RA according to formula 1 wherein Z is according to formula E1, in particular with R₁ being H and R₂ being NH₂, a short acting GLP-1 RA, such as exenatide and a short acting glucagon RA such as human glucagon.

In an embodiment, the composition comprises a short acting amylin RA according to formula 1 wherein Z is according to formula A1, in particular with R₁ being H and R₂ being NH₂, a short acting GLP-1 RA, such as exenatide and a short acting glucagon RA, such as dasiglucagon.

In an embodiment, the composition comprises a short acting amylin RA according to formula 1 wherein Z is according to formula B1, in particular with R₁ being H and R₂ being NH₂, a short acting GLP-1 RA, such as exenatide and a short acting glucagon RA, such as dasiglucagon.

In an embodiment, the composition comprises a short acting amylin RA according to formula 1 wherein Z is according to formula C1, in particular with R₁ being H and R₂ being NH₂, a short acting GLP-1 RA, such as exenatide and a short acting glucagon RA, such as dasiglucagon.

In an embodiment, the composition comprises a short acting amylin RA according to formula 1 wherein Z is according to formula D1, in particular with R₁ being H and R₂ being NH₂, a short acting GLP-1 RA, such as exenatide and a short acting glucagon RA, such as dasiglucagon.

In an embodiment, the composition comprises a short acting amylin RA according to formula 1 wherein Z is according to formula E1, in particular with R₁ being H and R₂ being NH₂, a short acting GLP-1 RA, such as exenatide and a short acting glucagon RA, such as dasiglucagon.

In an embodiment, the composition comprises a short acting amylin RA according to formula 1 wherein Z is according to formula A1, in particular with R₁ being H and R₂ being NH₂, a short acting GLP-1 RA, such as lixisenatide and a short acting glucagon RA such as human glucagon.

In an embodiment, the composition comprises a short acting amylin RA according to formula 1 wherein Z is according to formula B1, in particular with R₁ being H and R₂ being NH₂, a short acting GLP-1 RA, such as lixisenatide and a short acting glucagon RA such as human glucagon.

In an embodiment, the composition comprises a short acting amylin RA according to formula 1 wherein Z is according to formula C1, in particular with R₁ being H and R₂ being NH₂, a short acting GLP-1 RA, such as lixisenatide and a short acting glucagon RA such as human glucagon.

In an embodiment, the composition comprises a short acting amylin RA according to formula 1 wherein Z is according to formula D1, in particular with R₁ being H and R₂ being NH₂, a short acting GLP-1 RA, such as lixisenatide and a short acting glucagon RA such as human glucagon.

In an embodiment, the composition comprises a short acting amylin RA according to formula 1 wherein Z is according to formula E1, in particular with R₁ being H and R₂ being NH₂, a short acting GLP-1 RA, such as lixisenatide and a short acting glucagon RA such as human glucagon.

In an embodiment, the composition comprises a short acting amylin RA according to formula 1 wherein Z is according to formula A1, in particular with R₁ being H and R₂ being NH₂, a short acting GLP-1 RA, such as lixisenatide and a short acting glucagon RA such as dasiglucagon.

In an embodiment, the composition comprises a short acting amylin RA according to formula 1 wherein Z is according to formula B1, in particular with R₁ being H and R₂ being NH₂, a short acting GLP-1 RA, such as lixisenatide and a short acting glucagon RA such as dasiglucagon.

In an embodiment, the composition comprises a short acting amylin RA according to formula 1 wherein Z is according to formula C1, in particular with R₁ being H and R₂ being NH₂, a short acting GLP-1 RA, such as lixisenatide and a short acting glucagon RA such as dasiglucagon.

In an embodiment, the composition comprises a short acting amylin RA according to formula 1 wherein Z is according to formula D1, in particular with R₁ being H and R₂ being NH₂, a short acting GLP-1 RA, such as lixisenatide and a short acting glucagon RA such as dasiglucagon.

In an embodiment, the composition comprises a short acting amylin RA according to formula 1 wherein Z is according to formula E1, in particular with R₁ being H and R₂ being NH₂, a short acting GLP-1 RA, such as lixisenatide and a short acting glucagon RA such as dasiglucagon.

In an embodiment, the composition comprises a short acting amylin RA according to formula 1 wherein Z is according to formula A1-1, in particular with R₁ being H and R₂ being NH₂, a short acting GLP-1 RA, such as exenatide and a short acting glucagon RA such as human glucagon.

In an embodiment, the composition comprises a short acting amylin RA according to formula 1 wherein Z is according to formula A1-2, in particular with R₁ being H and R₂ being NH₂, and a short acting GLP-1 RA, such as exenatide, a short acting glucagon RA such as human glucagon.

In an embodiment, the composition comprises a short acting amylin RA according to formula 1 wherein Z is according to formula A1-3, in particular with R₁ being H and R₂ being NH₂, and a short acting GLP-1 RA, such as exenatide, a short acting glucagon RA such as human glucagon.

In an embodiment, the composition comprises a short acting amylin RA according to formula 1 wherein Z is according to formula A1-4, in particular with R₁ being H and R₂ being NH₂, and a short acting GLP-1 RA, such as exenatide, a short acting glucagon RA such as human glucagon.

In an embodiment, the composition comprises a short acting amylin RA according to formula 1 wherein Z is according to formula B1-1, in particular with R₁ being H and R₂ being NH₂, and a short acting GLP-1 RA, such as exenatide, a short acting glucagon RA such as human glucagon.

In an embodiment, the composition comprises a short acting amylin RA according to formula 1 wherein Z is according to formula B1-2, in particular with R₁ being H and R₂ being NH₂, and a short acting GLP-1 RA, such as exenatide, a short acting glucagon RA such as human glucagon.

In an embodiment, the composition comprises a short acting amylin RA according to formula 1 wherein Z is according to formula B1-3, in particular with R₁ being H and R₂ being NH₂, and a short acting GLP-1 RA, such as exenatide, a short acting glucagon RA such as human glucagon.

In an embodiment, the composition comprises a short acting amylin RA according to formula 1 wherein Z is according to formula C1-1, in particular with R₁ being H and R₂ being NH₂, and a short acting GLP-1 RA, such as exenatide, a short acting glucagon RA such as human glucagon.

In an embodiment, the composition comprises a short acting amylin RA according to formula 1 wherein Z is according to formula C1-2, in particular with R₁ being H and R₂ being NH₂, and a short acting GLP-1 RA, such as exenatide, a short acting glucagon RA such as human glucagon.

In an embodiment, the composition comprises a short acting amylin RA according to formula 1 wherein Z is according to formula C1-3, in particular with R₁ being H and R2 being NH₂, and a short acting GLP-1 RA, such as exenatide, a short acting glucagon RA such as human glucagon.

In an embodiment, the composition comprises a short acting amylin RA according to formula 1 wherein Z is according to formula C1-4, in particular with R1 being H and R₂ being NH₂, and a short acting GLP-1 RA, such as exenatide, a short acting glucagon RA such as human glucagon.

In an embodiment, the composition comprises a short acting amylin RA according to formula 1 wherein Z is according to formula C1-5, in particular with R₁ being H and R₂ being NH₂, and a short acting GLP-1 RA, such as exenatide, a short acting glucagon RA such as human glucagon.

In an embodiment, the composition comprises a short acting amylin RA according to formula 1 wherein Z is according to formula D1-1, in particular with R₁ being H and R₂ being NH₂, and a short acting GLP-1 RA, such as exenatide, a short acting glucagon RA such as human glucagon.

In an embodiment, the composition comprises a short acting amylin RA according to formula 1 wherein Z is according to formula D1-2, in particular with R₁ being H and R₂ being NH₂, and a short acting GLP-1 RA, such as exenatide, a short acting glucagon RA such as human glucagon.

In an embodiment, the composition comprises a short acting amylin RA according to formula 1 wherein Z is according to formula D1-3, in particular with R₁ being H and R₂ being NH₂, and a short acting GLP-1 RA, such as exenatide, a short acting glucagon RA such as human glucagon.

In an embodiment, the composition comprises a short acting amylin RA according to formula 1 wherein Z is according to formula E1-1, in particular with R₁ being H and R₂ being NH₂, and a short acting GLP-1 RA, such as exenatide, a short acting glucagon RA such as human glucagon.

In an embodiment, the composition comprises a short acting amylin RA according to formula 1 wherein Z is according to formula E1-2, in particular with R₁ being H and R₂ being NH₂, and a short acting GLP-1 RA, such as exenatide, a short acting glucagon RA such as human glucagon.

In an embodiment, the composition comprises a short acting amylin RA according to formula 1 wherein Z is according to formula A1-1, in particular with R₁ being H and R₂ being NH₂, a short acting GLP-1 RA, such as exenatide and a short acting glucagon RA such as dasiglucagon.

In an embodiment, the composition comprises a short acting amylin RA according to formula 1 wherein Z is according to formula A1-2, in particular with R₁ being H and R₂ being NH₂, and a short acting GLP-1 RA, such as exenatide, a short acting glucagon RA such as dasiglucagon.

In an embodiment, the composition comprises a short acting amylin RA according to formula 1 wherein Z is according to formula A1-3, in particular with R₁ being H and R₂ being NH₂, and a short acting GLP-1 RA, such as exenatide, a short acting glucagon RA such as dasiglucagon.

In an embodiment, the composition comprises a short acting amylin RA according to formula 1 wherein Z is according to formula A1-4, in particular with R₁ being H and R₂ being NH₂, and a short acting GLP-1 RA, such as exenatide, a short acting glucagon RA such as dasiglucagon.

In an embodiment, the composition comprises a short acting amylin RA according to formula 1 wherein Z is according to formula B1-1, in particular with R₁ being H and R₂ being NH₂, and a short acting GLP-1 RA, such as exenatide, a short acting glucagon RA such as dasiglucagon.

In an embodiment, the composition comprises a short acting amylin RA according to formula 1 wherein Z is according to formula B1-2, in particular with R₁ being H and R₂ being NH₂, and a short acting GLP-1 RA, such as exenatide, a short acting glucagon RA such as dasiglucagon.

In an embodiment, the composition comprises a short acting amylin RA according to formula 1 wherein Z is according to formula B1-3, in particular with R₁ being H and R₂ being NH₂, and a short acting GLP-1 RA, such as exenatide, a short acting glucagon RA such as dasiglucagon.

In an embodiment, the composition comprises a short acting amylin RA according to formula 1 wherein Z is according to formula C1-1, in particular with R₁ being H and R₂ being NH₂, and a short acting GLP-1 RA, such as exenatide, a short acting glucagon RA such as dasiglucagon.

In an embodiment, the composition comprises a short acting amylin RA according to formula 1 wherein Z is according to formula C1-2, in particular with R₁ being H and R₂ being NH₂, and a short acting GLP-1 RA, such as exenatide, a short acting glucagon RA such as dasiglucagon.

In an embodiment, the composition comprises a short acting amylin RA according to formula 1 wherein Z is according to formula C1-3, in particular with R₁ being H and R2 being NH₂, and a short acting GLP-1 RA, such as exenatide, a short acting glucagon RA such as dasiglucagon.

In an embodiment, the composition comprises a short acting amylin RA according to formula 1 wherein Z is according to formula C1-4, in particular with R1 being H and R₂ being NH₂, and a short acting GLP-1 RA, such as exenatide, a short acting glucagon RA such as dasiglucagon.

In an embodiment, the composition comprises a short acting amylin RA according to formula 1 wherein Z is according to formula C1-5, in particular with R₁ being H and R₂ being NH₂, and a short acting GLP-1 RA, such as exenatide, a short acting glucagon RA such as dasiglucagon.

In an embodiment, the composition comprises a short acting amylin RA according to formula 1 wherein Z is according to formula D1-1, in particular with R₁ being H and R₂ being NH₂, and a short acting GLP-1 RA, such as exenatide, a short acting glucagon RA such as dasiglucagon.

In an embodiment, the composition comprises a short acting amylin RA according to formula 1 wherein Z is according to formula D1-2, in particular with R₁ being H and R₂ being NH₂, and a short acting GLP-1 RA, such as exenatide, a short acting glucagon RA such as dasiglucagon.

In an embodiment, the composition comprises a short acting amylin RA according to formula 1 wherein Z is according to formula D1-3, in particular with R₁ being H and R₂ being NH₂, and a short acting GLP-1 RA, such as exenatide, a short acting glucagon RA such as dasiglucagon.

In an embodiment, the composition comprises a short acting amylin RA according to formula 1 wherein Z is according to formula E1-1, in particular with R₁ being H and R₂ being NH₂, and a short acting GLP-1 RA, such as exenatide, a short acting glucagon RA such as dasiglucagon.

In an embodiment, the composition comprises a short acting amylin RA according to formula 1 wherein Z is according to formula E1-2, in particular with R₁ being H and R₂ being NH₂, and a short acting GLP-1 RA, such as exenatide, a short acting glucagon RA such as dasiglucagon.

In an embodiment, the composition comprises a short acting amylin RA according to formula 1 wherein Z is according to formula A1-1, in particular with R₁ being H and R₂ being NH₂, and a short acting GLP-1 RA, such as lixisenatide, a short acting glucagon RA such as human glucagon.

In an embodiment, the composition comprises a short acting amylin RA according to formula 1 wherein Z is according to formula A1-2, in particular with R₁ being H and R₂ being NH₂, and a short acting GLP-1 RA, such as lixisenatide, a short acting glucagon RA such as human glucagon.

In an embodiment, the composition comprises a short acting amylin RA according to formula 1 wherein Z is according to formula A1-3, in particular with R₁ being H and R₂ being NH₂, and a short acting GLP-1 RA, such as lixisenatide, a short acting glucagon RA such as human glucagon.

In an embodiment, the composition comprises a short acting amylin RA according to formula 1 wherein Z is according to formula A1-4, in particular with R₁ being H and R₂ being NH₂, and a short acting GLP-1 RA, such as lixisenatide, a short acting glucagon RA such as human glucagon.

In an embodiment, the composition comprises a short acting amylin RA according to formula 1 wherein Z is according to formula B1-1, in particular with R₁ being H and R₂ being NH₂, and a short acting GLP-1 RA, such as lixisenatide, a short acting glucagon RA such as human glucagon.

In an embodiment, the composition comprises a short acting amylin RA according to formula 1 wherein Z is according to formula B1-2, in particular with R₁ being H and R₂ being NH₂, and a short acting GLP-1 RA, such as lixisenatide, a short acting glucagon RA such as human glucagon.

In an embodiment, the composition comprises a short acting amylin RA according to formula 1 wherein Z is according to formula B1-3, in particular with R₁ being H and R₂ being NH₂, and a short acting GLP-1 RA, such as lixisenatide, a short acting glucagon RA such as human glucagon.

In an embodiment, the composition comprises a short acting amylin RA according to formula 1 wherein Z is according to formula C1-1, in particular with R₁ being H and R₂ being NH₂, and a short acting GLP-1 RA, such as lixisenatide, a short acting glucagon RA such as human glucagon.

In an embodiment, the composition comprises a short acting amylin RA according to formula 1 wherein Z is according to formula C1-2, in particular with R₁ being H and R₂ being NH₂, and a short acting GLP-1 RA, such as lixisenatide, a short acting glucagon RA such as human glucagon.

In an embodiment, the composition comprises a short acting amylin RA according to formula 1 wherein Z is according to formula C1-3, in particular with R₁ being H and R₂ being NH₂, and a short acting GLP-1 RA, such as lixisenatide, a short acting glucagon RA such as human glucagon.

In an embodiment, the composition comprises a short acting amylin RA according to formula 1 wherein Z is according to formula C1-4, in particular with R₁ being H and R₂ being NH₂, and a short acting GLP-1 RA such as lixisenatide, a short acting glucagon RA such as human glucagon.

In an embodiment, the composition comprises a short acting amylin RA according to formula 1 wherein Z is according to formula C1-5, in particular with R₁ being H and R₂ being NH₂, and a short acting GLP-1 RA, such as lixisenatide, a short acting glucagon RA such as human glucagon.

In an embodiment, the composition comprises a short acting amylin RA according to formula 1 wherein Z is according to formula D1-1, in particular with R₁ being H and R₂ being NH₂, and a short acting GLP-1 RA, such as lixisenatide, a short acting glucagon RA such as human glucagon.

In an embodiment, the composition comprises a short acting amylin RA according to formula 1 wherein Z is according to formula D1-2, in particular with R₁ being H and R₂ being NH₂, and a short acting GLP-1 RA, such as lixisenatide, a short acting glucagon RA such as human glucagon.

In an embodiment, the composition comprises a short acting amylin RA according to formula 1 wherein Z is according to formula D1-3, in particular with R₁ being H and R₂ being NH2, and a short acting GLP-1 RA, such as lixisenatide, a short acting glucagon RA such as human glucagon.

In an embodiment, the composition comprises a short acting amylin RA according to formula 1 wherein Z is according to formula E1-1, in particular with R₁ being H and R₂ being NH₂, and a short acting GLP-1 RA, such as lixisenatide, a short acting glucagon RA such as human glucagon.

In an embodiment, the composition comprises a short acting amylin RA according to formula 1 wherein Z is according to formula E1-2, in particular with R₁ being H and R₂ being NH₂, and a short acting GLP-1 RA, such as lixisenatide, a short acting glucagon RA such as human glucagon.

In an embodiment, the composition comprises a short acting amylin RA according to formula 1 wherein Z is according to formula A1-1, in particular with R₁ being H and R₂ being NH₂, and a short acting GLP-1 RA, such as lixisenatide, a short acting glucagon RA such as dasiglucagon.

In an embodiment, the composition comprises a short acting amylin RA according to formula 1 wherein Z is according to formula A1-2, in particular with R₁ being H and R₂ being NH₂, and a short acting GLP-1 RA, such as lixisenatide, a short acting glucagon RA such as dasiglucagon.

In an embodiment, the composition comprises a short acting amylin RA according to formula 1 wherein Z is according to formula A1-3, in particular with R₁ being H and R₂ being NH₂, and a short acting GLP-1 RA, such as lixisenatide, a short acting glucagon RA such as dasiglucagon.

In an embodiment, the composition comprises a short acting amylin RA according to formula 1 wherein Z is according to formula A1-4, in particular with R₁ being H and R₂ being NH₂, and a short acting GLP-1 RA, such as lixisenatide, a short acting glucagon RA such as dasiglucagon.

In an embodiment, the composition comprises a short acting amylin RA according to formula 1 wherein Z is according to formula B1-1, in particular with R₁ being H and R₂ being NH₂, and a short acting GLP-1 RA, such as lixisenatide, a short acting glucagon RA such as dasiglucagon.

In an embodiment, the composition comprises a short acting amylin RA according to formula 1 wherein Z is according to formula B1-2, in particular with R₁ being H and R₂ being NH₂, and a short acting GLP-1 RA, such as lixisenatide, a short acting glucagon RA such as dasiglucagon.

In an embodiment, the composition comprises a short acting amylin RA according to formula 1 wherein Z is according to formula B1-3, in particular with R₁ being H and R₂ being NH₂, and a short acting GLP-1 RA, such as lixisenatide, a short acting glucagon RA such as dasiglucagon.

In an embodiment, the composition comprises a short acting amylin RA according to formula 1 wherein Z is according to formula C1-1, in particular with R₁ being H and R₂ being NH₂, and a short acting GLP-1 RA, such as lixisenatide, a short acting glucagon RA such as dasiglucagon.

In an embodiment, the composition comprises a short acting amylin RA according to formula 1 wherein Z is according to formula C1-2, in particular with R₁ being H and R₂ being NH₂, and a short acting GLP-1 RA, such as lixisenatide, a short acting glucagon RA such as dasiglucagon.

In an embodiment, the composition comprises a short acting amylin RA according to formula 1 wherein Z is according to formula C1-3, in particular with R₁ being H and R₂ being NH₂, and a short acting GLP-1 RA, such as lixisenatide, a short acting glucagon RA such as dasiglucagon.

In an embodiment, the composition comprises a short acting amylin RA according to formula 1 wherein Z is according to formula C1-4, in particular with R₁ being H and R₂ being NH₂, and a short acting GLP-1 RA such as lixisenatide, a short acting glucagon RA such as dasiglucagon.

In an embodiment, the composition comprises a short acting amylin RA according to formula 1 wherein Z is according to formula C1-5, in particular with R₁ being H and R₂ being NH₂, and a short acting GLP-1 RA, such as lixisenatide, a short acting glucagon RA such as dasiglucagon.

In an embodiment, the composition comprises a short acting amylin RA according to formula 1 wherein Z is according to formula D1-1, in particular with R₁ being H and R₂ being NH₂, and a short acting GLP-1 RA, such as lixisenatide, a short acting glucagon RA such as dasiglucagon.

In an embodiment, the composition comprises a short acting amylin RA according to formula 1 wherein Z is according to formula D1-2, in particular with R₁ being H and R₂ being NH₂, and a short acting GLP-1 RA, such as lixisenatide, a short acting glucagon RA such as dasiglucagon.

In an embodiment, the composition comprises a short acting amylin RA according to formula 1 wherein Z is according to formula D1-3, in particular with R₁ being H and R₂ being NH2, and a short acting GLP-1 RA, such as lixisenatide, a short acting glucagon RA such as dasiglucagon.

In an embodiment, the composition comprises a short acting amylin RA according to formula 1 wherein Z is according to formula E1-1, in particular with R₁ being H and R₂ being NH₂, and a short acting GLP-1 RA, such as lixisenatide, a short acting glucagon RA such as dasiglucagon.

In an embodiment, the composition comprises a short acting amylin RA according to formula 1 wherein Z is according to formula E1-2, in particular with R₁ being H and R₂ being NH₂, and a short acting GLP-1 RA, such as lixisenatide, a short acting glucagon RA such as dasiglucagon.

In an embodiment the composition comprises a short acting amylin RA that has a sequence corresponding to [Glu14,His17,His35]-pramlintide (Cpd 1), exenatide and human glucagon.

In an embodiment the composition comprises a short acting amylin RA that has a sequence corresponding to [Glu14,His17,His35]-pramlintide (Cpd 1), exenatide and dasiglucagon.

In an embodiment the composition comprises a short acting amylin RA that has a sequence corresponding to [Glu14,His17,His35]-pramlintide (Cpd 1), lixisenatide and human glucagon.

In an embodiment the composition comprises a short acting amylin RA that has a sequence corresponding to [Glu14,His17,His35]-pramlintide (Cpd 1), lixisenatide and dasiglucagon.

In an embodiment the composition comprises a short acting amylin RA that has a sequence corresponding to [Glu14,Arg17,His35]-pramlintide (Cpd 2), exenatide and human glucagon.

In an embodiment the composition comprises a short acting amylin RA that has a sequence corresponding to [Glu14,Arg17,His35]-pramlintide (Cpd 2), exenatide and dasiglucagon

In an embodiment the composition comprises a short acting amylin RA that has a sequence corresponding to [Glu14,Arg17,His35]-pramlintide (Cpd 2), lixisenatide and human glucagon.

In an embodiment the composition comprises a short acting amylin RA that has a sequence corresponding to [Glu14,Arg17,His35]-pramlintide (Cpd 2), lixisenatide and dasiglucagon.

In an embodiment the composition comprises a short acting amylin RA that has a sequence corresponding to [Glu14,His17,Pro37]-pramlintide (Cpd 3), exenatide and human glucagon.

In an embodiment the composition comprises a short acting amylin RA that has a sequence corresponding to [Glu14,His17,Pro37]-pramlintide (Cpd 3), exenatide and dasiglucagon.

In an embodiment the composition comprises a short acting amylin RA that has a sequence corresponding to [Glu14,His17,Pro37]-pramlintide (Cpd 3), lixisenatide and human glucagon.

In an embodiment the composition comprises a short acting amylin RA that has a sequence corresponding to [Glu14,His17,Pro37]-pramlintide (Cpd 3), lixisenatide and dasiglucagon.

In an embodiment the composition comprises a short acting amylin RA that has a sequence corresponding to [Arg1,Glu14,His17,Pro37]-pramlintide (Cpd 4), exenatide and human glucagon.

In an embodiment the composition comprises a short acting amylin RA that has a sequence corresponding to [Arg1,Glu14,His17,Pro37]-pramlintide (Cpd 4), exenatide and dasiglucagon.

In an embodiment the composition comprises a short acting amylin RA that has a sequence corresponding to [Arg1,Glu14,His17,Pro37]-pramlintide (Cpd 4), lixisenatide and human glucagon.

In an embodiment the composition comprises a short acting amylin RA that has a sequence corresponding to [Arg1,Glu14,His17,Pro37]-pramlintide (Cpd 4), lixisenatide and dasiglucagon.

In an embodiment the composition comprises a short acting amylin RA that has a sequence corresponding to [Glu14,Arg17,Pro37]-pramlintide (Cpd 5), exenatide and human glucagon.

In an embodiment the composition comprises a short acting amylin RA that has a sequence corresponding to [Glu14,Arg17,Pro37]-pramlintide (Cpd 5), exenatide and dasiglucagon.

In an embodiment the composition comprises a short acting amylin RA that has a sequence corresponding to [Glu14,Arg17,Pro37]-pramlintide (Cpd 5), lixisenatide and human glucagon.

In an embodiment the composition comprises a short acting amylin RA that has a sequence corresponding to [Glu14,Arg17,Pro37]-pramlintide (Cpd 5), lixisenatide and dasiglucagon.

In an embodiment the composition comprises a short acting amylin RA has the sequence EASELSTAALGRLSAELHELATLPRTETGPESP-NH₂ (SEQ ID NO: 16) (Cpd 6), exenatide and human glucagon.

In an embodiment the composition comprises a short acting amylin RA has the sequence EASELSTAALGRLSAELHELATLPRTETGPESP-NH₂ (SEQ ID NO: 16) (Cpd 6), exenatide and dasiglucagon.

In an embodiment the composition comprises a short acting amylin RA has the sequence EASELSTAALGRLSAELHELATLPRTETGPESP-NH₂ (SEQ ID NO: 16) (Cpd 6), lixisenatide and human glucagon.

In an embodiment the composition comprises a short acting amylin RA has the sequence EASELSTAALGRLSAELHELATLPRTETGPESP-NH₂ (SEQ ID NO: 16) (Cpd 6), lixisenatide and dasiglucagon.

In an embodiment the composition comprises a short acting amylin RA that has the following sequence RC()NTATC()ATQRLAEFLHRSSF-Gly(Me)-A-Ile(Me)-LSSTEVGSETP-NH₂ (SEQ ID NO: 24) (Cpd 7), exenatide and human glucagon.

In an embodiment the composition comprises a short acting amylin RA that has the following sequence RC()NTATC()ATQRLAEFLHRSSF-Gly(Me)-A-Ile(Me)-LSSTEVGSETP-NH₂ (SEQ ID NO: 24) (Cpd 7), exenatide and dasiglucagon.

In an embodiment the composition comprises a short acting amylin RA that has the following sequence RC()NTATC()ATQRLAEFLHRSSF-Gly(Me)-A-Ile(Me)-LSSTEVGSETP-NH₂ (SEQ ID NO: 24) (Cpd 7), lixisenatide and human glucagon.

In an embodiment the composition comprises a short acting amylin RA that has the following sequence RC()NTATC()ATQRLAEFLHRSSF-Gly(Me)-A-Ile(Me)-LSSTEVGSETP-NH₂ (SEQ ID NO: 24) (Cpd 7), lixisenatide and dasiglucagon.

In an embodiment the composition comprises a short acting amylin RA that has the following sequence R-C()NTATC()ATQRLAEFLHRSSNNF-Gly(Me)-A-Ile(Me)-LSSTNVGSNTP -NH₂ (SEQ ID NO: 25) (Cpd 8), exenatide and human glucagon.

In an embodiment the composition comprises a short acting amylin RA that has the following sequence R-C()NTATC()ATQRLAEFLHRSSNNF-Gly(Me)-A-Ile(Me)-LSSTNVGSNTP -NH₂ (SEQ ID NO: 25) (Cpd 8), exenatide and dasiglucagon.

In an embodiment the composition comprises a short acting amylin RA that has the following sequence R-C()NTATC()ATQRLAEFLHRSSNNF-Gly(Me)-A-Ile(Me)-LSSTNVGSNTP -NH₂ (SEQ ID NO: 25) (Cpd 8), lixisenatide and human glucagon.

In an embodiment the composition comprises a short acting amylin RA that has the following sequence R-C()NTATC()ATQRLAEFLHRSSNNF-Gly(Me)-A-Ile(Me)-LSSTNVGSNTP -NH₂ (SEQ ID NO: 25) (Cpd 8), lixisenatide and dasiglucagon.

In an embodiment the composition comprises a short acting amylin RA that has the following sequence R-C()-GTAT-C()-ATERLAHFLQRSSF-Gly(Me)-A-Ile(Me)-LSSTEVGSNT-Hyp-NH₂ (SEQ ID NO: 29) (Cpd 9), exenatide and human glucagon.

In an embodiment the composition comprises a short acting amylin RA that has the following sequence R-C()-GTAT-C()-ATERLAHFLQRSSF-Gly(Me)-A-Ile(Me)-LSSTEVGSNT-Hyp-NH₂ (SEQ ID NO: 29) (Cpd 9), exenatide and dasiglucagon.

In an embodiment the composition comprises a short acting amylin RA that has the following sequence, RD()GTATK()ATQRLADFLQRSSF-Gly(Me)-A-Ile(Me)-LSSTE-VGSNT-Hyp-NH₂ (SEQ ID NO: 32) (Cpd 10), exenatide and human glucagon.

In an embodiment the composition comprises a short acting amylin RA that has the following sequence, RD()GTATK()ATQRLADFLQRSSF-Gly(Me)-A-Ile(Me)-LSSTE-VGSNT-Hyp-NH₂ (SEQ ID NO: 32) (Cpd 10), exenatide and dasiglucagon.

In an embodiment the composition comprises a short acting amylin RA that has the following sequence, RD()GTATK()ATERLA-Aad-FLQRSSF-Gly(Me)-A-Ile(Me)-LSSTEVGSNT-Hyp-NH2 (SEQ ID NO: 33) (Cpd 11), exenatide and human glucagon.

In an embodiment the composition comprises a short acting amylin RA that has the following sequence, RD()GTATK()ATERLA-Aad-FLQRSSF-Gly(Me)-A-Ile(Me)-LSSTEVGSNT-Hyp-NH2 (SEQ ID NO: 33) (Cpd 11), exenatide and dasiglucagon.

In an embodiment the composition comprises a short acting amylin RA that has the following sequence, RD()GTATK()ATERLA-Aad-FLQRTTF-Gly(Me)-A-Ile(Me)-LSSTEVGSNT-Hyp-NH₂ (SEQ ID NO: 34) (Cpd 12), exenatide and human glucagon.

In an embodiment the composition comprises a short acting amylin RA that has the following sequence, RD()GTATK()ATERLA-Aad-FLQRTTF-Gly(Me)-A-Ile(Me)-LSSTEVGSNT-Hyp-NH₂ (SEQ ID NO: 34) (Cpd 12), exenatide and dasiglucagon.

In an embodiment the composition comprises a short acting amylin RA that has the following sequence, RD()GTATK()ATERLAHFLQRSSF-Gly(Me)-AIle(Me)-LSSTE-VGSNT-Hyp-NH₂ (SEQ ID NO: 35) (Cpd 13), exenatide and human glucagon.

In an embodiment the composition comprises a short acting amylin RA that has the following sequence, RD()GTATK()ATERLAHFLQRSSF-Gly(Me)-AIle(Me)-LSSTE-VGSNT-Hyp-NH₂ (SEQ ID NO: 35) (Cpd 13), exenatide and dasiglucagon.

In an embodiment the composition comprises a short acting amylin RA that has the following sequence, KD()GTATK()ATQRLA-Aad-FLQRSSF-Gly(Me)-A-Ile(Me)-LSSTEVGSNTHyp-NH₂ (SEQ ID NO: 36) (Cpd 14), exenatide and human glucagon.

In an embodiment the composition comprises a short acting amylin RA that has the following sequence, KD()GTATK()ATQRLA-Aad-FLQRSSF-Gly(Me)-A-Ile(Me)-LSSTEVGSNTHyp-NH₂ (SEQ ID NO: 36) (Cpd 14), exenatide and dasiglucagon.

In an embodiment the composition comprises a short acting amylin RA that has the following sequence R-C()-GTAT-C()-ATERLAHFLQRSSF-Gly(Me)-A-Ile(Me)-LSSTE-VGSNT-Hyp-NH₂ (SEQ ID NO: 29) (Cpd 9), lixisenatide and human glucagon.

In an embodiment the composition comprises a short acting amylin RA that has the following sequence R-C()-GTAT-C()-ATERLAHFLQRSSF-Gly(Me)-A-Ile(Me)-LSSTE-VGSNT-Hyp-NH₂ (SEQ ID NO: 29) (Cpd 9), lixisenatide and dasiglucagon.

In an embodiment the composition comprises a short acting amylin RA that has the following sequence, RD()GTATK()ATQRLADFLQRSSF-Gly(Me)-A-Ile(Me)-LSSTEVGSNT-Hyp-NH₂ (SEQ ID NO: 32) (Cpd 10), lixisenatide and human glucagon.

In an embodiment the composition comprises a short acting amylin RA that has the following sequence, RD()GTATK()ATQRLADFLQRSSF-Gly(Me)-A-Ile(Me)-LSSTEVGSNT-Hyp-NH₂ (SEQ ID NO: 32) (Cpd 10), lixisenatide and dasiglucagon.

In an embodiment the composition comprises a short acting amylin RA that has the following sequence, RD()GTATK()ATERLA-Aad-FLQRSSF-Gly(Me)-A-Ile(Me)-LSSTEVGSNT-Hyp-NH₂ (SEQ ID NO: 33) (Cpd 11), lixisenatide and human glucagon.

In an embodiment the composition comprises a short acting amylin RA that has the following sequence, RD()GTATK()ATERLA-Aad-FLQRSSF-Gly(Me)-A-Ile(Me)-LSSTEVGSNT-Hyp-NH₂ (SEQ ID NO: 33) (Cpd 11), lixisenatide and dasiglucagon.

In an embodiment the composition comprises a short acting amylin RA that has the following sequence, RD()GTATK()ATERLA-Aad-FLQRTTF-Gly(Me)-A-Ile(Me)-LSSTE-VGSNT-Hyp-NH2 (SEQ ID NO: 34) (Cpd 12), lixisenatide and human glucagon.

In an embodiment the composition comprises a short acting amylin RA that has the following sequence, RD()GTATK()ATERLA-Aad-FLQRTTF-Gly(Me)-A-Ile(Me)-LSSTE-VGSNT-Hyp-NH2 (SEQ ID NO: 34) (Cpd 12), lixisenatide and dasiglucagon.

In an embodiment the composition comprises a short acting amylin RA that has the following sequence, RD()GTATK()ATERLAHFLQRSSF-Gly(Me)-AIle(Me)-LSSTEVGSNTHyp-NH₂ (SEQ ID NO: 35) (Cpd 13), lixisenatide and human glucagon.

In an embodiment the composition comprises a short acting amylin RA that has the following sequence, RD()GTATK()ATERLAHFLQRSSF-Gly(Me)-AIle(Me)-LSSTEVGSNTHyp-NH₂ (SEQ ID NO: 35) (Cpd 13), lixisenatide and dasiglucagon.

In an embodiment the composition comprises a short acting amylin RA that has the following sequence, KD()GTATK()ATQRLA-Aad-FLQRSSF-Gly(Me)-A-Ile(Me)-LSSTEVGSNTHyp-NH₂ (SEQ ID NO: 36) (Cpd 14), lixisenatide and human glucagon.

In an embodiment the composition comprises a short acting amylin RA that has the following sequence, KD()GTATK()ATQRLA-Aad-FLQRSSF-Gly(Me)-A-Ile(Me)-LSSTEVGSNTHyp-NH₂ (SEQ ID NO: 36) (Cpd 14), lixisenatide and dasiglucagon.

In an embodiment the composition further comprises
- a short-acting Glucose dependant Insulinotropic Polypeptide ("GIP" also known as Gastric Inhibitoy Polypeptide) Polypeptide RA and/or
- a short-acting Peptide Tyrosine Tyrosine (or "PYY") analog.

According to an embodiment, the composition further comprises a short acting GIP RA.

In an embodiment the short-acting GIP RA is chosen amongst the peptides according to Formula 2:

**R'₁-Z'-R'₂** Formula 2

wherein
- R'₁ is a modification of the N-terminal amino group wherein the modification is selected from the group consisting of acetyl and absent
- R'₂ is is absent or a modification of the C-terminal group, wherein the modification is amidation; and
- Z' is an amino acid sequence according to formula A'1 with the following amino acid sequence as set forth in (SEQ ID NO: 41):
   A'1 being: X₁X₂EGTX₆ISDYSIX₁₃LDX₁₆X₁₇X₁₈QX₂₀X₂₁X₂₂VX₂₄X₂₅X₂₆LX₂₈X₂₉GPSSGAPPPS wherein
   - X1 is selected from the group consisting of Y and D-Tyr;
   - X2 is selected from the group consisting of Aib, A, and D-Ala;
   - X6 is selected from the group consisting of F, aMeF, Iva, L, aMeL, and aMeF(2F);
   - X13 is selected from the group consisting of aMeL, A, L, and Aib;
   - X16 is selected from the group consisting of K, E, and Orn;
   - X17 is I
   - X18 is selected from the group consisting of H, A, and R;
   - X20 is selected from the group consisting of Aib and Q;
   - X21 is selected from the group consisting of D and E;
   - X22 is selected from the group consisting of F and aMeF;
   - X24 is selected from the group consisting of E, N, Q, and D-Glu;
   - X25 is selected from the group consisting of Y, 4-Pal, W, and aMeY;
   - X26 is L
   - X28 is selected from the group consisting of E and A;
   - X29 is selected from the group consisting of G, A, Q, and T;
or a pharmaceutically acceptable salt thereof.

In an embodiment the short acting GIP RA is Cpd 15 and has the following sequence: Y-(D-Ala)-EGTFISDYSILLDKIAQ-Aib-DFVNWLLAQGPSSGAPPPS-NH2 (SEQ ID NO: 42).

In an embodiment the short acting GIP RA is Cpd 16 and has the following sequence : Y-Aib-EGTFISDYSI-aMeL-LDKIHQ-Aib-DFVE-4-Pal-LLEAGPSSGAPPPS-NH2 (SEQ ID NO: 43).

In an embodiment the short acting GIP RA is Cpd 17 and has the following sequence : Y-Aib-EGTFISDYSI-aMeL-LD-Orn-IHQ-Aib-DFVE-4-Pal-LLEAGPSSGAPPPS-NH2 (SEQ ID NO: 44).

In an embodiment the short acting GIP RA is Cpd 18 and has the following sequence : Y-Aib-EGTFISDYSI-aMeL-LDKIHQ-Aib-DFVEYLLEGGPSSGAPPPS-NH2 (SEQ ID NO: 45).

In an embodiment the short acting GIP RA is Cpd 19 and has the following sequence : Y-Aib-EGTFISDYSI-aMeL-LD-Orn-IHQ-Aib-DFVEYLLEGGPSSGAPPPS-NH2 (SEQ ID NO: 46).

In an embodiment the short acting GIP RA is Cpd 20 and has the following sequence : Y-Aib-EGTFISDYSI-aMeL-LD-Orn-IHQ-Aib-EFV-(D-Glu)-YLLEGGPSSGAPPPS-NH2 (SEQ ID NO: 47).

In an embodiment the short acting GIP RA is chosen amongst the peptides according to Formula 3:

**R"₁-Z"-R"₂** Formula 3

wherein
- R"₁ is H, Ac or pGlu pyroglutamic acid (pGlu; (S)-(-)-2-pyrrolidone-5-carboxylic acid), C1-4 alkyl, acetyl, formyl, benzoyl and trifluoroacetyl,
- R"₂ is is -NH2 or -OH; and
- Z" is an amino acid sequence according to Formula B'1 with the following amino acid sequence as set forth in (SEQ ID NO: 48): Tyr-X₂-Glu-Gly-Thr-Phe-Ile-Ser-Asp-X₁₀-X₁₁ -X₁₂-Glu-Leu-X₁₅-X₁₆-X₁₇-X₁₈-X₁₉-X₂₀-X₂₁-Phe-X₂₃-X₂₄-X₂₅-Leu-X₂₇-X₂₈-X₂₉-X₃₀
   wherein
   - X2 is Aib, Ala, D-Ala, Gly, Ser, N-Me-Ser, Ac3c, Ac4c or Ac5c;
   - X10 is Tyr, Leu or Ser;
   - X11 is Ser or Leu;
   - X12 is Lys, or Ile;
   - X15 is Asp or Glu;
   - X16 is Ser, Glu or Lys;
   - X17 is Ile, Lys, Gln or Arg;
   - X18 is His, Arg or Ala;
   - X19 is Gln, Lys, Ala or Glu;
   - X20 is Gln, Lys, Ala, His or Arg;
   - X21 is Ala, Leu, Asp or Glu;
   - X23 is Val or Ile;
   - X24 is Asn or Glu;
   - X25 is Tyr or Trp;
   - X27 is Leu, Glu, Ser, Lys or Val;
   - X28 is Ala, Ser or Arg;
   - X29 is Aib, Gly, Ala, Gln, Thr, Ser or Lys or is absent;
   - X30 is Lys-Gly, Gly-Pro-Ser-Ser-Gly-Ala-Pro-Pro-Pro-Ser, Gly-Pro-Ser-Ser-Gly-Ala-Pro-Pro-Ser, Pro-Ser-Ser-Gly-Ala-Pro-Pro-Pro-Ser, Pro-Ser-Ser-Giy-Ala-Pro-Pro-Ser, Gly-Lys-Lys-Asn-Asp-Trp-Lys-His-Asn-Ile-Thr-Gln or absent;
or a pharmaceutically acceptable salt or solvate thereof.

In an embodiment R"₁ is H-, Ac or pGlu.

In an embodiment X30 is absent in Formula B'1.

In an embodiment the short acting GIP RA is chosen amongst the peptides according to Formula 4

**R‴₁-Z‴-R‴₂** Formula4

wherein
- R‴₁ is H, Ac or pGlu
- R‴₂ is is -NH2 or -OH; and
- Z'" is an amino acid sequence according to Formula C'1 with the following amino acid sequence as set forth in (SEQ ID NO: 49): Tyr-X₂-Glu-Gly-Thr-Phe-Ile-Ser-Asp-Tyr-Ser-X₁₂-Glu-Leu-X₁₅-X₁₆-X₁₇-X₁₈-X₁₉-X₂₀-X₂₁-Phe-X₂₃-X₂₄-X₂₅-Leu-X₂₇-X₂₈-X₂₉-X₃₀
   wherein
   - X2 is Aib, Ala, D-Ala or Gly;
   - X12 is Lys or Ile;
   - X15 is Asp or Glu;
   - X16 is Ser, Glu or Lys;
   - X17 is Ile, Lys, Gln or Arg;
   - X18 is His, Arg or Ala;
   - X19 is Gln or Ala;
   - X20 is Gln, Lys, Ala, His or Arg;
   - X21 is Ala, Asp or Glu;
   - X23 is lie or Val;
   - X24 is Asn or Glu;
   - X25 is Tyr or Trp;
   - X27 is Leu, Glu, Ser, Lys or Val;
   - X28 is Ala, Ser or Arg;
   - X29 is Aib, Gly, Ala, Gln, Thr, Ser or Lys or is absent;
   - X30 is Lys-Gly, Gly-Pro-Ser-Ser-Gly-Ala-Pro-Pro-Pro-, Giy-Pro-Ser-Ser-Gly-Ala-Pro-Pro-Ser, Pro-Ser-Ser-Gly-Ala-Pro-Pro-Pro-Ser, Pro-Ser-Ser-Gly-Ala-Pro-Pro-Ser, Gly-Lys-Lys-Asn-Asp-Trp-Lys-His-Asn-Ile-Thr-Gln or absent;
or a pharmaceutically acceptable salt or solvate thereof.

In an embodiment X30 is absent in Formula C'1.

In an embodiment the short acting GIP RA is chosen amongst the peptides according to Formula 5:

**R₁""₁-Z""**-**R₂""₂** Formula **5**

wherein
- R""₁ is H, Ac or pGlu pyroglutamic acid (pGlu);
- R""₂ is is -NH2 or -OH; and
- Z"" is an amino acid sequence according to Formula D'1 with the following amino acid sequence as set forth in (SEQ ID NO: 50): Tyr-Aib-Glu-Gly-Thr-Phe-Ile-Ser-Asp-Tyr-Ser-Ile-Glu-Leu-X₁₅-X₁₆-X₁₇-X₁₈-X₁₉-X₂₀-X₂₁-Phe-Val-X₂₄-X₂₅-Leu-Leu-Ala-X₂₉-X₃₀
   wherein
   - X15 is Asp or Glu;
   - X16 is Lys;
   - X17 is Ile;
   - X18 is His or Ala;
   - X19 is Gln or Ala;
   - X20 is Gln, Lys or Arg;
   - X21 is Ala, Asp or Glu;
   - X24 is Asn or Glu;
   - X25 is Tyr or Trp;
   - X28 is Ala, Ser or Arg;
   - X29 is Gln or is absent;
   - X30 is Lys-Gly, Gly-Pro-Ser-Ser-Gly-Ala-Pro-Pro-Pro-Ser, Gly-Pro-Ser-Ser-Gly-Ala-Pro-Pro-Ser, Pro-Ser-Ser-Gly-Ala-Pro-Pro-Pro-Ser, Pro-Ser-Ser-Gly-Ala-Pro-Pro-Ser, Gly-Lys-Lys-Asn-Asp-Trp-Lys-His-Asn-Ile-Thr-Gln or absent;
or a pharmaceutically acceptable salt or solvate thereof.

In an embodiment X30 is absent in Formula D'1.

In an embodiment the short acting GIP RA is chosen amongst the peptides according to Formula 6:

**R‴ʺ₁-Z‴ʺ-R‴ʺ₂** Formula 6

wherein
- R‴ʺ₁ represents a group represented by formula -RA₁, -CO-RA₁, -CO-ORA₁, -CO-CORA₁, -SO-RA₁, -SO₂-RA₁, -SO₂-ORA₁, -CO-NRA₂-RA₃, -SO₂-NRA₂-RA₃, or - C(=NRA₁)-NRA₂RA₃
   ∘ wherein RA₁, RA₂, and RA₃ each independently represent a hydrogen atom or a hydrocarbon group comprising at most 6 carbon atoms,
- R‴ʺ₂ is is -NH2 or -OH; and
- Z‴ʺ is an amino acid sequence according to Formula E'1 with the following amino acid sequence as set forth in (SEQ ID NO: 51): Tyr-X2-Glu-Gly-Thr-X6-X7-X8-X9-X10-X11-X12-X13-X14-X15-X16-X17-X18-X19-X20-X1-X22-X23-X24-X25-X26-X27-X28-X29-X30-X31-X32-X33-X34-X35-X36-X37-X38-X39-X40
   Wherein
   - X2 represents Aib or D-Ala;
   - X6 represents Iva, Phe, or Val;
   - X7 represents Ile, Lys, or Val;
   - X8 represents Ser;
   - X9 represents Asp, Leu, or Phe;
   - X10 represents Tyr;
   - X11 represents Aib or Ser;
   - X12 represents Ile;
   - X13 represents Aib, Ala, Gln, Leu, Tyr, or D-Iva;
   - X14 represents Leu;
   - X15 represents Asp, Glu, Lys, Ser, or Tyr;
   - X16 represents Arg or Lys;
   - X17 represents Aib, Gln, or Ile;
   - X18 represents Ala or His;
   - X19 represents Gln or Ser;
   - X20 represents Aib, Ala, or Gln;
   - X21 represents Asn, Asp, Glu, Leu, or Ser;
   - X22 represents Phe or aMePhe;
   - X23 represents lie or Val;
   - X24 represents Arg, Asn, Asp, Lys, or Lys(Ac);
   - X25 represents Trp;
   - X26 represents Aib, Iva, or Leu;
   - X27 represents Leu;
   - X28 represents Ala, Arg, Lys, or Lys(Ac);
   - X29 represents Gln or Gly;
   - X30 represents Arg, Gly, or a deletion;
   - X31 represents Pro or a deletion;
   - X32 represents Ser or a deletion;
   - X33 represents Ser or a deletion;
   - X34 represents Gly or a deletion;
   - X35 represents Ala or a deletion;
   - X36 represents Pro or a deletion;
   - X37 represents Pro or a deletion;
   - X38 represents Pro or a deletion;
   - X39 represents Lys, Ser, or a deletion; and
   - X40 represents Arg, Lys, or a deletion;
provided that where all X31 to X40 represent deletions, then X2 represents Aib, or a salt thereof

In the present specification, examples of the "hydrocarbon group" (including "hydrocarbon group" of "optionally substituted hydrocarbon group") include a C1-6 alkyl group, a C2-6 alkenyl group, a C2-6 alkynyl group, a C3-6 cycloalkyl group, a C3-6 cycloalkenyl group and a C6 aryl group.

In an embodiment, the composition comprises from 10 to 1500 µg/ml of short-acting GIP RA.

In an embodiment, the composition comprises from 100 to 1000 µg/ml of short-acting GIP RA.

In an embodiment the short acting PYY is chosen amongst the following peptides:
- Tyr Pro Ile Lys Pro Glu Ala Pro Gly Glu Asp Ala Ser Pro Glu Glu Leu Asn Arg Tyr Tyr Ala Ser Leu Arg His Tyr Leu Asn Leu Val Thr Arg Gln Arg Tyr (Seq ID NO: 52), (PYY 1-36)
- Ile Lys Pro Glu Ala Pro Gly Glu Asp Ala Ser Pro Glu Glu Leu Asn Arg Tyr Tyr Ala Ser Leu Arg His Tyr Leu Asn Leu Val Thr Arg Gln Arg Tyr (Seq ID NO: 53) (PYY 3-36), and
- Lys Pro Glu Ala Pro Gly Glu Asp Ala Ser Pro Glu Glu Leu Asn Arg Tyr Tyr Ala Ser Leu Arg His Tyr Leu Asn Leu Val Thr Arg Gln Arg Tyr (Seq ID NO: 54) (PYY 4-36).

In an embodiment, the composition comprises from 10 to 1500 µg/ml of short-acting PYY analog.

In an embodiment, the composition comprises from 100 to 1000 µg/ml of short-acting PYY analog.

In an embodiment, the composition is a clear solution.

In another embodiment the composition is an injectable solution.

According to an embodiment, the composition is an aqueous injectable solution.

According to an embodiment, the composition is an injectable solution comprising an aprotic polar solvent.

According to an embodiment the composition comprises at least 10 % w/w of water.

According to an embodiment the composition comprises at least 50 % w/w of water.

According to an embodiment the composition comprises at least 70 % w/w of water.

According to an embodiment the composition comprises at least 80 % w/w of water.

According to an embodiment the composition comprises at least 10 % of water.

According to an embodiment the composition is a solution comprising at least 10 % v/v of water.

According to an embodiment the composition is a solution comprising at least 30 % v/v of water.

According to an embodiment the composition is a solution comprising at least 50 % v/v of water.

According to an embodiment the composition is a solution comprising at least 70 % v/v of water.

According to an embodiment the composition is a solution comprising at least 75 % v/v of water.

According to an embodiment the composition is a solution comprising at least 80 % v/v of water.

According to an embodiment the composition is a solution comprising at least 85 % v/v of water.

According to an embodiment the composition is an aqueous injectable solution. By "aqueous solution" is meant that at least 80 % of the volume of the solution is water.

According to an embodiment, the aqueous solution comprises less than 1 % v/v of aprotic polar solvent.

According to an embodiment, the composition comprises an aprotic polar solvent.

In an embodiment, the aprotic polar solvent is chosen from the group consisting of dimethylsulfoxide (DMSO), *N,N*-dimethylformamide (DMF), ethyl acetate, *N*-methyl-2-pyrrolidone (NMP), *N,N*-dimethylacetamide (DMA), and propylene carbonate.

In an embodiment, the aprotic polar solvent is DMSO, NMP or a mixture thereof.

In an embodiment, the aprotic polar solvent is DMSO.

According to an embodiment, the composition comprises at least 10 % of an aprotic polar solvent.

According to an embodiment the composition is a solution comprising at least 10 % v/v of aprotic polar solvent.

According to an embodiment the composition is a solution comprising at least 20 % v/v of aprotic polar solvent.

According to an embodiment the composition is a solution comprising at least 30 % v/v of aprotic polar solvent.

According to an embodiment the composition is a solution comprising at least 40 % v/v of aprotic polar solvent.

According to an embodiment the composition is a solution comprising at least 50 % v/v of aprotic polar solvent.

According to an embodiment the composition is a solution comprising at least 60 % v/v of aprotic polar solvent.

According to an embodiment the composition is a solution comprising at least 70 % v/v of aprotic polar solvent.

According to an embodiment the composition is a solution comprising at least 80 % v/v of aprotic polar solvent.

By "aprotic polar solvent solution" is meant a solution comprising at least 50 % v/v of aprotic polar solvent.

According to an embodiment the composition is a solution comprising an aprotic polar solvent and at least 10 % v/v of water.

According to an embodiment the composition is a solution comprising an aprotic polar solvent and at least 20 % v/v of water.

According to an embodiment the composition is a solution comprising an aprotic polar solvent and at least 30 % v/v of water.

According to an embodiment the composition is a solution comprising an aprotic polar solvent and at least 40 % v/v of water.

According to an embodiment the composition is a solution comprising an aprotic polar solvent and at least 50 % v/v of water.

According to an embodiment the composition is a solution comprising from 50 to 90 % v/v of aprotic polar solvent and from 10 to 50 % v/v of water.

According to an embodiment, the composition comprising an aprotic polar solvent further comprises 1 to 50 mM of an ionization stabilizing excipient.

According to an embodiment, the composition comprising an aprotic polar solvent further comprises 2 to 20 mM of an ionization stabilizing excipient.

According to an embodiment, the composition comprising an aprotic polar solvent further comprises 2 to 10 mM of an ionization stabilizing excipient.

According to an embodiment, the composition comprising an aprotic polar solvent further comprises 3 to 7 mM of an ionization stabilizing excipient.

According to an embodiment the ionization stabilizing excipient is a mineral acid. Said mineral acid may be selected from hydrochloric acid, sulfuric acid, nitric acid and phosphoric acid.

According to an embodiment the ionization stabilizing excipient is an organic acid, such as acids having a carboxylic acid -COOH functional group. Said organic acids may be selected from acetic acid, citric acid, and amino acids.

When the composition is a solution, the concentrations are expressed in weight or Units to volume, such as U/mL, mg/ml or µg/mL.

When the composition is in liquid form that contains enough water to allow a reliable measure of pH, its pH may be as listed below.

In an embodiment the pH of the composition is ranging from 3.0 to 8.0.

In an embodiment the pH of the composition is ranging from 3.2 to 7.8.

In an embodiment the pH of the composition is ranging from 3.4 to 7.6.

In an embodiment the pH of the composition is ranging from 3.6 to 7.4.

In an embodiment the pH of the composition is ranging from 3.4 to 7.2.

In an embodiment the pH of the composition is ranging from 3.2 to 7.0.

In an embodiment the pH of the composition is ranging from 3.0 to 6.8.

In an embodiment the pH of the composition is ranging from 3.0 to 6.6.

In an embodiment the pH of the composition is ranging from 3.0 to 4.4.

In an embodiment the pH of the composition is ranging from 3.2 to 4.8.

In an embodiment the pH of the composition is ranging from 3.5 to 4.4.

In an embodiment the pH of the composition is ranging from 3.6 to 4.4.

In an embodiment the pH of the composition is ranging from 3.7 to 4.3.

In an embodiment the pH of the composition is ranging from 3.5 to 4.2.

In an embodiment the pH of the composition is ranging from 3.6 to 4.4.

In an embodiment the pH of the composition is ranging from 3.7 to 4.0.

In another embodiment the pH of the composition is ranging from 3.8 to 4.2.

In another embodiment the pH of the composition is ranging from 3.2 to 3.8.

In another embodiment the pH of the composition is ranging from 3.3 to 3.7.

In another embodiment the pH of the composition is 4.0.

In another embodiment the pH of the composition is 3.9.

In another embodiment the pH of the composition is 3.8.

In another embodiment the pH of the composition is 3.7.

In another embodiment the pH of the composition is 3.6.

In an embodiment the pH of the composition is 3.5.

In an embodiment the pH of the composition is 3.4.

In an embodiment the pH of the composition is ranging from 5.0 to 8.0.

In an embodiment the pH of the composition is ranging from 5.5 to 7.8.

In an embodiment the pH of the composition is ranging from 6.0 to 7.6.

In an embodiment the pH of the composition is ranging from 6.5 to 7.4.

In an embodiment the pH of the composition is ranging from 6.8 to 7.4.

According to an embodiment the composition comprises one short-acting GLP-1 RA and one short-acting amylin RA.

According to an embodiment the composition comprises exenatide and pramlintide.

According to an embodiment the composition comprises lixisenatide and pramlintide.

According to an embodiment the composition comprises one short-acting GLP-1 RA and one short-acting glucagon RA.

According to an embodiment the composition comprises exenatide and human glucagon.

According to an embodiment the composition comprises exenatide and dasiglucagon.

According to an embodiment the composition comprises lixisenatide and human glucagon.

According to an embodiment the composition comprises lixisenatide and dasiglucagon.

According to an embodiment the composition comprises one short-acting glucagon RA and one short-acting amylin RA.

According to an embodiment the composition comprises human glucagon and pramlintide.

According to an embodiment the composition comprises dasiglucagon and pramlintide.

According to an embodiment the composition comprises one short-acting glucagon RA, one short-acting GLP-1 RA and one short-acting amylin RA.

According to an embodiment the composition comprises pramlintide, human glucagon and exenatide.

According to an embodiment the composition comprises pramlintide, human glucagon and lixisenatide.

According to an embodiment the composition comprises pramlintide, dasiglucagon and exenatide.

According to an embodiment the composition comprises pramlintide, dasiglucagon and lixisenatide.

According to an embodiment the composition comprises at least 2 short-acting peptides chosen in at least two of the following groups, GLP-1 RA, Amylin RA and Glucagon RA and a copolyamino acid bearing carboxylate charges and hydrophobic radicals Hy.

According to an embodiment the compositions comprises a copolyamino acid bearing carboxylate charges and hydrophobic radicals Hy , said copolyamino acid consisting of glutamic or aspartic units and said hydrophobic radicals -Hy being chosen among the radicals according to formula X as defined below: 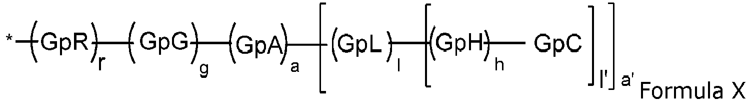 wherein
- GpR is chosen among the radicals according to formulas VII, VII' or VII": 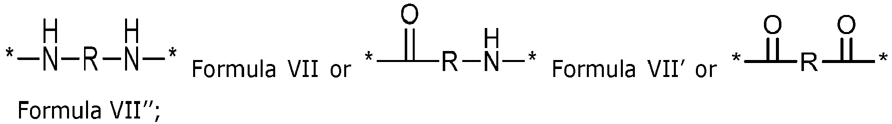
- GpG and GpH identical or different are chosen among the radicals according to formulas XI or XI': 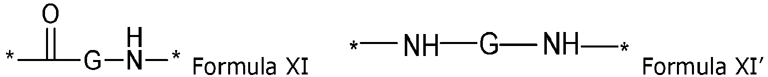
- GpA is chosen among the radicals according to formula VIIIa or VIIIb 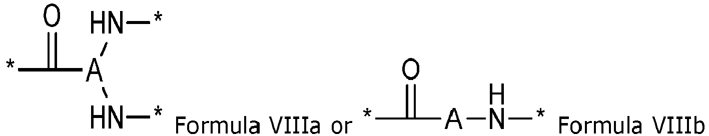
- -GpL is a radical according to formula XII 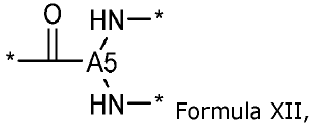
- GpC is a radical according to formula IX: 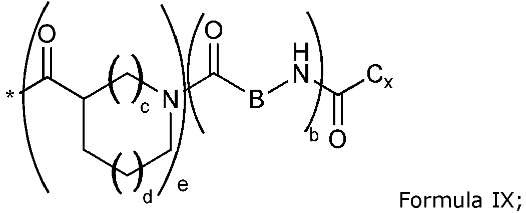
   - the ^{∗} indicate the binding sites of the different groups bound by an amide function;
   - a is an integer equal to 0 or to 1 and a' = 1 if a = 0 and a' = 1, 2 or 3 if a = 1;
   - a' is an integer equal to 1, to 2 or to 3;
   - b is an integer equal to 0 or to 1;
   - c is an integer equal to 0 or to 1, and if c is equal to 0 then d is equal to 1 or to 2;
   - d is an integer equal to 0, to 1 or to 2;
   - e is an integer equal to 0 or to 1;
   - g is an integer equal to 0, to 1, to 2, to 3 to 4 to 5 or to 6;
   - h is an integer equal to 0, to 1, to 2, to 3 to 4 to 5 or to 6;
   - l is an integer equal to 0 or 1 and l' = 1 if l = 0 and l' = 2 if l = 1;
   - l' is an integer equal to 1 or to 2;
   - r is an integer equal to 0, 1 or to 2, and
   - s' is an integer equal to 0 or 1;
   - And if e is different from 0, then at least one of g, h or I is different from 0
   - And if a = 0, then l = 0;
   - A and A₅ identical or different are linear or branched alkyl radicals comprising from 1 to 8 carbon atoms, and optionally substituted by a radical from a saturated, unsaturated or aromatic ring;
   - B is a radical chosen in the group consisting of a non-substituted ether or polyether radical comprising from 4 to 14 carbon atoms and from 1 to 5 oxygen atoms or a linear or branched alkyl radical, optionally comprising an aromatic nucleus, comprising from 1 to 9 carbon atoms;
   - Cₓ is a radical chosen in the group consisting of a linear or branched monovalent alkyl radical, optionally comprising a cyclic part, wherein x indicates the number of carbon atoms and 6≤x≤25, in particular
      - When the hydrophobic radical -Hy bears 1 -GpC, then 9 ≤ x ≤ 25,
      - When the hydrophobic radical -Hy bears 2 -GpC, then 9 ≤ x ≤ 15,
   - G is a linear or branched divalent alkyl radical of 1 to 8 carbon atoms, said alkyl radical bearing one or a plurality of free carboxylic acid function(s),
   - R is a radical chosen in the group consisting of a linear or branched, divalent alkyl radical comprising from 1 to 12 carbon atoms or a non-substituted ether or polyether radical comprising from 4 to 14 carbon atoms and from 1 to 5 oxygen atoms.

In an embodiment, e = 1.

In an embodiment said hydrophobic radicals are chosen amongst the radicals
according to formula X wherein g=l=0 according to formula Xa as defined below :

-(GpR)r-(GpA)a-[(GpH)h-GpC]l' formula Xa

wherein GpR, GpA, GpH, GpC, r, a, h and l' are as defined according to formula X.

In one embodiment, said hydrophobic radicals -Hy is chosen among radicals according to formula X wherein g=l=0 according to formula Xa as defined below :

-(GpR)ᵣ-(GpA)ₐ-[(GpH)ₕ-GpC]_{l'} formula Xa

wherein GpC is a radical according to formula IX wherein e=1 and b=0 and GpC is according to the following formula IXa:

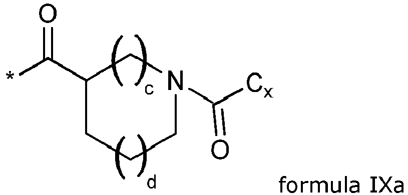

In one embodiment, said hydrophobic radical -Hy is chosen radicals according to formula X wherein g=l=0 according to formula Xa as defined below :

-(GpR)ᵣ-(GpA)ₐ-[(GpH)ₕ-GpC]_{l'} formula Xa

wherein GpR, GpA, GpH, GpC, r, h, a and l' are as defined above and h = 1, 2 or 3.

In one embodiment, said hydrophobic radical -Hy is chosen radicals according to formula X wherein g=l=0 and a=1 and l'=2 according to formula Xb as defined below :

-(GpR)ᵣ-GpA-[(GpH)ₕ-GpC]₂ formula Xb

wherein GpR, GpA, GpH, GpC, h and r are as defined above.

In one embodiment, said hydrophobic radical -Hy is chosen radicals according to formula X wherein g=l=0 and a=1 and l'=2 according to formula Xb as defined below :

-(GpR)ᵣ-GpA-[(GpH)ₕ-GpC]₂ formula Xb

wherein GpR, GpA, GpH, r and h are as defined above and GpC is according to formula IXa as defined below :

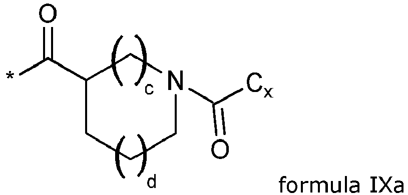

In one embodiment, said hydrophobic radical -Hy is chosen radicals according to formula X wherein g=l=0 and a=1 and l'=2 according to formula Xb as defined below :

-(GpR)ᵣ-GpA-[(GpH)ₕ-GpC]₂ formula Xb

wherein GpR, GpA, GpH, GpC and r are as defined above and h = 1, 2 or 3.

In an embodiment said hydrophobic radicals are chosen among the radicals according to formula X wherein g=l=0 and r= 1 according to formula Xc as defined below:

-GpR- (GpA)a-[(GpH)h-GpC]l' formula Xc

wherein GpR is a radical according to formula VII

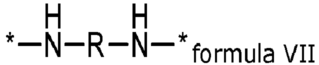

And GpA, GpH, GpC, al, h and l' are as defined above.

In an embodiment said hydrophobic radicals are chosen amongst the radicals according to formula X wherein r=g=l=0 according tot formula Xd as defined below:

-(GpA)ₐ-[(GpH)ₕ-GpC]_{l'} formula Xd

wherein GpA, GpH, GpC, a, h and l' are as defined above.

In an embodiment, said hydrophobic radical is according to formula X, Xa , Xb, Xc or Xd in which h = 1, 2 or 3.

In an embodiment, said hydrophobic radical is according to formula X, Xa Xb, Xc or Xd in which h = 1.

In an embodiment, said hydrophobic radical is according to formula X, Xa Xb, Xc or Xd in which h = 2.

In an embodiment, said hydrophobic radical is according to formula X, Xa Xb, Xc or Xd in which h = 3.

In an embodiment, the composition is characterized in that the hydrophobic radical according to formula X, Xa, Xb, Xc or Xd is a radical wherein R is a divalent linear alkyl radical comprising from 2 to 12 carbon atoms.

In an embodiment, the composition is characterized in that the hydrophobic radical according to formula X, Xa, Xb, Xc or Xd is a radical wherein R is a divalent linear alkyl radical comprising from 2 to 6 carbon atoms.

In an embodiment, the composition is characterized in that the hydrophobic radical according to formula X, Xa, Xb, Xc or Xd is a radical wherein R is a divalent linear alkyl radical comprising from 2 to 4 carbon atoms.

In an embodiment, the composition is characterized in that the hydrophobic radical according to formula X, Xa, Xb, Xc or Xd is a radical wherein R is a divalent linear alkyl radical comprising 2 carbon atoms.

In an embodiment, the composition is characterized in that the hydrophobic radical according to formula X, Xa, Xb, Xc or Xd is a radical wherein R is a divalent branched alkyl radical comprising from 2 to 12 atoms of carbon.

In an embodiment, the composition is characterized in that the hydrophobic radical according to formula X, Xa, Xb, Xc or Xd is a radical wherein R is an unsubstituted linear ether or polyether radical comprising 4 to 14 carbon atoms and from 1 to 5 oxygen atoms.

In an embodiment, the composition is characterized in that the hydrophobic radical according to formula X, Xa, Xb, Xc or Xd is a radical wherein R is an ether radical.

In an embodiment, the composition according to the invention is characterized in that the hydrophobic radical according to formula X, Xa, Xb, Xc or Xd wherein the radical GpA is according to formula VIIIa or VIIIb and in which A is chosen from the group consisting of the radicals represented by the formulas below:

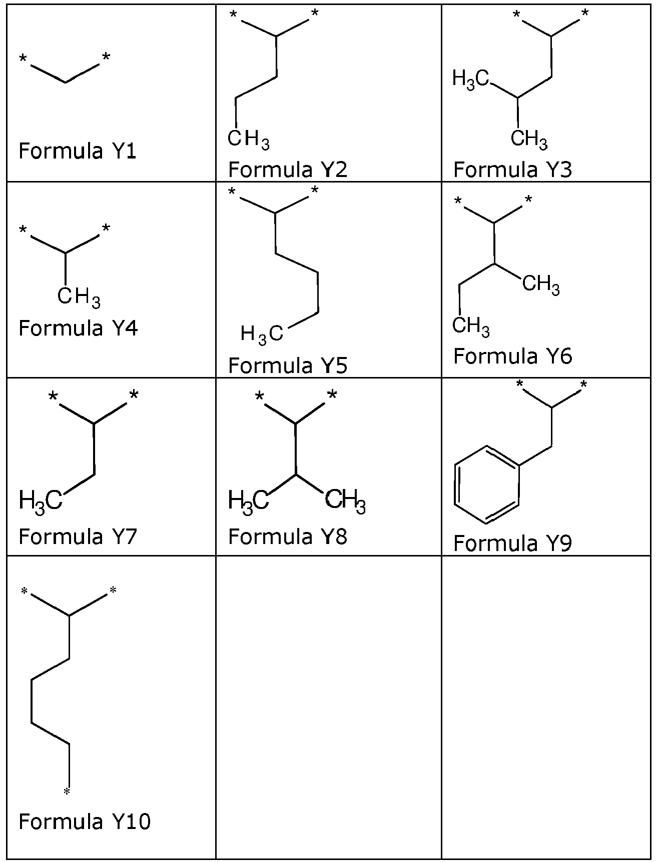

In one embodiment, the composition is characterized in that the hydrophobic radical according to formulas X, Xa, Xb, Xc or Xd is a radical wherein the radical GpG and/or GpH is according to formula XI' in which G is an alkyl radical comprising 6 carbon atoms represented by formula Z below:

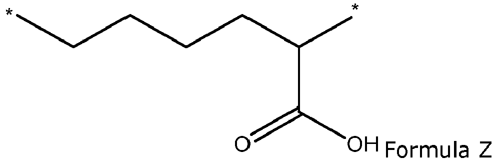

In one embodiment, the composition is characterized in that the hydrophobic radical according to formulas X, Xa, Xb, Xc or Xd is a radical wherein the radical GpG and/or GpH is according to formula XI wherein is an alkyl radical comprising 4 carbon atoms represented by formula Z' below:

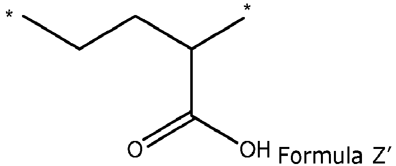

In one embodiment, the composition is characterized in that the hydrophobic radical according to formulas X, Xa, Xb, Xc or Xd is a radical wherein the radical GpC according to formula IXa, is chosen among radicals according to formulas IXb, IXc, and IXd represented below:

| | |
|---|---|
| 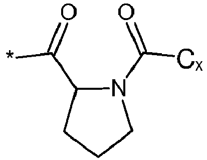 | Formula IXb |
| 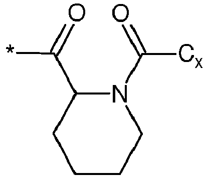 | Formula IXc |
| 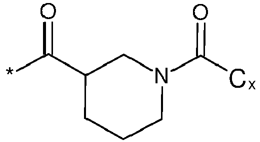 | Formula IXd |

In one embodiment, the composition is characterized in that the hydrophobic radical according to formulas X, Xa, Xb, Xc or Xd is a radical wherein Cx is a linear or branched monovalent alkyl radical comprising from 9 to 25 carbon atoms.

In one embodiment, the composition is characterized in that the hydrophobic radical according to formulas X, Xa, Xb, Xc or Xd is a radical wherein Cx is a linear or branched monovalent alkyl radical comprising from 11 to 17 carbon atoms.

In one embodiment, the composition is characterized in that the hydrophobic radical according to formulas X, Xa, Xb, Xc or Xd is a radical wherein Cx is a linear or branched monovalent alkyl radical comprising from 13 to 15 carbon atoms

In one embodiment, the composition is characterized in that the hydrophobic radical according to formulas X, Xa, Xb, Xc or Xd is a radical wherein Cx is a linear or branched monovalent alkyl radical comprising 13 carbon atoms.

In one embodiment, the composition is characterized in that the hydrophobic radical according to formulas X, Xa, Xb, Xc or Xd is a radical wherein Cx is a linear or branched monovalent alkyl radical comprising 15 carbon atoms.

In one embodiment, the composition according to the invention is characterized in that the copolyamino acid bearing carboxylate charges and hydrophobic radicals is chosen from the copolyamino acids according to the following formula XXXa : 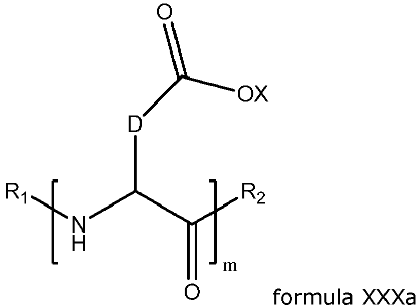 wherein
- D represents, independently, either a -CH₂- group (aspartic unit) or a -CH₂-CH₂-group (glutamic unit),
- Hy is a hydrophobic radical chosen from the hydrophobic radicals according to formula X,
- R₁ is a hydrophobic radical chosen from the hydrophobic radicals according to formula X or a radical chosen from the group consisting of an H, a linear acyl group in C2 to C10, a branched acyl group in C4 to C10, a benzyl, a terminal amino acid unit and a pyroglutamate,
- R₂ is a hydrophobic radical chosen from the hydrophobic radicals according to formula X in which r=1 and GpR is a radical according to formula VII, or a radical -NR'R", with R' and R", either identical or different, being chosen from the group comprised by H, the linear or branched or cyclic alkyls in C2 to C10, the benzyl and said alkyls R' and R" which may form together one or more saturated, unsaturated and/or aromatic carbonated cycles, and/or may comprise hetero-atoms, chosen from the group comprised of O, N and S;
- at least R₁ or R₂ is a hydrophobic radical according to formula X,
- X represents a cationic entity chosen from the group comprising alkaline cations;
- m represents the degree of polymerization DP of the copolyamino acid, that means, the average number of monomeric units per copolyamino acid chain and 5 ≤ m ≤ 250.

In one embodiment, the composition according to the invention is characterized in that the copolyamino acid bearing carboxylate charges and hydrophobic radicals is chosen among the copolyamino acids according to formula XXXa and R₁ is a hydrophobic radical according to formula X.

In one embodiment, the composition according to the invention is characterized in that the copolyamino acid bearing carboxylate charges and hydrophobic radicals is chosen among the copolyamino acids according to formula XXXa and R₂ is a hydrophobic radical according to formula X.

In one embodiment, the composition according to the invention is characterized in that the copolyamino acid bearing carboxylate charges and hydrophobic radicals is chosen from the copolyamino acids according to the following formula XXXb 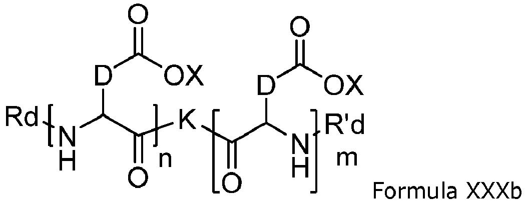 wherein
- D represents, independently, either a -CH₂- group (aspartic unit) or a -CH₂-CH₂-group (glutamic unit),
- X represents a cationic entity chosen from the group comprising alkaline cations;
- Rd et R'd, identical or different, are a hydrophobic radical -Hy according to formula X,
- K is a divalent linker chosen among the following formulas III or IV, 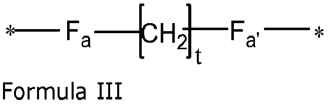 wherein
   ∘ 1 ≤ t ≤ 8, this means that t is an integer comprised from 1 to 8,
   ∘ Fa et Fa', identical or different represent a -NH- function 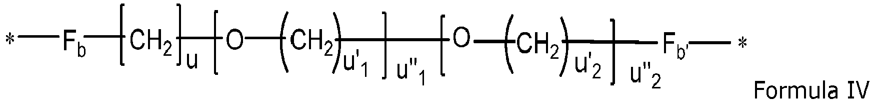 wherein :
      - Fb et Fb', identical or different represent a -NH- function
      - at least one of u₁" or u₂" is différent from 0.
      - if u₁" ≠ 0 then u₁' ≠ 0 and if u₂" ≠ 0 then u₂' ≠ 0,
      - u₁' and u₂' are identical or different and,
      - 2 ≤ u ≤ 4,
      - 0 ≤ u₁' ≤ 4,
      - 0 ≤ u₁" ≤ 4,
      - 0 ≤ u₂' ≤ 4,
      - 0 ≤ u₂" ≤ 4,
      - said linker K is bound to the at least two glutamic or aspartic chains of the copolyamino acid by amides functions;
      - n + m represents the degree of polymerization DP of the copolyamino acid, that means, the average number of monomeric units per copolyamino acid chain and 5 ≤ n + m ≤ 250.

In one embodiment, the composition according to the invention is characterized in that the copolyamino acid bearing carboxylate charges and hydrophobic radicals is chosen from the copolyamino acids according to the following formula XXXc 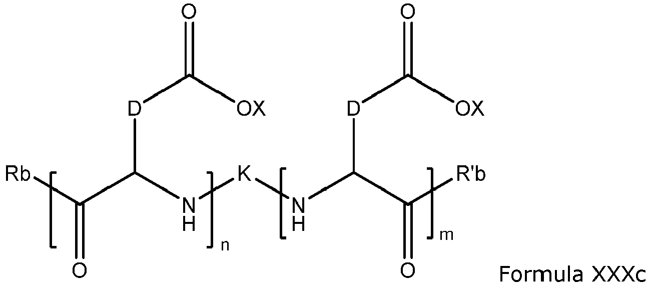 wherein,
- D and X are as defined according to formula XXXb,
- Rb and R'b, identical or different, are a hydrophobic radical -Hy,
- K is as defined according to formula XXXb, except than
   ∘ Fa and Fa', identical or different represent a -CO- function and
   ∘ Fb and Fb', identical or different represent a -CO- function.
- n + m is as defined according to formula XXXb.

In an embodiment the composition comprises the copolyamino acid bearing carboxylate charges and hydrophobic radicals in a concentration ranging from 1 to 50 mg/ml.

In an embodiment the composition comprises the copolyamino acid bearing carboxylate charges and hydrophobic radicals in a concentration ranging from 2 to 30 mg/ml.

In an embodiment the composition comprises the copolyamino acid bearing carboxylate charges and hydrophobic radicals in a concentration ranging from 4 to 20 mg/ml.

In an embodiment the composition comprises the copolyamino acid bearing carboxylate charges and hydrophobic radicals in a concentration ranging from 5 to 15 mg/ml.

According to an embodiment the composition is an aprotic polar solvent injectable solution comprising one short-acting GLP-1 RA and one short-acting amylin RA.

According to an embodiment the composition is an aprotic polar solvent injectable solution comprising exenatide and pramlintide.

According to an embodiment the composition is an aprotic polar solvent injectable solution comprising lixisenatide and pramlintide.

According to an embodiment the composition is an aprotic polar solvent injectable solution comprising one short-acting GLP-1 RA and one short-acting glucagon RA.

According to an embodiment the composition is an aprotic polar solvent injectable solution comprising exenatide and human glucagon.

According to an embodiment the composition is an aprotic polar solvent injectable solution comprising exenatide and dasiglucagon.

According to an embodiment the composition is an aprotic polar solvent injectable solution comprising lixisenatide and human glucagon.

According to an embodiment the composition is an aprotic polar solvent injectable solution comprising lixisenatide and dasiglucagon.

According to an embodiment the composition is an aprotic polar solvent injectable solution comprising one short-acting glucagon RA and one short-acting amylin RA.

According to an embodiment the composition is an aprotic polar solvent injectable solution comprising human glucagon and pramlintide.

According to an embodiment the composition is an aprotic polar solvent injectable solution comprising dasiglucagon and pramlintide.

According to an embodiment the composition is an aprotic polar solvent injectable solution comprising one short-acting glucagon RA, one short-acting GLP-1 RA and one short-acting amylin RA.

According to an embodiment the composition is an aprotic polar solvent injectable solution comprising pramlintide, human glucagon and exenatide.

According to an embodiment the composition is an aprotic polar solvent injectable solution comprising pramlintide, human glucagon and lixisenatide.

According to an embodiment the composition is an aprotic polar solvent injectable solution comprising pramlintide, dasiglucagon and exenatide.

According to an embodiment the composition is an aprotic polar solvent injectable solution comprising pramlintide, dasiglucagon and lixisenatide.

According to an embodiment the composition is an aqueous solution having a pH ranging from 3.0 to 8.0.

According to an embodiment the composition is an aqueous solution having a pH ranging from 3.6 to 7.6.

According to an embodiment the composition is an aqueous solution having a pH ranging from 3.0 to 6.6.

According to an embodiment the composition is an aqueous injectable solution having a pH ranging from 3.0 to 8.0, in particular ranging from 3.6 to 7.6 and more particularly from 3.0 to 6.6, and comprising one short-acting GLP-1 RA and one short-acting amylin RA.

According to an embodiment the composition is an aqueous injectable solution having a pH ranging from 3.0 to 8.0, in particular ranging from 3.6 to 7.6 and more particularly from 3.0 to 6.6, and comprising exenatide and pramlintide.

According to an embodiment the composition is an aqueous injectable solution having a pH ranging from 3.0 to 8.0, in particular ranging from 3.6 to 7.6 and more particularly from 3.0 to 6.6, and comprising lixisenatide and pramlintide.

According to an embodiment the composition is an aqueous injectable solution having a pH ranging from 3.0 to 8.0, in particular ranging from 3.6 to 7.6 and more particularly from 3.0 to 6.6, and comprising one short-acting GLP-1 RA and one short-acting glucagon RA.

According to an embodiment the composition is an aqueous injectable solution having a pH ranging from 3.0 to 8.0, in particular ranging from 3.6 to 7.6 and more particularly from 3.0 to 6.6, and comprising exenatide and dasiglucagon.

According to an embodiment the composition is an aqueous injectable solution having a pH ranging from 3.0 to 8.0, in particular ranging from 3.6 to 7.6 and more particularly from 3.0 to 6.6, and comprising lixisenatide and dasiglucagon.

According to an embodiment the composition is an aqueous injectable solution having a pH ranging from 3.0 to 8.0, in particular ranging from 3.6 to 7.6 and more particularly from 3.0 to 6.6, and comprising one short-acting glucagon RA and one short-acting amylin RA.

According to an embodiment the composition is an aqueous injectable solution having a pH ranging from 3.0 to 8.0, in particular ranging from 3.6 to 7.6 and more particularly from 3.0 to 6.6, and comprising dasiglucagon and pramlintide.

According to an embodiment the composition is an aqueous injectable solution having a pH ranging from 3.0 to 8.0, in particular ranging from 3.6 to 7.6 and more particularly from 3.0 to 6.6, and comprising one short-acting glucagon RA, one short-acting GLP-1 RA and one short-acting amylin RA.

According to an embodiment the composition is an aqueous injectable solution having a pH ranging from 3.0 to 8.0, in particular ranging from 3.6 to 7.6 and more particularly from 3.0 to 6.6, and comprising pramlintide, dasiglucagon and exenatide.

According to an embodiment the composition is an aqueous injectable solution having a pH ranging from 3.0 to 8.0, in particular ranging from 3.6 to 7.6 and more particularly from 3.0 to 6.6, and comprising pramlintide, dasiglucagon and lixisenatide.

According to an embodiment the composition is an aqueous solution having a pH ranging from 3.0 to 4.8.

According to an embodiment the composition is an aqueous solution having a pH ranging from 3.3 to 4.4.

According to an embodiment the composition is an aqueous injectable solution having a pH ranging from 3.0 to 4.8, in particular ranging from 3.3 to 4.4, and comprising one short-acting GLP-1 RA and one short-acting amylin RA.

According to an embodiment the composition is an aqueous injectable solution having a pH ranging from 3.0 to 4.8, in particular ranging from 3.3 to 4.4, and comprising exenatide and pramlintide.

According to an embodiment the composition is an aqueous injectable solution having a pH ranging from 3.0 to 4.8, in particular ranging from 3.3 to 4.4, and comprising lixisenatide and pramlintide.

According to an embodiment the composition is an aqueous injectable solution having a pH ranging from 3.0 to 4.8, in particular ranging from 3.3 to 4.4, and comprising one short-acting GLP-1 RA and one short-acting glucagon RA.

According to an embodiment the composition is an aqueous injectable solution having a pH ranging from 3.0 to 4.8, in particular ranging from 3.3 to 4.4, and comprising exenatide and dasiglucagon.

According to an embodiment the composition is an aqueous injectable solution having a pH ranging from 3.0 to 4.8, in particular ranging from 3.3 to 4.4, and comprising lixisenatide and dasiglucagon.

According to an embodiment the composition is an aqueous injectable solution having a pH ranging from 3.0 to 4.8, in particular ranging from 3.3 to 4.4, and comprising one short-acting glucagon RA and one short-acting amylin RA.

According to an embodiment the composition is an aqueous injectable solution having a pH ranging from 3.0 to 4.8, in particular ranging from 3.3 to 4.4, and comprising dasiglucagon and pramlintide.

According to an embodiment the composition is an aqueous injectable solution having a pH ranging from 3.0 to 4.8, in particular ranging from 3.3 to 4.4, and comprising one short-acting glucagon RA, one short-acting GLP-1 RA and one short-acting amylin RA.

According to an embodiment the composition is an aqueous injectable solution having a pH ranging from 3.0 to 4.8, in particular ranging from 3.3 to 4.4, and comprising pramlintide, dasiglucagon and exenatide.

According to an embodiment the composition is an aqueous injectable solution having a pH ranging from 3.0 to 4.8, in particular ranging from 3.3 to 4.4, and comprising pramlintide, dasiglucagon and lixisenatide.

According to an embodiment the composition is an aqueous solution having a pH ranging from 5.0 to 8.0.

According to an embodiment the composition is an aqueous solution having a pH ranging from 5.5 to 7.8.

According to an embodiment the composition is an aqueous injectable solution having a pH ranging from 5.0 to 8.0, in particular ranging from 5.5 to 7.8, and comprising one short-acting GLP-1 RA and one short-acting amylin RA.

According to an embodiment the composition is an aqueous injectable solution having a pH ranging from 5.0 to 8.0, in particular ranging from 5.5 to 7.8, and comprising one short-acting GLP-1 RA and one short-acting glucagon RA.

According to an embodiment the composition is an aqueous injectable solution having a pH ranging from 5.0 to 8.0, in particular ranging from 5.5 to 7.8, and comprising exenatide and dasiglucagon.

According to an embodiment the composition is an aqueous injectable solution having a pH ranging from 5.0 to 8.0, in particular ranging from 5.5 to 7.8, and comprising one short-acting glucagon RA and one short-acting amylin RA.

According to an embodiment the composition is an aqueous injectable solution having a pH ranging from 5.0 to 8.0, in particular ranging from 5.5 to 7.8, and comprising one short-acting glucagon RA, one short-acting GLP-1 RA and one short-acting amylin RA.

According to an embodiment the composition is an aqueous solution having a pH ranging from 5.0 to 8.0, in particular from 6.0 to 7.8, comprising a copolyamino acid bearing carboxylate charges and hydrophobic radicals according to formula X and at least two short-acting peptides chosen in at least two of the following groups, GLP-1 RA, amylin RA and glucagon RA.

In an embodiment the composition does not comprise long-acting peptides.

Among long-acting peptides can be cited semaglutide, dulaglutide, liraglutide, cagrilintide, albiglutide, davalintide, tirzepatide, cotadutide or MEDI-0382,, albiglutide, BI 456906 and SAR 425899.

In an embodiment, the invention relates to a composition free of protamine salt.

In another embodiment, the invention relates to a composition free of protamine salt or other positively charged peptides comprising a percentage of positively charged amino acids which is greater than or equal to 40%.

In an embodiment the composition does not contain amylin, in particular human amylin.

In an embodiment the composition does not contain salmon calcitonin (sCT).

In an embodiment the composition does not contain GLP-1, in particular human GLP-1.

In an embodiment the composition according does not contain insulin.

In an embodiment the composition does not contain grafted insulin.

In an embodiment the composition does not contain acylated insulin.

In an embodiment the composition does not contain pegylated insulin.

In an embodiment, the composition does not contain a long-acting insulin.

In an embodiment, the composition does not contain insulin glargine.

In an embodiment, the composition does not contain a prandial insulin.

In an embodiment, the composition does not contain a prandial insulin with a substitution on the A21 position.

According to an embodiment, the composition does not comprise a copolyamino acid bearing carboxylate charges and hydrophobic radicals.

According to an embodiment, the composition does not comprise a copolyamino acid consisting of more than 80 % of glutamate and aspartate residues and bearing carboxylate charges and hydrophobic radicals.

According to an embodiment, the composition does not comprise a copolyamino acid consisting of more than 80 % of glutamate and aspartate residues and bearing an acyl group comprising more than 6 carbon atoms.

In an embodiment, the composition according to the invention does not contain a copolyaminoacid consisting of glutamate or aspartate units and said copolyaminoacid bearing at least one acyl group comprising from 6 and 25 carbon atoms.

In an embodiment the composition according to the invention does not comprise an amphiphilic compound comprising a hydrophilic backbone bearing at least one acyl group comprising from 6 and 25 carbon atoms.

In an embodiment the composition according to the invention does not comprise a copolyamino acid bearing carboxylate charges and hydrophobic radicals described in patent applications WO2017211918 and WO2017211917.

In an embodiment the composition according to the invention does not comprise a copolyamino acid bearing carboxylate charges and hydrophobic radicals described in patent application WO2019/110837.

In an embodiment the composition according to the invention does not comprise a copolyamino acid bearing carboxylate charges and hydrophobic radicals described in patent application WO2019110836.

In an embodiment the composition according to the invention does not comprise a copolyamino acid bearing carboxylate charges and hydrophobic radicals described in patent application WO2019110838.

In an embodiment the composition according to the invention does not comprise a copolyamino acid bearing carboxylate charges and hydrophobic radicals described in patent application WO2020025825.

In an embodiment the composition according to the invention does not comprise a copolyamino acid bearing carboxylate charges and hydrophobic radicals described in patent application EP20217255.7.

In an embodiment the composition according to the invention does not comprise a copolyamino acid bearing carboxylate charges and hydrophobic radicals described in patent application EP20217270.6

In an embodiment, the pharmaceutical formulation comprising the composition according to the invention is not an aerosol form.

According to an embodiment the composition does not comprise long-acting glucagon suppressor.

According to an embodiment the composition does not comprise long-acting GLP-1 RA.

According to an embodiment the composition does not comprise long-acting acylated GLP-1 RA.

According to an embodiment the composition does not comprise long-acting GLP-1 RA acylated with an acid comprising an alkyl chain of at least 10 carbon atoms.

In an embodiment, the composition does not comprise amylin.

According to an embodiment the composition does not comprise long-acting amylin RA.

According to an embodiment the composition does not comprise long-acting acylated amylin RA.

According to an embodiment the composition does not comprise long-acting amylin RA acylated with an acid comprising an alkyl chain of at least 10 carbon atoms.

According to an embodiment the composition does not comprise long-acting glucagon RA.

According to an embodiment the composition does not comprise long-acting acylated glucagon RA.

According to an embodiment the composition does not comprise long-acting glucagon RA acylated with an acid comprising an alkyl chain of at least 10 carbon atoms.

In an embodiment, the invention relates to a pharmaceutical composition comprising compositions as defined above.

The invention also concerns a container comprising a pharmaceutical formulation according to the invention.

In one embodiment the container is a cartridge.

In one embodiment the container is a vial.

The invention also concerns a method of preparation of stable injectable compositions.

In one embodiment, the composition according to the invention also comprise buffers.

In one embodiment, the composition according to the invention comprises buffers at concentrations from 0 to 100 mM.

In one embodiment, the composition according to the invention comprises buffers at concentrations from 15 to 50 mM.

In one embodiment, the composition according to the invention comprise a buffer chosen from the group consisting of a phosphate buffer, acetate buffer, Tris (trishydroxymethyl)aminomethane and sodium citrate.

In one embodiment, the buffer is sodium phosphate.

In one embodiment, the buffer is Tris (trishydroxymethyl)aminomethane.

In one embodiment, the buffer is sodium citrate.

In one embodiment, the buffer is sodium acetate.

In one embodiment, the composition according to the invention comprises at least one antioxydant.

In one embodiment, the antioxydant is methionine.

In one embodiment, the concentration in antioxydant is comprised from 0.5 to 20 mM.

In one embodiment, the concentration in antioxydant is comprised from 2 to 10 mM.

In one embodiment, the composition according to the invention also comprise at least one preservative.

In one embodiment, the preservative is chosen from the group consisting of m-cresol and phenol, alone or in mixture.

In one embodiment, the concentration of the preservative is comprised from 10 to 50 mM.

In one embodiment, the concentration of the preservative is comprised from 10 to 40 mM.

In one embodiment, the concentration of the preservative is comprised from 20 to 30 mM.

In one embodiment, the composition according to the invention further comprise at least a surfactant.

In one embodiment, the surfactant is chosen in the group consisting of propylene glycol and polysorbates such as polysorbate 20 also called Tween^{®}20.

In one embodiment, the surfactant is chosen in the group of polysorbates.

In one embodiment, the concentration in polysorbate is comprised from 4 to 20 µM.

Composition according to the invention may further comprise additives such as tonicity agents.

In one embodiment, the tonicity agents are chosen in the group consisting of glycerine, sodium chloride, mannitol and glycine.

Composition according to the invention may further comprise all excipients compliant with pharmacopoeias and compatible with the insulins used at the customary concentrations.

The invention also relates to a pharmaceutical formulation according to the invention, obtained by drying and/or lyophilization.

The composition may be used for preventing or treating overweight, obesity and related diseases.

By "preventing or treating overweight, obesity and related diseases" is meant preventing or treating obesity, NASH, NAFLD, overweight, reducing body weight and/or food intake, or inducing energy expenditure or satiety.

In an embodiment the composition is used for preventing or treating obesity.

In an embodiment the composition is used for preventing or treating overweight.

In an embodiment the composition is used for preventing or treating NASH.

In an embodiment the composition is used for preventing or treating NAFLD.

In an embodiment the composition is used for reducing body weight.

In an embodiment the composition is used for reducing food intake.

In an embodiment the composition is used for inducing satiety.

In an embodiment the composition is used for inducing energy expenditure.

In another embodiment, the composition is used for preventing or treating neurodegenerative diseases.

In another embodiment, the composition is used for preventing or treating Type 2 Diabetes.

By "treating overweight, obesity and related diseases" is meant treating obesity, NASH, NAFLD, overweight, reducing body weight and/or food intake, or inducing energy expenditure or satiety.

The composition may be used for treating obesity and related diseases.

In an embodiment the composition is used for treating obesity.

In an embodiment the composition is used for treating overweight.

In an embodiment the composition is used for treating NASH.

In an embodiment the composition is used for treating NAFLD.

In another embodiment, the composition is used for treating Type 2 Diabetes.

In another embodiment, the composition is used for treating neurodegenerative disease. In particular the neurodegenerative disease is parkinson disease or alzheimer diseases.

According to an embodiment, the invention relates to a scheme of administration of a composition as defined above.

According to a further embodiment, the composition is delivered by a device as defined in the instant specification.

In an embodiment the scheme of administration comprises periods where the delivery is decreased or stopped.

In a further embodiment the scheme of administration comprises modulation of the delivered dose.

In a further embodiment the scheme of administration comprises modulation of the delivered dose, such as increasing and decreasing the delivery, or even stop and restart periods.

In a further embodiment the scheme of administration comprises modulation periods that allow a modulation of the daily dose.

In a further embodiment the scheme of administration comprises modulation periods that allow a decrease of the daily dose until it is discontinued, temporarily or definitively.

In a further embodiment the scheme of administration comprises stop and restart periods.

In a further embodiment the scheme of administration comprises modulation of the delivered dose during the day without changing the total daily dose.

In an embodiment the patient decides for the decrease of the delivery.

In another embodiment the decrease or total stop of delivery is done automatically for certain periods, for exemple during the night or part of the night.

The increase or the decrease of the delivery may be instantaneous or can reach the desired value through steps or through a ramp.

In an embodiment the patient may stop or decrease the delivery of the composition due to undesired side effects due to the composition or to take a short break of the treatment, for example to have a "normal" eating behaviour. These periods may help the patient to improve his state of mind and then to improve his compliance on the long-term of the treatment.

The administration of the composition may be modulated.

The modulation may be a decrease of the administration, for example by 10, 20, 30, 40, 50, 60, 70, 80, 90 or even 100 %.

The modulation may be an increase of the administration, for example by 10, 20, 30, 40, 50, 60, 70, 80, 90, 100, 150, 200, 300, 400, 500, 600, 700, 800, 900, or 1000 %.

By increase or decrease of the composition administered it may be the administration per hour or per day.

The modulation may last at least 1, 2, 3, 6, 12, 24, 36 or 48h.

The modulation may last at most 2, 4, 8, 12, 24, 36, 48 or 72h.

The period between a stop and a restart can be the same than those defined for modulation above.

For example the administration may stop for a few hours overnight or during the sleeping time of the patient and restarts in the early morning or a short time before the patient wakes up.

The administration may be diminished or stopped at 10pm, 11pm or 12pm and start again at 4am, 5am or 6am.

In an embodiment, the administration goes back to the rate of administration it was before the stop or the diminution.

In an embodiment, the administration starts again at a lower rate of administration than the one before the stop or diminution and goes back proggressively to the rate before the stop or diminution.

In an embodiment the modulation of the rate of delivery happens intraday.

In an embodiment this intraday modulation lasts at least 1, 2, 3, 4, 6, 8, 12, 16 h.

In an embodiment this intraday modulation lasts at most 2, 3, 4, 6, 8, 12, 16, 20 h.

In an embodiment the daily dose remains stable for a week, 2 weeks, 4 weeks, 2 months, 4 months, 6 months, a year or 2 years.

By "the daily dose remains stable" is meant the daily dose varies by less than 10%, in particular less than 5%.

In an embodiment, the daily dose may increase over 2, 4, 8 months. In particular during the beginning of the treatment until reaching the optimal daily dose.

In an embodiment, the daily dose may decrease over a period of 2, 4, 8 months. In particular before stopping the treatment.

According to an embodiment, the device delivers the composition via subcutaneous injection.

Referring to figure 2, the administering apparatus 2 can comprise:
- a housing 21
- a reservoir 22,
- a drive mechanism 23,
- a control unit 24,
- a power source 25,
- an injection set 26,
- optionally one or more sensor 27, and
- optionally an output/display 28.

The housing 21 can be of any suitable shape and size. For instance, the housing 21 may be in the shape of a square, rectangle, circle, cylinder or the like.

The housing 21 may be dimensioned so as to be comfortably associated with the patient and/or hidden from view, for instance, within the clothes of the patient. For instance, the housing 21 may have width and height of about 1 to about 15 centimeters, and a thickness of about 0.5 to about 2 centimeters. As used herein, the term "about" in the context of a given value or range refers to a value or range that is within 20%, preferably within 10%, and more preferably within 5% of the given value or range.

The materials of the housing 21 may vary as well. For instance, the housing 21 may be made of metal or plastic.

The housing 21 is designed to be worn by the patient. In one embodiment, the housing 21 includes gripping means (not represented) for fastening the administering apparatus on a patient's cloth, such as a belt. In another embodiment, the housing 21 includes an adhesive patch for adhesively attaching the administering apparatus to an infusion site on the patient's skin.

The reservoir 22 is adapted to store the composition 1 to be administered to the patient.

The reservoir 22 may be a single-use reservoir or a refillable reservoir.

The reservoir 22 have any suitable shape and size configured for receiving, storing, and releasing the composition 1. The reservoir 22 may be a container formed by a flexible material or a rigid material. The reservoir 22 may have a capacity of about 1 to about 10 milliliters.

In some embodiments, the reservoir 22 is integrated to the housing 21. In other embodiments, the reservoir 22 is separated from the housing 21. In that case, the housing 21 includes a receiving slot for reversibly attaching/detaching the reservoir 22 to the housing 21.

The drive mechanism 23 is configured for pumping the composition 1 contained in the reservoir 22 to an outlet port of the housing 21 connected to the injection set 26.

The drive mechanism 23 can be a pump, such as a peristaltic drive pump, or any other suitable type of drive mechanism known by the skilled person for pumping a fluid from a container to an outlet port.

The control unit 24 allows driving the drive mechanism 23, exchanging data with the supervisory terminal 3.

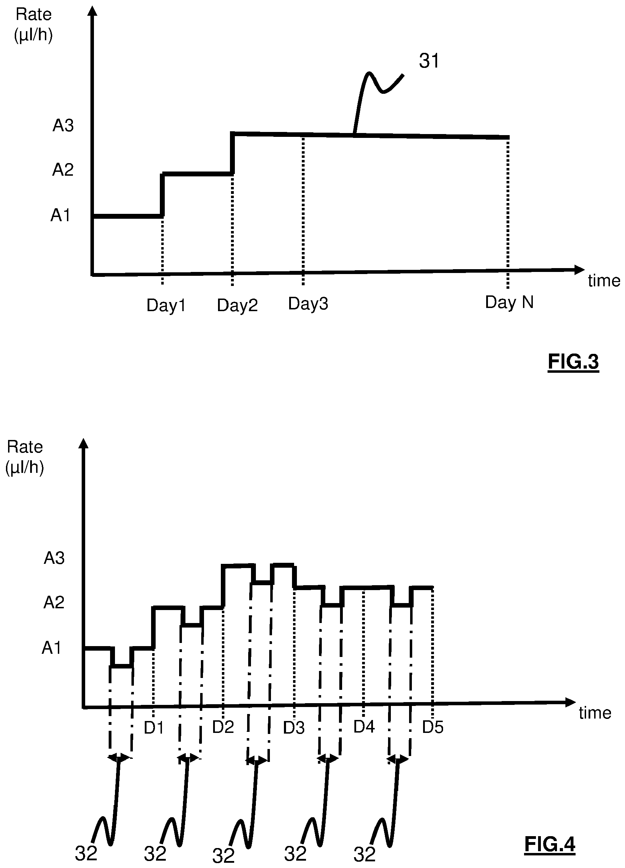

Referring to figure 3, the control unit 24 may include:
- a memory for storing one or more injection profile, one or more user parameters, one or more data measured by a sensor (or sensors) of the administering apparatus 2,
- a transceiver (wired or wireless) for transmitting/receiving information with the supervisory terminal 3,
- an alarm (visual indicator and/or audible indicator and/or tactile indicator) for providing visual/audible/tactile feedback to the patient regarding an operation of the administering apparatus 2, such as:
   ∘ the need to replace or refile the reservoir 22, or
   ∘ The need to replace or charge the power source 25, etc.
- a processor to control the overall functions of the drive mechanism 23.

The processor may also include programming that allows the processor to receive signals and/or other data from the supervisory terminal 3, or from various sensors (included as a part of the administering device 2 or used in conjunction therewith) and to store the same in the memory.

The power source 25 allows powering the administering apparatus 2 with electrical energy. The power source 25 may be composed of one or more batteries - which can be rechargeable or not. The power source 25 can be integrated within the housing 21 or separated from the housing 21 and connected to the housing 21 via electric wires.

Such power source 25 is well known in the art and will not be described in more details below.

The injection set 26 allows conveying the composition 1 into the skin of the patient.

Depending on the type of the administering apparatus 2 ("*conventional pump*" or "*patch pump*"), the injection set 26 can be integrated to or separated from the housing 21.

If the administering apparatus 2 is a *"conventional pump"*, then the infusion set 26 is separated from the administering apparatus 2 and may include:
- a connector,
- a length of tubing, and
- a hub from which a cannula, in the form of a hollow metal infusion needle or flexible plastic catheter extends; advantageously, the hub can have an adhesive that retains the hub on the skin surface during use.

If the administering apparatus 2 is a "*patch pump*", then the infusion set 26 is integrated to the administering apparatus 2. Indeed, a "*patch pump*" is an integrated device that combines most or all of the fluidic components (including the fluid reservoir 22, the drive mechanism 23 and the components adapted for automatically inserting the cannula) in a single housing which is adhesively attached to an infusion site on the patient's skin, and does not require the use of a separate infusion set. Configuring the administering apparatus 2 as a "*patch pump*" allows minimizing the size of the device in order to limit the interference between the use of said device and the activities of the patient. Hence, configuring the administering apparatus 2 as a *"patch pump*" allows reducing the discomfort associated to its use for the patient.

In any case, the infusion set 26 includes a cannula for the subcutaneous delivery of the composition to the patient.

As mentioned above, the administering apparatus 2 may comprise one or more sensor 27:
- for measuring physiological parameters of the patient, such as an accelerometer, a gyroscope, a pressure sensor, a temperature sensor, a blood glucose sensor, and the like, and/or
- for monitoring the administering apparatus 2, such as a sensor measuring the amount of energy available in the power source 25, a sensor measuring the amount of composition 1 contained in the reservoir 22, a sensor detecting a malfunctioning of the drive mechanism 23 (for example due to an occlusion preventing delivery of the composition 1, etc.).

For some applications, the administering apparatus 2 can include a display screen 28.

The type of display 28 may include LCD displays, LED displays, plasma displays, OLED displays and the like. The display 28 may also be an interactive or touch sensitive screen having an input device such as a touch screen, a capacitance screen, a resistive screen or the like.

Moreover, in some embodiments, the administering apparatus 2 may additionally include data entry means - such as a keyboard or other input device known in the art - for data entry, which may be separate from the output/display 28.

The supervisory terminal 3 allows controlling the administering apparatus 2. The supervisory terminal 3 can be integrated to the administering apparatus 2, or separated from the administering apparatus 2, as shown on figure 1.

For instance, the supervisory terminal 3 can consist of one (or several) work station(s) and/or one (or several) computer(s) or may be of any other type known to one skilled in the art.

The supervisory terminal 3 can also consist of a mobile phone (such as a smartphone), an electronic tablet (such as an IPAD^{®}), a Personal Digital Assistant (or "PDA"), etc.

The supervisory terminal 3 comprises a processing unit programmed in order to:
- communicate with the administering apparatus 2,
- estimate an injection profile according to user parameters and/or physiological parameters of the patient,
- process signals measured by sensors, and/or
- display information to the patient on a display element such as a display screen.

With regard to the processing of the signals, the processing unit can be implemented in order to alert a practitioner and/or the patient if a signal measured by a sensor (or signals measured by a plurality of sensors) exceed a heath threshold representative of a risk for the health of the patient.

Advantageously, the supervisory terminal 3 can include a Graphic User Interface (GUI) allowing the patient to input user parameter(s) with regard to the administration of the composition 1.

Indeed, the device can be configured to adapt the injection profile of the composition 1 according to one (or a plurality of) user parameter(s).

The supervisory terminal 3 may comprise a database including a plurality of different injection profiles 31, each infusion profile 31 defining one rate, or multiple rates over time for the continuous administration of the composition 1 to the patient. An example of infusion profile is given on figure 3. This injection profile is composed of different stages of rates increasing as a function of time. The skilled person will appreciate that the injection profile can also consist in a rate evolution according to a substantially constant rate ramp up, with the gradient of this ramp being on the order of 0.1 to 100 % per hour. This allows a progressive increase of the amount of composition that is injected to the patient in order to adjust his body, thus limiting the side effects, such as nausea or diarrhea, felt by the patient.

Advantageously, the administering of the composition can be implemented by injecting a plurality of successive bolus doses several times (per hour or per day). In an embodiment, the volume of each bolus dose being in a range of 0.01 to 10 µL, more preferably in a range of 0.05 to 5 µL, even more preferably in a range of 0.05 to 2 µL, especially preferably in a range of 0.05 to 1 µL.

As discussed above, the injection of the composition 1 can induce several side effects to the patient, such as nausea. These side effects are more pronounced at the beginning of the treatment (i.e., the three or four first weeks of administration of the composition). Moreover, these side effects can vary from one patient to another. This is why the device can be configured to take into account a feeling of the patient in order to determine an injection profile 31 suitable with the comforting sensations induced by the composition 1.

In particular, the supervisory terminal 3 may be configured to extract one injection profile of the plurality of injection profiles contained in the database according to one (or more than one) parameter(s) as will be discussed in more details below. Alternatively (or in combination), the supervisory terminal 3 can be configured to modify a current injection profile defined for a patient according to one (or more than one) parameter(s), for instance by increasing or decreasing a rate of the injection profile at specific moments of the day.

Moreover, in some cases, the satiety sensation does not need to be provided to the patient every time. For instance, when a patient is sleeping the injection rate of the composition 1 can be decreased in order to limit the amount of composition 1 administered continuously to the patient. This allows optimizing the consumption of the composition 1 contained in the reservoir 22 in order to:
- limit the risks associated with the administration of the composition 1 to the patient,
- extend the operating life of the device .

Different embodiments allowing an adjustment of the injection profile 31 according to a single parameter are discussed below. The skilled person will appreciate that the different parameters can be combined in order to adjust an injection profile 31.

In one embodiment, the device allows adapting the injection profile 31 according to a level of comfort defined by the patient.

More particularly, when the patient is supplied with the device , the supervisor terminal 3 extracts a first predetermined injection profile from the database and transmits said first injection profile to the control unit 24 of the administering apparatus 2.

The control unit 24 drives the drive mechanism 23 according to the first injection profile for continuously injecting the correct amount of composition 1 to the patient.

If the patient wishes to inform the supervisory terminal 3 of his feeling in response to the injection of the composition 1, the patient can input a level of comfort on the GUI of the supervisory terminal 3. Alternately, the supervisory terminal 3 can send a message to the patient requesting him to notify the supervisory terminal 3 of his level of comfort on the GUI of the supervisory terminal 3.

The level of comfort can be defined by a plurality of values representative of a sensation of nausea felt by the patient. For instance, the sense of nausea can be defined on a "*1 to 5*" scale:
- a value of "*1*" corresponding to a state where the patient does not feel side effect(s), such as nausea or diarrhea, and
- a value of "5" corresponding to a state where the patient does not feel fine, related to side effects.

Alternately or in combination, the level of comfort can be defined by a plurality of values representative of a sensation of hunger felt by the patient. For instance, the sense of hunger can be defined on a "*1 to 5*" scale:
- a value of *"1*" corresponding to a state where the patient does not feel hungry, and
- a value of "5" corresponding to a state where the patient feels hungry.

The skilled person will appreciate that other information can be used for notifying the supervisory terminal 3 with the level of comfort of the patient, such as a sense of stress, a sense of irritation, etc.

In response to the level of comfort defined by the user, the device can adapt the injection rate of the composition 1. In particular, the supervisory terminal 3 can estimate whether the injection profile determined for the patient needs to be amended or replaced.

For instance, if the patient informs the supervisory terminal 3 that he feels side effects, the supervisory terminal 3 can:
- determine a second injection profile wherein the injection rate(s) of the composition is (are) lower, and
- transmit this second injection profile to the control unit 24 in order to decrease the amount of composition 1 injected subcutaneously to the patient.

Since the composition 1 is a short-acting composition, the patient will feel better rapidly.

Alternatively, the supervisory terminal 3 can modify the rate of the current injection profile set to the patient by decreasing the rates defined in the injection profile for a given period of time 32. In some embodiments, this given period 32 of rate decrease can be automatically applied for each day of the injection profile (as shown on figure 4) so that the patient does not have to inform the supervisory terminal 3 of its feeling each day of the treatment.

On the contrary, if the patient informs the supervisory terminal 3 that he feels hungry, the supervisory terminal 3 can:
- extract a third injection profile from the database (or compute a third injection profile) where the injection rate(s) is (are) higher, and
- transmit this third injection profile to the control unit 24 in order to increase the amount of composition 1 administered to the patient.

If the supervisory terminal 3 compute the third injection profile, a comparison of the computed rate(s) with a predetermined maximum threshold (representative of a risk for the patient's health) can be implemented. Again, any increase of an injection rate for a given period of time of a day can be applied to the other days of the injection profile.

Advantageously in some embodiments, levels of comfort defined by the patient at different times of the day can be used by the supervisory terminal 3 in order to adapt the injection profile. In particular, the amount of composition 1 administered to the patient can be decreased at one (or plural) given time(s) of a current day, if the patient informed the supervisory terminal 3 of a side effect feeling at said given time(s) of a previous day. This allows dynamically adapting the treatment to the patient.

Conversely, the amount of composition administered to the patient can be increased at one (or plural) given time(s) of a current day, if the patient did not inform the supervisory terminal 3 of a nausea feeling at said given time(s) of a previous day. This allows increasing the effect of the treatment, for example improving the satiety feeling, at specific time of the day (for instance just before breakfast/lunch/diner time, or at specific period(s) defined by the user as being more critical with regard to his eating pattern, etc.) in order to improve the efficency of the solution according to the invention.

In another embodiment, the device allows adjusting the injection profile according to the patient's activity.

More particularly, when the patient is supplied with the device , the supervisor terminal 3 can modify the injection profile according to the motions of the patient.

For instance, if the administering apparatus 2 includes one (or more) sensor(s) - such as a gyroscope of an accelerometer - the control unit 24 can detect when the patient is in an active state or in a passive state, and can communicate this information to the supervisor terminal 3:
- when the patient is detected as being in an active state, the supervisor terminal 3 determines a first injection profile defining at least one first rate, and transmits this first injection profile to the control unit 24 for driving the drive mechanism 23,
- when the patient is detected as being in a passive state (for instance the patient is sleeping), the supervisor terminal 3 determines a second injection profile defining at least one second rate smaller than the first rate, and transmits this second injection profile to the control unit 24 for driving the drive mechanism 23.

In further other embodiments, the device may be configured to adjust the injection profile according to other parameters, such as the weight of the patient, or the occurrence of a special event in the life of the patient (a wedding, a birthday, etc.) during which the patient wants to feel "normal", or on the contrary wants to increase the effect of its treatment.

For instance, the patient can input his weight and/or the occurrence of a special event on the GUI of the supervisory terminal 3. In response to these user parameters, the supervisory terminal 3 can adjust the injection profile in order to decrease (or increase) the amount of composition 1 to be continuously administered to the patient subcutaneously.

As mentioned above, the administering apparatus 2 of the device can include a lot of features, in particular when the supervisory terminal 3 is incorporated within the administering apparatus 2 (memory, transmitter/receiver, alarm, input device, processor, output/display, graphic user interface, drive mechanism, processing unit, sensors, etc.).

However, for some applications, the administering apparatus 2 can be more basic. This allows reducing the size and the complexity of use of the administering apparatus 2.

In one embodiment the device can be composed of a set of different administering apparatuses 2, each administering apparatus 2 of the set being preprogrammed with a single predetermined injection profile that is different from the other administering apparatus 2 of the set.

In this case, each administering apparatus 2 comprises a housing 21, a reservoir 22, a drive mechanism 23, a basic control unit 24, a power source 25, and an injection set 26. The basic control unit 24 of each administering apparatus 2 comprises: a memory storing a single predetermined injection profile, optionally an alarm, optionally data entry means, and a processor controlling the drive mechanism 23 according to the preprogrammed injection profile stored in the memory of the administering apparatus 2.

With such a basic administering apparatus 2, the patient can act by stopping (ON/OFF) the injection of the composition 1 for a given period of time (for example 1 hour, 2 hours, 1 day, etc.), for instance when he feels side effects. To this end, the administering apparatus 2 can include an actuation element - such as a button - that allows deactivating the administering apparatus 2 for a short period of time when said actuation element is pressed. After this short time period, the administering apparatus 2 is automatically reactivated and the continuous administering of the composition 1 restarts. In some embodiments of the invention, the given period of time can be defined by the patient (for example by using data entry means of the administering apparatus 2). Alternately to the stopping of the injection procedure, the patient can request a decrease of the injection rate of the composition (for instance a decrease of 10%, 20%, etc.) by using the data entry means. The skilled person will appreciate that the decreasing (or stopping) of the injection procedure can be implemented progressively, according to different stages of rates decreasing as a function of time, or according to a substantially constant rate ramp down.

Advantageously, if the patient stops the injection of the composition 1 for a given period of time that exceeds a time threshold, the administering apparatus 2 can be configured to:
- activate an alarm (visuable and/or tactile and/or audible stimuli) in order to warn the patient that the treatment has been interrupted for a too long period of time, and that the injection of the composition must be restarted; and/or to
- automatically restart the injection of the composition.

The restart of the injection of the composition can be implemented according to different stages of rates increasing as a function of time, or according to a substantially constant rate ramp up in order to progressively increase the amount of composition that is injected to the patient.

Before each replacement of the administering apparatus 2 (for instance when the reservoir is empty), a practitioner determines whether an identical administering apparatus 2, or an administering apparatus 2 designed with another injection profile (defining a higher/lower injection rate) must be provided to the patient.

The above disclosed device allows an efficient treatment for a patient while limiting the side effects associated with the administration of the composition 1.

Advantageously, the different injection profiles (preprogrammed or calculated by the supervisory terminal 3) are defined in a range of rates that are:
- above an efficiency threshold, and preferably
- below a discomfort threshold,
in order to provide the patient with a solution that does not cause a problem of tolerability of the medication.

The skilled person will have understood that many modifications may be provided to the invention described above without materially departing from the new teachings and advantages described here.

### EXAMPLES

### Part A - Synthesis of the hydrophobic copolyamino acids

The intermediate hydrophobic compounds Hy and the copolyamino acids disclosed below can be synthesized according to the procedures described in WO2019110773, WO2019110837, WO2019110788, WO2019110797, WO2019110836, WO2019110774, WO202011533 and WO2020245470.

### i) Co-polyamino acids according to formula XXXa

**Table 1: Copolyamino acids according to formula XXXa**

| No. | CO-POLYAMINO ACIDS BEARING CARBOXYLATE CHARGES AND HYDROPHOBIC RADICALS |
|---|---|
| B1 | 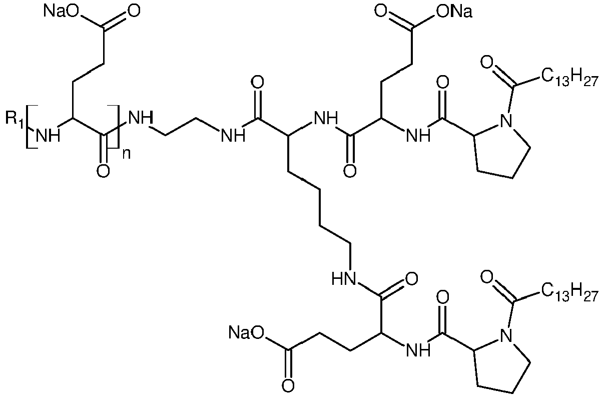 |
| | i=0,043, DP = 23 |
| | R₁ = H or pyroglutamate |
| B2 | 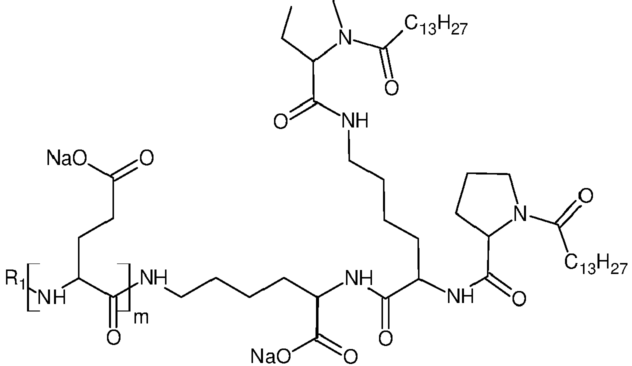 |
| | i = 0.038, DP = 26 |
| | R₁ = H or pyroglutamate |
| B3 | 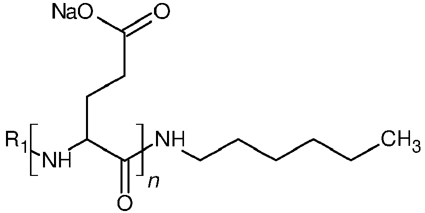 |
| | i = 0,042, DP = 24 |
| | R₁ = |
| | 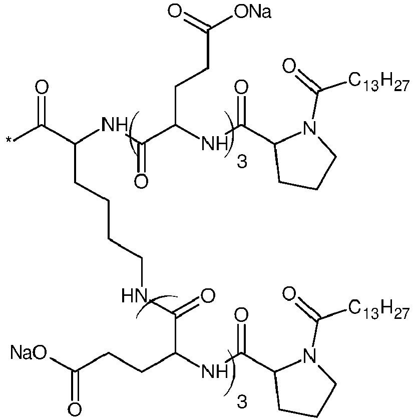 |
| B4 | 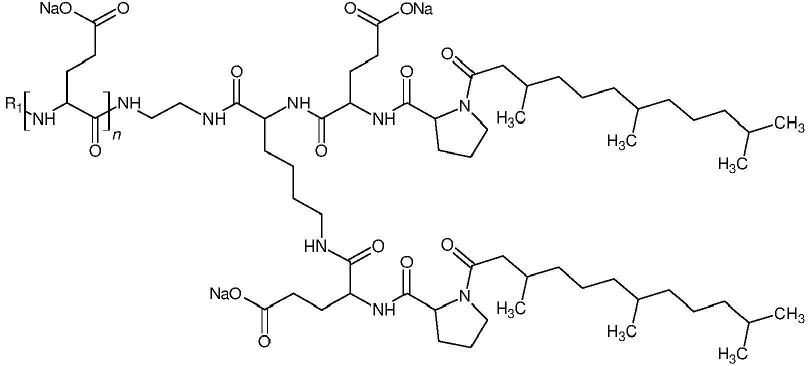 |
| | i = 0.042, DP = 24 |
| | R₁ = H or pyroglutamate |
| B5 | 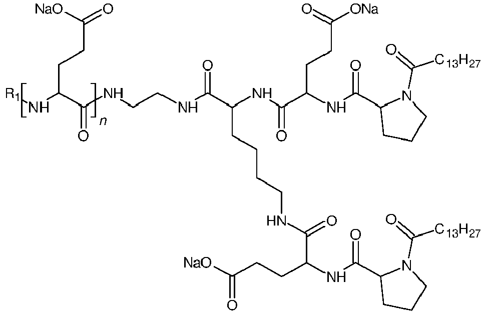 |
| | i = 0.1, DP = 10 |
| | R₁ = H or pyroglutamate |
| B6 | 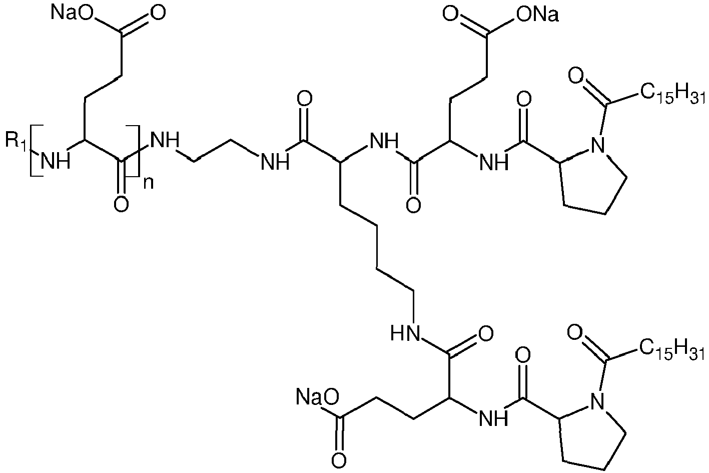 |
| | i = 0,042, DP = 24 |
| | R₁ = H or pyroglutamate |
| B7 | 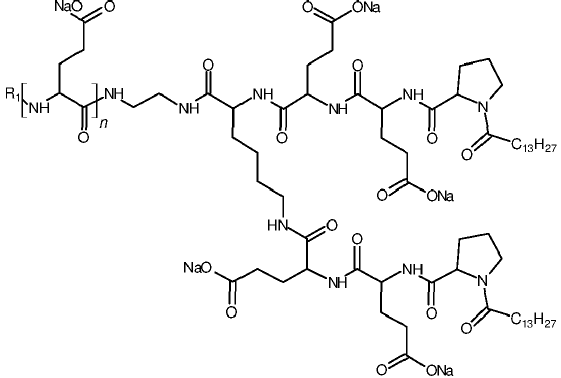 |
| | i=0,043, DP = 23 |
| | R₁ = H or pyroglutamate |

### ii) Co-polyamino acids according to formula XXXb

**Table 2: Copolyamino acids according to formula XXXb**

| | |
|---|---|
| B8 | 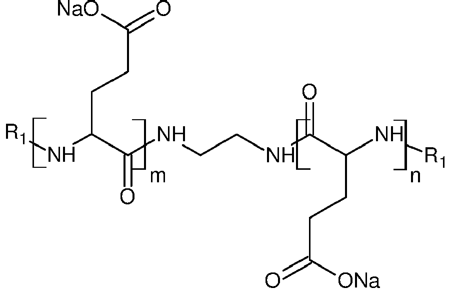 |
| | i= 0,0833, DP (m + n) = 24 |
| | R₁ = H, pyroglutamate or |
| | 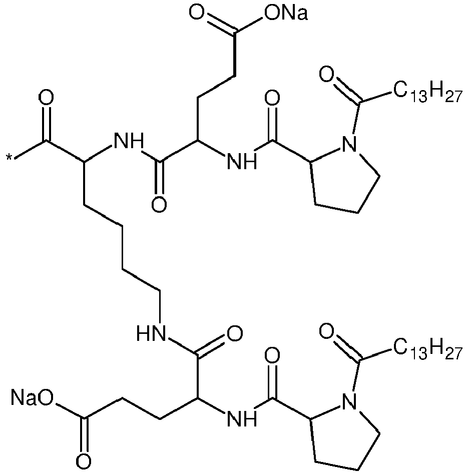 |
| B9 | 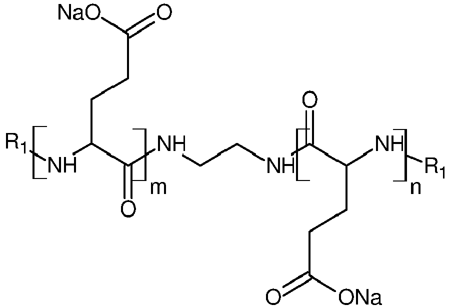 |
| | i= 0,079, DP (m + n) = 24 |
| | R₁ = H, pyroglutamate or |
| | 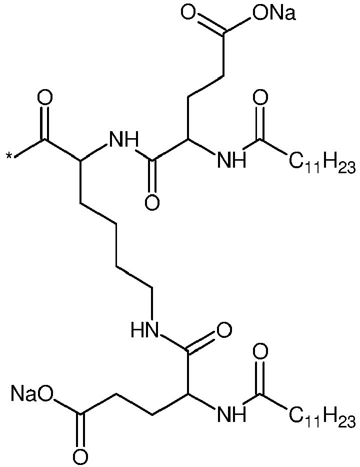 |
| B10 | 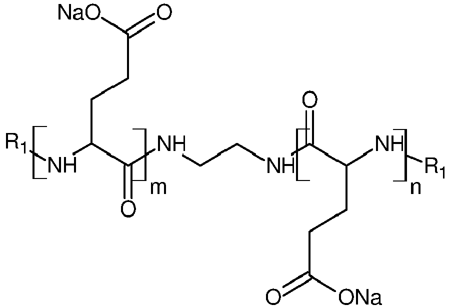 |
| | i= 0,072, DP (m + n) = 24 |
| | R₁ = H, pyroglutamate or |
| | 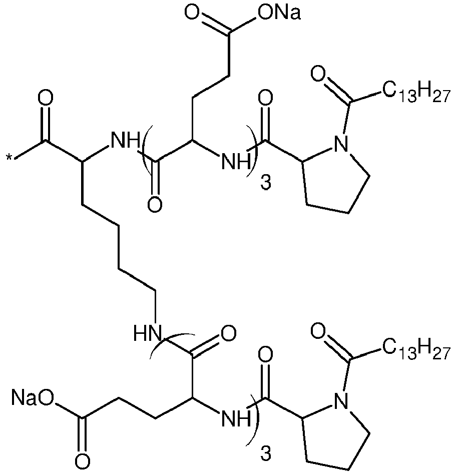 |
| B11 | 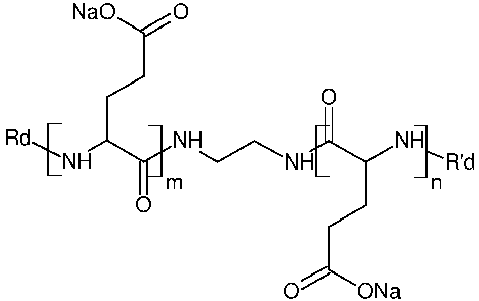 |
| | i=0,083, DP (m + n) = 24 |
| | R_{d}, R_{'d} = H, pyroglutamate or |
| | 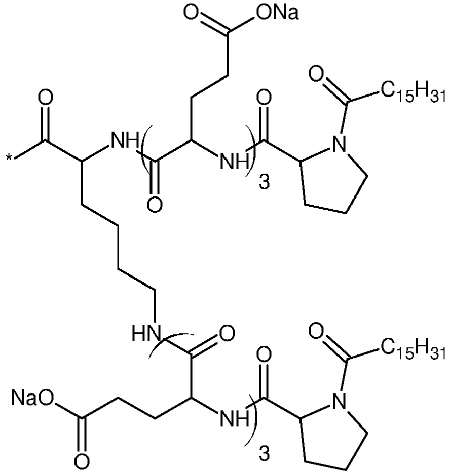 |

### Part B - Compositions

In the following examples, pramlintide, short acting amylin RA, exenatide, lixisenatide, human glucagon and dasiglucagon, PYY and GIP RA have been obtained through peptide synthesis.

### 1 - Compositions comprising pramlintide and a GLP-1 receptor agonist (GLP-1 RA)

Example C7 : Preparation of solutions of pramlintide in combination with exenatide or lixisenatide at pH 4.

A stock solution of pramlintide at pH 2.0 - 3.0 was prepared using pramlintide as a powder, water was added and hydrochloric acid was used for pH adjustment.

A stock solution of exenatide or lixisenatide was prepared using exenatide or lixisenatide as powder, water was added and the pH was not adjusted.

In a container, stock solutions of excipients were added to water: m-cresol, mannitol or glycerol, and acetate buffer. The concentrations of m-cresol, mannitol, glycerol and acetate buffer were chosen to reach the desired concentrations in the compositions described in table 3. Then were added in the following order: exenatide or lixisenatide stock solution then pramlintide stock solution. The pH was adjusted to 3.7 - 4.0 by addition of a solution of hydrochloric acid or of sodium hydroxide.

The compositions obtained are detailed in table 3. The pH was adjusted to 4 by addition of a solution of hydrochloric acid or of sodium hydroxide.

| Compo sition | Pramlint ide (mg/m L) | Exenatide (µg/mL) | Lixisenatide (µg/mL) | Excipients |
|---|---|---|---|---|
| C7-1 | 0.6 | 24 | - | m-cresol (20 mM), Acetate buffer (20 mM), Mannitol (250 mM) |
| C7-2 | 0.6 | 36 | - | m-cresol (20 mM), Acetate buffer (20 mM), Mannitol (250 mM) |
| C7-3 | 0.6 | 48 | - | m-cresol (20 mM), Acetate buffer (20 mM), Mannitol (250 mM) |
| C7-4 | 0.6 | 60 | - | m-cresol (20 mM), Acetate buffer (20 mM), Mannitol (250 mM) |
| C7-5 | 0.6 | 72 | - | m-cresol (20 mM), Acetate buffer (20 mM), Mannitol (250 mM) |
| C7-6 | 1 | 40 | - | m-cresol (20 mM), Acetate buffer (20 mM), Mannitol (250 mM) |
| C7-7 | 1 | 60 | - | m-cresol (20 mM), Acetate buffer (20 mM), Mannitol (250 mM) |
| C7-8 | 1 | 80 | - | m-cresol (20 mM), Acetate buffer (20 mM), Mannitol (250 mM) |
| C7-9 | 1 | 100 | - | m-cresol (20 mM), Acetate buffer (20 mM), Mannitol (250 mM) |
| C7-10 | 1 | 120 | - | m-cresol (20 mM), Acetate buffer (20 mM), Mannitol (250 mM) |
| C7-11 | 2 | 80 | - | m-cresol (20 mM), Acetate buffer (20 mM), Mannitol (250 mM) |
| C7-12 | 2 | 120 | - | m-cresol (20 mM), Acetate buffer (20 mM), Mannitol (250 mM) |
| C7-13 | 2 | 160 | - | m-cresol (20 mM), Acetate buffer (20 mM), Mannitol (250 mM) |
| C7-14 | 2 | 200 | - | m-cresol (20 mM), Acetate buffer (20 mM), Mannitol (250 mM) |
| C7-15 | 2 | 240 | - | m-cresol (20 mM), Acetate buffer (20 mM), Mannitol (250 mM) |
| C7-16 | 3 | 120 | - | m-cresol (20 mM), Acetate buffer (20 mM), Mannitol (250 mM) |
| C7-17 | 3 | 180 | - | m-cresol (20 mM), Acetate buffer (20 mM), Mannitol (250 mM) |
| C7-18 | 3 | 240 | - | m-cresol (20 mM), Acetate buffer (20 mM), Mannitol (250 mM) |
| C7-19 | 3 | 300 | - | m-cresol (20 mM), Acetate buffer (20 mM), Mannitol (250 mM) |
| C7-20 | 3 | 360 | - | m-cresol (20 mM), Acetate buffer (20 mM), Mannitol (250 mM) |
| C7-21 | 4 | 160 | - | m-cresol (20 mM), Acetate buffer (20 mM), Mannitol (250 mM) |
| C7-22 | 4 | 240 | - | m-cresol (20 mM), Acetate buffer (20 mM), Mannitol (250 mM) |
| C7-23 | 4 | 320 | - | m-cresol (20 mM), Acetate buffer (20 mM), Mannitol (250 mM) |
| C7-24 | 4 | 400 | - | m-cresol (20 mM), Acetate buffer (20 mM), Mannitol (250 mM) |
| C7-25 | 4 | 460 | - | m-cresol (20 mM), Acetate buffer (20 mM), Mannitol (250 mM) |
| C8-1a | 0.6 | - | 60 | m-cresol (20 mM), Acetate buffer (20 mM), Mannitol (250 mM) |
| C8-1b | 0.6 | - | 90 | m-cresol (20 mM), Acetate buffer (20 mM), Mannitol (250 mM) |
| C8-2 | 0.6 | - | 120 | m-cresol (20 mM), Acetate buffer (20 mM), Mannitol (250 mM) |
| C8-3 | 0.6 | - | 150 | m-cresol (20 mM), Acetate buffer (20 mM), Mannitol (250 mM) |
| C8-4 | 0.6 | - | 180 | m-cresol (20 mM), Acetate buffer (20 mM), Mannitol (250 mM) |
| C8-5 | 1 | - | 100 | m-cresol (20 mM), Acetate buffer (20 mM), Mannitol (250 mM) |
| C8-6 | 1 | - | 150 | m-cresol (20 mM), Acetate buffer (20 mM), Mannitol (250 mM) |
| C8-7 | 1 | - | 200 | m-cresol (20 mM), Acetate buffer (20 mM), Mannitol (250 mM) |
| C8-8 | 1 | - | 250 | m-cresol (20 mM), Acetate buffer (20 mM), Mannitol (250 mM) |
| C8-9 | 1 | - | 300 | m-cresol (20 mM), Acetate buffer (20 mM), Mannitol (250 mM) |
| C8-10 | 2 | - | 200 | m-cresol (20 mM), Acetate buffer (20 mM), Mannitol (250 mM) |
| C8-11 | 2 | - | 300 | m-cresol (20 mM), Acetate buffer (20 mM), Mannitol (250 mM) |
| C8-12 | 2 | - | 400 | m-cresol (20 mM), Acetate buffer (20 mM), Mannitol (250 mM) |
| C8-13 | 2 | - | 500 | m-cresol (20 mM), Acetate buffer (20 mM), Mannitol (250 mM) |
| C8-14 | 2 | - | 600 | m-cresol (20 mM), Acetate buffer (20 mM), Mannitol (250 mM) |
| C8-15 | 3 | - | 300 | m-cresol (20 mM), Acetate buffer (20 mM), Mannitol (250 mM) |
| C8-16 | 3 | - | 450 | m-cresol (20 mM), Acetate buffer (20 mM), Mannitol (250 mM) |
| C8-17 | 3 | - | 600 | m-cresol (20 mM), Acetate buffer (20 mM), Mannitol (250 mM) |
| C8-18 | 3 | - | 750 | m-cresol (20 mM), Acetate buffer (20 mM), Mannitol (250 mM) |
| C8-19 | 3 | - | 900 | m-cresol (20 mM), Acetate buffer (20 mM), Mannitol (250 mM) |
| C8-20 | 4 | - | 400 | m-cresol (20 mM), Acetate buffer (20 mM), Mannitol (250 mM) |
| C8-21 | 4 | - | 600 | m-cresol (20 mM), Acetate buffer (20 mM), Mannitol (250 mM) |
| C8-22 | 4 | - | 800 | m-cresol (20 mM), Acetate buffer (20 mM), Mannitol (250 mM) |
| C8-23 | 4 | - | 1000 | m-cresol (20 mM), Acetate buffer (20 mM), Mannitol (250 mM) |
| C8-24 | 4 | - | 1200 | m-cresol (20 mM), Acetate buffer (20 mM), Mannitol (250 mM) |
| Table 3: compositions according to the invention C7-1 to C7-25 and C8-1a to C8-24. | | | | |

### 1A - Compositions comprising a short acting amylin RA and a GLP-1 receptor agonist (GLP-1 RA)

### Exemple C1A' : Preparation of 2 mg/mL short acting amylin RA compositions at pH 4

Short acting amylin RA powder (obtained by peptide synthesis) is dissolved with a 10 mM HCl solution. An acetate buffer solution is then added to give the composition C1A'. The short acting amylin RA are as follows detailed in Table 3a.

**Table 3a: Compositions C1A'-1 to C1A'-5**

| Composition | Short acting amylin RA Formula (Cpd) |
|---|---|
| C1A'-1 | (Cpd 5) |
| C1A'-2 | (Cpd 6) |
| C1A'-3 | (Cpd 8) |
| C1A'-4 | (Cpd 9) |
| C1A'-5 | (Cpd 11) |

### Example C1A" : Preparation of solutions of short acting amylin RA in combination with exenatide or lixisenatide at pH 4.

Compositions comprising an amylin RA and exenatide /or lixisenatide are prepared according to the protocol described in example C7. The compositions obtained are disclosed in table 3b.

**Table 3b: Compositions C1A"-1 to C1A"-10 comprising a short acting amylin RA and exenatide or lixisenatide at pH 4.**

| Compo sition | Amylin RA (mg/m L) | Exenatide (µg/mL) | Lixisenatide (µg/mL) | Excipients |
|---|---|---|---|---|
| C1A"-1 | Cpd 5 (1) | 100 | - | m-cresol (20 mM), Acetate buffer (20 mM), Mannitol (250 mM) |
| C1A"-2 | Cpd 6 (1) | 100 | - | m-cresol (20 mM), Acetate buffer (20 mM), Mannitol (250 mM) |
| C1A"-3 | Cpd 8 (1) | 100 | - | m-cresol (20 mM), Acetate buffer (20 mM), Mannitol (250 mM) |
| C1A"-4 | Cpd 9 (1) | 100 | - | m-cresol (20 mM), Acetate buffer (20 mM), Mannitol (250 mM) |
| C1A"-5 | Cpd 11 (1) | 100 | - | m-cresol (20 mM), Acetate buffer (20 mM), Mannitol (250 mM) |
| C1A"-6 | Cpd 5 (1) | - | 250 | m-cresol (20 mM), Acetate buffer (20 mM), Mannitol (250 mM) |
| C1A"-7 | Cpd 6 (1) | - | 250 | m-cresol (20 mM), Acetate buffer (20 mM), Mannitol (250 mM) |
| C1A"-8 | Cpd 8 (1) | - | 250 | m-cresol (20 mM), Acetate buffer (20 mM), Mannitol (250 mM) |
| C1A"-9 | Cpd 9 (1) | - | 250 | m-cresol (20 mM), Acetate buffer (20 mM), Mannitol (250 mM) |
| C1A"-10 | Cpd 11 (1) | - | 250 | m-cresol (20 mM), Acetate buffer (20 mM), Mannitol (250 mM) |

### 1B - Compositions comprising pramlintide, a GLP-1 receptor agonist (GLP-1 RA) and PYY(3-36)

Exemple C1B': Preparation of 2 mg/mL PYY(3-36) composition at pH 4 (Compositions C1B').

PYY(3-36) powder (obtained by peptide synthesis) is dissolved with a 10 mM HCl solution. An acetate buffer solution is then added to give the composition C1B'.

Example C1B": Preparation of solutions of pramlintide, a GLP-1 RA and PYY(3-36) at pH 4.

Stock solutions of pramlintide, GLP-1 RA and PYY(3-36) respectively described in examples C7 and C1B' are mixed to give the compositions detailed in the table below.

**Table 3c: Compositions C1B"-1 to C1B"-4 comprising pramlintide, exenatide or lixisenatide and PYY(3-36) at pH 4.**

| Compo sition | Pramlint ide (mg/m L) | GLP-1 RA (µg/mL) | PYY(3-36) (mg/mL) | Excipients |
|---|---|---|---|---|
| C1B"-1 | 1 | Exenatide (100) | 0.2 | m-cresol (20 mM), Acetate buffer (20 mM), Mannitol (250 mM) |
| C1B"-2 | 1 | Lixisenatid e (250) | 0.2 | m-cresol (20 mM), Acetate buffer (20 mM), Mannitol (250 mM) |
| C1B"-3 | 1.5 | Exenatide (200) | 0.2 | m-cresol (20 mM), Acetate buffer (20 mM), Mannitol (250 mM) |
| C1B"-4 | 1.5 | Lixisenatid e (670) | 0.2 | m-cresol (20 mM), Acetate buffer (20 mM), Mannitol (250 mM) |

### 1C - Compositions comprising pramlintide, a GLP-1 receptor agonist (GLP-1 RA) and a short-acting GIP

Exemple C1C' : Preparation of 2 mg/mL GIP composition at pH 4 (Compositions C1B').

GIP powder (obtained by peptide synthesis) is dissolved with a 10 mM HCl solution. An acetate buffer solution is then added to give the composition C1C'.

Example C1C": Preparation of solutions of pramlintide, a GLP-1 RA and GIP at pH 4.

Stock solutions of pramlintide, GLP-1 RA and GIP respectively described in examples C7 and C1C' are mixed to give the compositions detailed in the table below.

**Table 3d: Compositions C1C"-1 to C1C"-4 comprising pramlintide, exenatide or lixisenatide and cagrilintide at pH 4.**

| Compo sition | Pramlint ide (mg/m L) | GLP-1 RA (µg/mL) | GIP (mg/mL) | Excipients |
|---|---|---|---|---|
| C1C"-1 | 1 | Exenatide (100) | Cpd 19 (0.2) | m-cresol (20 mM), Acetate buffer (20 mM), Mannitol (250 mM) |
| C1C"-2 | 1 | Lixisenatid e (250) | Cpd 19 (0.2) | m-cresol (20 mM), Acetate buffer (20 mM), Mannitol (250 mM) |
| C1C"-3 | 1.5 | Exenatide (200) | Cpd 19 (0.2) | m-cresol (20 mM), Acetate buffer (20 mM), Mannitol (250 mM) |
| C1C"-4 | 1.5 | Lixisenatid e (670) | Cpd 19 (0.2) | m-cresol (20 mM), Acetate buffer (20 mM), Mannitol (250 mM) |

2 - Compositions comprising pramlintide, a GLP-1 receptor agonist (GLP-1 RA), and a glucagon receptor agonist (glucagon RA).

Example C9: Preparation of compositions comprising pramlintide, a GLP-1 RA and/or a glucagon RA.

A stock solution of pramlintide at pH 2.0 - 3.0 was prepared using pramlintide as a powder, water was added and hydrochloric acid was used for pH adjustment.

A stock solution of GLP-1 RA was prepared using a GLP-1 RA as powder, water was added and the pH was not adjusted.

A stock solution of glucagon receptor agonist at pH 2.5 - 3.5 was prepared using glucagon RA as powder, water was added and hydrochloric acid was used for pH adjustment.

In a container, stock solutions of excipients were added to water: m-cresol, mannitol or glycerol, and acetate buffer. The concentrations of m-cresol, mannitol, glycerol and acetate buffer were chosen to reach the desired concentrations in the compositions described in table 4. Then were added in the following order: GLP-1 RA stock solution, glucagon RA stock solution and pramlintide stock solution. The pH was adjusted to 3.7 - 4.0 by addition of a solution of hydrochloric acid or of sodium hydroxide.

The compositions obtained are detailed in table 4. The pH was adjusted to 4 by addition of a solution of hydrochloric acid or of sodium hydroxide.

**Table 4: Compositions comprising pramlintide, a GLP-1 RA and/or a glucagon RA prepared according to example C9**

| Compo sition | Pramlinti de (mg/m L) | GLP-1 RA (µg/mL) | Glucagon RA (mg/mL) | Molar ratio glucagon RA / GLP-1 RA | Excipients |
|---|---|---|---|---|---|
| C9-1 | 1.5 | Exenatide (200) | 0 | - | m-cresol (25 mM), Acetate buffer (30 mM), Mannitol (236 mM) |
| C9-2 | 1.5 | Lixisenati de (670) | 0 | - | m-cresol (25 mM), Acetate buffer (30 mM), Glycerol (184 mM) |
| C9-3 | 0 | Exenatide (100) | Dasigluca gon (1) | 12.4 | m-cresol (25 mM), Acetate buffer (30 mM), Glycerol (184 mM) |
| C9-4 | 0 | Lixisenati de (330) | Dasigluca gon (1) | 4.4 | m-cresol (25 mM), Acetate buffer (30 mM), Glycerol (184 mM) |
| C9-5 | 1.5 | 0 | Dasigluca gon (1) | - | m-cresol (25 mM), Acetate buffer (30 mM), Glycerol (184 mM) |
| C9-6 | 1.5 | Exenatide (200) | Dasigluca gon (1) | 6.2 | m-cresol (25 mM), Acetate buffer (30 mM), Glycerol (184 mM) |
| C9-7 | 1.5 | Lixisenati de (330) | Dasigluca gon (1) | 4.4 | m-cresol (25 mM), Acetate buffer (30 mM), Glycerol (184 mM) |

Compositions according to the invention allowed the formation of a co-formulation of pramlintide and/or GLP-1 RA and/or dasiglucagon at acidic pH.

2A - Compositions comprising a short acting amylin RA, a short-acting GLP-1 receptor agonist (GLP-1 RA) and a short-acting glucagon receptor agonist (glucagon RA).

Example C2A" : Preparation of solutions of short acting amylin RA, a short-acting GLP-1 RA and a short-acting glucagon RA.

Compositions comprising a short-acting amylin RA, a short acting GLP-1 RA and a short-acting glucagon RA are prepared according to the protocol described in example C9. The compositions obtained are disclosed in table 4a.

**Table 4a : Compositions comprising a short-acting amylin RA, a short-acting GLP-1 RA and a short-acting glucagon RA prepared according to example C2A"**

| Compo sition | Short-acting amylin RA (mg/m L) | GLP-1 RA (µg/mL) | Glucagon RA (mg/mL) | Molar ratio glucagon RA / GLP-1 RA | Excipients |
|---|---|---|---|---|---|
| C2A"-1 | Cpd 5 (1.5) | 0 | Dasigluca gon (1) | - | m-cresol (25 mM), Acetate buffer (30 mM), Glycerol (184 mM) |
| C2A"-2 | Cpd 6 (1.5) | 0 | Dasigluca gon (1) | - | m-cresol (25 mM), Acetate buffer (30 mM), Glycerol (184 mM) |
| C2A"-3 | Cpd 8 (1.5) | 0 | Dasigluca gon (1) | - | m-cresol (25 mM), Acetate buffer (30 mM), Glycerol (184 mM) |
| C2A"-4 | Cpd 9 (1.5) | 0 | Dasigluca gon (1) | - | m-cresol (25 mM), Acetate buffer (30 mM), Glycerol (184 mM) |
| C2A"-5 | Cpd 11 (1.5) | 0 | Dasigluca gon (1) | - | m-cresol (25 mM), Acetate buffer (30 mM), Glycerol (184 mM) |
| C2A"-6 | Cpd 5 (1.5) | Exenatide (200) | Dasigluca gon (1) | 6.2 | m-cresol (25 mM), Acetate buffer (30 mM), Glycerol (184 mM) |
| C2A"-7 | Cpd 6 (1.5) | Exenatide (200) | Dasigluca qon (1) | 6.2 | m-cresol (25 mM), Acetate buffer (30 mM), Glycerol (184 mM) |
| C2A"-8 | Cpd 8 (1.5) | Exenatide (200) | Dasigluca gon (1) | 6.2 | m-cresol (25 mM), Acetate buffer (30 mM), Glycerol (184 mM) |
| C2A"-9 | Cpd 9 (1.5) | Exenatide (200) | Dasigluca gon (1) | 6.2 | m-cresol (25 mM), Acetate buffer (30 mM), Glycerol (184 mM) |
| C2A"-10 | Cpd 11 (1.5) | Exenatide (200) | Dasigluca gon (1) | 6.2 | m-cresol (25 mM), Acetate buffer (30 mM), Glycerol (184 mM) |
| C2A"-11 | Cpd 5 (1.5) | Lixisenati de (330) | Dasigluca gon (1) | 4.4 | m-cresol (25 mM), Acetate buffer (30 mM), Glycerol (184 mM) |
| C2A"-12 | Cpd 6 (1.5) | Lixisenati de (330) | Dasigluca gon (1) | 4.4 | m-cresol (25 mM), Acetate buffer (30 mM), Glycerol (184 mM) |
| C2A"-13 | Cpd 8 (1.5) | Lixisenati de (330) | Dasigluca gon (1) | 4.4 | m-cresol (25 mM), Acetate buffer (30 mM), Glycerol (184 mM) |
| C2A"-14 | Cpd 9 (1.5) | Lixisenati de (330) | Dasigluca gon (1) | 4.4 | m-cresol (25 mM), Acetate buffer (30 mM), Glycerol (184 mM) |
| C2A"-15 | Cpd 11 (1.5) | Lixisenati de (330) | Dasigluca gon (1) | 4.4 | m-cresol (25 mM), Acetate buffer (30 mM), Glycerol (184 mM) |

2B - Compositions comprising pramlintide and/or a short-acting GLP-1 receptor agonist (GLP-1 RA) and/or a short-acting glucagon receptor agonist (glucagon RA) and PYY(3-36).

Example C2B" : Preparation of compositions comprising pramlintide and/or a short-acting GLP-1 receptor agonist (GLP-1 RA) and/or a short-acting glucagon receptor agonist (glucagon RA) and PYY(3-36).

Stock solutions of pramlintide, GLP-1 RA, glucagon RA and PYY(3-36) respectively described in examples C9 and C1B' are mixed to give the compositions detailed in the table below.

**Table 4b: Compositions comprising pramlintide and/or a GLP-1 receptor agonist (GLP-1 RA) and/or a glucagon receptor agonist (glucagon RA) and PYY(3-36)**

| Comp ositi on | Pramlin tide (mg/ mL) | GLP-1 RA (µg/mL ) | Glucagon RA (mg/mL) | PYY(3-36) (mg/m L) | Molar ratio glucagon RA / GLP-1 RA | Excipients |
|---|---|---|---|---|---|---|
| C9-3 | 0 | Exenatid e (100) | Dasigluca gon (1) | 0.2 | 12.4 | m-cresol (25 mM), Acetate buffer (30 mM), Glycerol (184 mM) |
| C9-4 | 0 | Lixisenat ide (330) | Dasigluca gon (1) | 0.2 | 4.4 | m-cresol (25 mM), Acetate buffer (30 mM), Glycerol (184 mM) |
| C9-5 | 1.5 | 0 | Dasigluca gon (1) | 0.2 | - | m-cresol (25 mM), Acetate buffer (30 mM), Glycerol (184 mM) |
| C2A"-4 | 1.5 | Exenatid e (200) | Dasigluca gon (1) | 0.2 | 6.2 | m-cresol (25 mM), Acetate buffer (30 mM), Glycerol (184 mM) |
| C2A"-7 | 1.5 | Lixisenat ide (330) | Dasigluca gon (1) | 0.2 | 4.4 | m-cresol (25 mM), Acetate buffer (30 mM), Glycerol (184 mM) |

2C - Compositions comprising pramlintide and/or a GLP-1 receptor agonist (GLP-1 RA) and/or a glucagon receptor agonist (glucagon RA) and a short acting GIP.

Example C2B" : Preparation of compositions comprising pramlintide and/or a GLP-1 receptor agonist (GLP-1 RA) and/or a glucagon receptor agonist (glucagon RA) and GIP.

Stock solutions of pramlintide, GLP-1 RA, glucagon RA and GIP respectively described in examples C9 and C1C' are mixed to give the compositions detailed in the table below.

**Table 4c: Compositions comprising pramlintide and/or a GLP-1 receptor agonist (GLP-1 RA) and/or a glucagon receptor agonist (glucagon RA) and a short acting GIP.**

| Comp ositi on | Pramlinti de (mg/m L) | GLP-1 RA (µg/mL ) | Glucagon RA (mg/mL) | GIP (mg/ mL) | Molar ratio glucag on RA / GLP-1 RA | Excipients |
|---|---|---|---|---|---|---|
| C9-3 | 0 | Exenatid e (100) | Dasiglucag on (1) | Cpd 19 (0.2) | 12.4 | m-cresol (25 mM), Acetate buffer (30 mM), Glycerol (184 mM) |
| C9-4 | 0 | Lixisenat ide (330) | Dasiglucag on (1) | Cpd 19 (0.2) | 4.4 | m-cresol (25 mM), Acetate buffer (30 mM), Glycerol (184 mM) |
| C9-5 | 1.5 | 0 | Dasiglucag on (1) | Cpd 19 (0.2) | - | m-cresol (25 mM), Acetate buffer (30 mM), Glycerol (184 mM) |
| C2A"-4 | 1.5 | Exenatid e (200) | Dasiglucag on (1) | Cpd 19 (0.2) | 6.2 | m-cresol (25 mM), Acetate buffer (30 mM), Glycerol (184 mM) |
| C2A"-7 | 1.5 | Lixisenat ide (330) | Dasiglucag on (1) | Cpd 19 (0.2) | 4.4 | m-cresol (25 mM), Acetate buffer (30 mM), Glycerol (184 mM) |

Example C10: Compositions comprising a GLP-1 RA and a glucagon RA at neutral pH.

A solution of exenatide was prepared using exenatide as powder. Water was added and the pH was not adjusted. This solution was gently stirred until the solutions was visually free of particulate matter.

A solution of dasiglucagon at pH 2.5 ― 3.5 was prepared using dasiglucagon as powder, water was added and hydrochloric acid for pH adjustment. After complete dissolution of dasiglucagon, this solution was then adjusted to pH 7 by addition of sodium hydroxide and gently stirred until the solution was visually free of particulate matter.

In a container, stock solutions of excipients are added to water: *m*-cresol and glycerol. The concentrations of *m*-cresol and glycerol were chosen to reach the desired concentrations in the compositions described in table 5. Then were added in the following order: the solution of dasiglucagon and a solution of exenatide. If necessary, the pH was adjusted to 7 by addition of a solution of hydrochloric acid or sodium hydroxide.

**Table 5: Compositions comprising a GLP-1 RA and a glucagon RA prepared according to example C10.**

| Composition | Exenatide (µg/mL) | Dasiglucagon (mg/mL) | Molar ratio glucagon RA / GLP-1 RA | Excipients | pH |
|---|---|---|---|---|---|
| C10-1 | 200 | 1 | 6.2 | *m*-cresol (29 mM) Glycerol (174 mM) | 7 |

Compositions according to the invention allowed the formation of a coformulation of exenatide and dasiglucagon at neutral pH.

Example C10A: Compositions comprising a GLP-1 RA, a glucagon RA and PYY(3-36) at neutral pH.

A solution of exenatide was prepared using exenatide as powder. Water was added and the pH was not adjusted. This solution was gently stirred until the solutions was visually free of particulate matter.

A solution of dasiglucagon at pH 2.5 ― 3.5 was prepared using dasiglucagon as powder, water was added and hydrochloric acid for pH adjustment. After complete dissolution of dasiglucagon, this solution was then adjusted to pH 7 by addition of sodium hydroxide and gently stirred until the solution was visually free of particulate matter.

A solution of PYY(3-36) was prepared using PYY(3-36) as powder. Water was added and the pH was not adjusted. This solution was gently stirred until the solutions was visually free of particulate matter.

In a container, stock solutions of excipients are added to water: m-cresol and glycerol. The concentrations of m-cresol and glycerol were chosen to reach the desired concentrations in the compositions described in table 5. Then were added in the following order: the solution of dasiglucagon and a solution of exenatide and the solution of PYY(3-36). If necessary, the pH was adjusted to 7 by addition of a solution of hydrochloric acid or sodium hydroxide.

**Table 5a: Compositions comprising a GLP-1 RA, a glucagon RA and PYY(3-36) prepared according to example C10A.**

| Composition | Exenatide (µg/mL) | Dasiglucagon (mg/mL) | PYY(3-36) (mg/mL) | Molar ratio glucagon RA / GLP-1 RA | Excipients | pH |
|---|---|---|---|---|---|---|
| C10-1 | 200 | 1 | 1 | 6.2 | *m*-cresol (29 mM) Glycerol (174 mM) | 7 |

Compositions according to the invention allowed the formation of a coformulation of exenatide, dasiglucagon and PYY(3-36) at neutral pH.

Example C10B: Compositions comprising a GLP-1 RA, a glucagon RA and GIP at neutral pH.

A solution of exenatide was prepared using exenatide as powder. Water was added and the pH was not adjusted. This solution was gently stirred until the solutions was visually free of particulate matter.

A solution of dasiglucagon at pH 2.5 ― 3.5 was prepared using dasiglucagon as powder, water was added and hydrochloric acid for pH adjustment. After complete dissolution of dasiglucagon, this solution was then adjusted to pH 7 by addition of sodium hydroxide and gently stirred until the solution was visually free of particulate matter.

A solution of GIP was prepared using GIP as powder. Water was added and the pH was not adjusted. This solution was gently stirred until the solutions was visually free of particulate matter.

In a container, stock solutions of excipients are added to water: m-cresol and glycerol. The concentrations of m-cresol and glycerol were chosen to reach the desired concentrations in the compositions described in table 5. Then were added in the following order: the solution of dasiglucagon and a solution of exenatide and the solution of GIP. If necessary, the pH was adjusted to 7 by addition of a solution of hydrochloric acid or sodium hydroxide.

**Table 5b: Compositions comprising a GLP-1 RA, a glucagon RA and GIP prepared according to example C10A.**

| Composition | Exenatide (µg/mL) | Dasiglucagon (mg/mL) | GIP (mg/mL) | Molar ratio glucagon RA / GLP-1 RA | Excipients | pH |
|---|---|---|---|---|---|---|
| C10-1 | 200 | 1 | Cpd 19 (0.2) | 6.2 | m-cresol (29 mM) | 7 |
| | | | | | Glycerol (174 mM) | |

Compositions according to the invention allowed the formation of a coformulation of exenatide, dasiglucagon and GIP at neutral pH.

Example C11: Solutions comprising pramlintide, a GLP-1 RA and a glucagon RA in DMSO.

A solution of pramlintide at pH 2.0 ― 3.0 was prepared using pramlintide as a powder, water was added and hydrochloric acid was used for pH adjustment.

Solutions of exenatide or lixisenatide were prepared using exenatide or lixisenatide as powder, water was added and the pH was not adjusted.

A solution of glucagon or dasiglucagon at pH 2.5 ― 3.5 was prepared using glucagon or dasiglucagon as powder, water was added and hydrochloric acid was used for pH adjustment.

In a container, DMSO was added to water. The quantity of DMSO was chosen to reach the desired proportion in the compositions described in table 6. Then were added in the following order: the solution of exenatide or lixisenatide, a solution of glucagon or dasiglucagon and a solution of pramlintide. The total quantity of hydrochloric acid was chosen to reach the desired concentration in the compositions described in table 6.

**Table 6: Compositions comprising pramlintide, a GLP-1 RA and/or a glucagon RA prepared according to example C11.**

| Comp ositio n | Pramlintide (mg/mL) | GLP-1 RA (µg/mL) | Glucagon RA (mg/mL) | Molar ratio glucagon RA / GLP-1 RA | DMSO (% v/v) | Hydrochlori c acid (mM) |
|---|---|---|---|---|---|---|
| C11-1 | 1.5 | Exenatide (200) | 0 | | 67 | 7 |
| C11-2 | 1.5 | Lixisenatide (670) | 0 | | 67 | 7 |
| C11-3 | 0 | Exenatide (100) | Human glucagon (1) | 12 | 67 | 7 |
| C11-4 | 0 | Lixisenatide (170) | Human glucagon (1.5) | 12.3 | 67 | 7 |
| C11-5 | 0 | Exenatide (100) | Dasiglucagon (1) | 12.4 | 67 | 7 |
| C11-6 | 0 | Lixisenatide (330) | Dasiglucagon (1) | 4.4 | 67 | 7 |
| C11-7 | 1.5 | 0 | Glucagon (2) | | 67 | 7 |
| C11-8 | 1.5 | 0 | Dasiglucagon (1) | | 67 | 7 |
| C11-9 | 1.5 | Exenatide (200) | Glucagon (2) | 12.1 | 67 | 7 |
| C11-10 | 1.5 | Lixisenatide (330) | Human glucagon (2) | 8.5 | 67 | 7 |
| C11-11 | 1.5 | Exenatide (200) | Dasiglucagon (1) | 6.2 | 67 | 7 |
| C11-12 | 1.5 | Lixisenatide (330) | Dasiglucagon (0.5) | 2.2 | 67 | 7 |

Compositions according to the invention allow the formation of a coformulation of pramlintide, exenatide or lixisenatide, and recombinant human glucagon or dasiglucagon in an aprotic polar solvent system.

3 - Compositions comprising a copolyamino acid, a glucagon RA and a GLP-1 RA.

Example C12: Compositions comprising a copolyamino acid, glucagon and exenatide at neutral pH

A solution of 5 mg/mL L-methionine was prepared using L-methionine as powder, water was added and the pH was not adjusted.

A solution of 1 mg/mL L-methionine was prepared using 5 mg/mL L-methionine stock solution, water was added and the pH was not adjusted.

A stock solution of GLP-1 RA was prepared using GLP-1 RA as powder, 1 mg/mL L-methionine solution was added and the pH was not adjusted.

A solution of 2 mg/mL L-methionine and 0.006 N hydrochloric acid was prepared using 5 mg/mL L-methionine stock solution, water and hydrochloric acid were added and the pH was not adjusted.

A 4 mg/mL stock solution of glucagon was prepared using glucagon as powder, solution of 2 mg/mL L-methionine and 0.006 N hydrochloric acid was added and the pH was not adjusted.

A 2-fold concentrated GLP1-RA /excipients solution was prepared. In a container, stock solutions of excipients were added to water: *m*-cresol, glycerol, and phosphate buffer. The concentrations of *m*-cresol, glycerol and phosphate buffer were chosen to reach twice the desired concentrations in the compositions described in table 7. Then was added the GLP1-RA stock solution.

To a lyophilizate of copolyamino acid, 2-fold concentrated GLP1-RA /excipients stock solution was added. After complete dissolution of the lyophilizate, glucagon stock solution was added. The pH was adjusted to 7.2 by addition of a solution of hydrochloric acid or sodium hydroxide stock solutions.

The obtained compositions are detailed in table 7. All solutions contain 250 mM of glycerol, 27 mM of m-cresol, 2 mM of phosphate buffer and 1 mg/mL of L-methionine. The pH is adjusted to 7.2.

**Table 7: Compositions comprising a copolyamino acid, exenatide or lixisenatide and human glucagon prepared according to example C12**

| Composition | GLP1-RA (µg/mL) | Human glucagon (mg/mL) | Molar ratio glucagon RA / GLP-1 RA | Copolyamino acid (mg/mL) |
|---|---|---|---|---|
| C12-1 | Exenatide (145) | 2 | 16.6 | B1 (7.8) |
| C12-2 | Exenatide (145) | 2 | 16.6 | B1 (9.1) |
| C12-3 | Exenatide (145) | 2 | 16.6 | B10 (11.3) |
| C12-4 | Exenatide (145) | 2 | 16.6 | B8 (7.3) |
| C12-5 | Lixisenatide (240) | 2 | 11.6 | B1 (7.12) |
| C12-6 | Lixisenatide (240) | 2 | 11.6 | B10 (6.4) |
| C12-7 | Lixisenatide (240) | 2 | 11.6 | B8 (4.12) |
| C12-8 | Lixisenatide (240) | 2 | 11.6 | B8 (6.4) |

Administering device accorfing to claim 19, allowing the delivery of the compositions according to any of the claism 1 to 15, according to a therapeutical scheme comprising modulation of the delivered dose.

## Claims

1. Composition comprising at least two short-acting peptides chosen in at least two of the following groups, short-acting GLP-1 RA, short-acting Amylin RA and short-acting Glucagon RA.

2. Composition according to claim 1, for use as a medicament.

3. Composition according to claim 1 or 2, for use in a method of preventing or treating overweight, obesity, and related diseases and/or neurodegenerative diseases and /or Type 2 Diabetes.

4. Composition according to any of claims 1 to 3, wherein the composition is administered in a therapeutical scheme that comprises modulation of the delivered dose, such as increasing and decreasing the delivery, or even stop and restart periods.

5. Composition according to any of claims 1 to 4, wherein it comprises one short-acting GLP-1 RA and one short-acting amylin RA.

6. Composition according to any of claims 1 to 5, wherein it comprises one short-acting GLP-1 RA and one short-acting glucagon RA.

7. Composition according to claim 6, wherein the molar ratio of short-acting GLP-1 RA and short-acting glucagon RA at steady state is comprised betweeen 0.1 to 15

8. Composition according to any of claims 1 to 7, wherein it comprises one short-acting amylin RA and one short-acting glucagon RA.

9. Composition according to any of claims 1 to 8, wherein it comprises one short-acting GLP-1 RA, one short-acting amylin RA and one short-acting glucagon RA.

10. Composition according to any of the claims 1 to 9, wherein GLP-1 receptor agonist (GLP-1 RA), is chosen amongst exenatide and lixisenatide.

11. Composition according to any of the claims 1 to 10, wherein short-acting amylin RA is pramlintide.

12. Composition according to any of the claims 1 to 9, wherein short-acting amylin RA is chosen amongst the peptides according to Formula 1:
**R₁-Z-R₂** Formula 1
wherein
- R₁ is chosen in the group consisting of hydrogen, C₁₋₄ acyl, benzoyl or C₁₋₄ alkyl;
- R₂ is OH or NHR₃, wherein R₃ is hydrogen or C₁₋₃-alkyl; and
- Z is an amino acid sequence according to formula A1 or B1 or C1 or D1 or E1, and,
Formula A1 is the following amino acid sequence as set forth in (SEQ ID NO:3): Xaa1-Cys-Xaa3-Thr-Ala-Thr-Cys-Ala-Thr-Gln-Arg-Leu-Ala-Xaa14-Phe-Leu-Xaa17-Xaa18-Ser-Ser-Xaa21-Xaa22-Phe-Gly-Xaa25-Xaa26-Xaa27-Xaa28-Xaa29-Thr-Xaa31-Val-Gly-Xaa34-Xaa35-Thr-Xaa37 (SEQ ID NO:3) wherein Xaa represents an amino acid and
wherein
- Xaa1 is absent or independently selected from Ala, Cys, Glu, Gly, His, Arg, Ser and Lys;
- Xaa3 is independently selected from Gly, His, Arg, Ser and Asn;
- Xaa14 is independently selected from Asp, Glu, His, Asn, Arg, Gly, Ala, Ser, Lys, Thr and Cys;
- Xaa17 is independently selected from His, Arg, Lys and Val;
- Xaa18 is independently selected from Arg, Lys and His;
- Xaa21 is independently selected from Ala, Lys, Gln, Ser, Asn and Pro;
- Xaa22 is independently selected from Glu, Gln, Ser, Thr and Asn;
- Xaa25 is independently selected from Pro and Ala;
- Xaa26 is independently selected from Pro, Arg and Ile;
- Xaa27 is independently selected from Pro and Leu;
- Xaa28 is independently selected from Pro and Ser;
- Xaa29 is independently selected from Pro and Ser;
- Xaa31 is independently selected from Ser, Glu, Asp, Pro and Asn;
- Xaa34 is independently selected from Pro, His, Lys, Arg and Ser;
- Xaa35 is independently selected from His, Arg, Lys, Asp, Glu, Gln and Asn;
- Xaa37 is independently selected from Pro and Tyr;
- and
Formula B1 is the following amino acid sequence as set forth in (SEQ ID NO:4): X(-1)-X1-X2-X3-X4-X5-X6-X7-X8-X9-X10-X11-X12-X13-X14-X15-X16-X17-X18-X19-X20-X21-X22-X23-X24-X25-X26-X27-X28-X29-X30-X31-X32-X33-X34-X35-X36 (SEQ ID NO: 4)
wherein X represents an amino acid and
wherein
- X(-1) is absent or E,
- X1 is absent or selected from the group consisting of E or A,
- X2 is selected from the group consisting of L, A or P,
- X3 is selected from the group consisting of S or P,
- X4 is selected from the group consisting of E, P, K, Q or G,
- X5 is selected from the group consisting of L, V, or I,
- X6 is S, T or H,
- X7 is T,
- X8 is selected from the group consisting of L or A,
- X9 is selected from the group consisting of A, V, I, S or T,
- X10 is selected from the group consisting of L, A, I, H or V,
- X11 is G,
- X12 is selected from the group consisting of R, H or K,
- X13 is L,
- X14 is selected from the group consisting of S, T or E,
- X15 is selected from the group consisting of A, Q, E, e or T,
- X16 is selected from the group consisting of R, E, K or Q,
- X17 is selected from the group consisting of L or I,
- X18 is selected from the group consisting of H or A,
- X19 is selected from the group consisting of E, R or K,
- X20 is selected from the group consisting of L, I or V,
- X21 is selected from the group consisting of A, Q, S, E or T,
- X22 is T,
- X23 is selected from the group consisting of T, Y or L,
- X24 is P,
- X25 is selected from the group consisting of R, P, H or K,
- X26 is T,
- X27 is selected from the group consisting of E, Q, G or K,
- X28 is selected from the group consisting of T or P,
- X29 is G,
- X30 is selected from the group consisting of P, S or T,
- X31 is selected from the group consisting of E, Q, G, A, P or K,
- X32 is selected from the group consisting of T, S, H, P or A,
- X33 is selected from the group consisting of P, Y, H, F, L, S, G or A,
- X34 is absent or G,
- X35 is absent or T, and
- X36 is absent or Y;
and
Formula C1 is the following amino acid sequence as set forth in (SEQ ID NO:5): X0-X1-Cys()-X3-Thr-Ala-Thr-Cys()-Ala-Thr-X10-Arg-X12-Ala-X14-Phe-X16-X17-X18-X19-X20-X21-X22-X23-X24-X25-X26-X27-X28-X29-X30-X31-Val-Gly-Ser-X35-Thr-X37 (SEQ ID NO: 5) wherein X represents an amino acid and
wherein
- X0 is a basic amino acid or is absent;
- X1 is a basic amino acid or is selected from the group consisting of Ala and Trp;
- X3 is selected from the group consisting of Asn, Gly, Ser, Pro, Asp and Glu;
- X10 is selected from the group consisting of Gln, Gly, Ala, Ser, Leu, Thr, Pro, Asp and Glu;
- X12 is selected from the group consisting of Leu and DLeu;
- X14 is selected from the group consisting of Glu, DGlu, Asp and DAsp;
- X16 is selected from the group consisting of Leu and DLeu, or is absent;
- X17 is selected from the group consisting of His, DHis, Val, DVal, Arg, DArg, Lys, DLys, Orn, DOrn, Dap and Dab, or is absent;
- X18 is selected from the group consisting of His, DHis, Arg, DArg, Dap and Dab, or is absent;
- X19 is selected from the group consisting of Ser and DSer, or is absent;
- X20 is selected from the group consisting of Ser and DSer, or is absent;
- X21 is selected from the group consisting of Asn, DAsn, Glu, DGlu, Asp, DAsp, Ser and DSer, or is absent;
- X22 is selected from the group consisting of Asn, DAsn, Glu, DGlu, Asp, DAsp, Ser and DSer, or is absent;
- X23 is selected from the group consisting of Phe and DPhe, or is absent;
- X24 is selected from the group consisting of Gly(Me), Ala(Me), DAla(Me), Ile(Me), DIle(Me), Ala, DAla, Pro and DPro, or is absent;
- X25 is selected from the group consisting of Ala, DAla, Pro and DPro, or is absent;
- X26 is selected from the group consisting of Gly(Me), Ile(Me), DIle(Me), Ile and DIle, or is absent;
- X27 is selected from the group consisting of Leu and DLeu, or is absent;
- X28 is selected from the group consisting of Ser, DSer, Pro, DPro, Ser(Me) and DSer(Me), or is absent;
- X29 is selected from the group consisting of Ser, DSer, Pro, DPro, Ser(Me) and DSer(Me), or is absent;
- X30 is selected from the group consisting of Thr and DThr, or is absent;
- X31 is selected from the group consisting of Asn, DAsn, Glu, DGlu, Asp and DAsp, or is absent;
- X35 is selected from the group consisting of His, DHis, Asn, DAsn, Glu, DGlu, Asp and DAsp, or is absent;
- X37 is selected from the group consisting of Tyr, DTyr, Pro, DPro, Hyp, Phe, Ser, Gly, Ala, Val, Ile, Leu, Thr, Tyr(Me), Gly(Me), Ala(Me), Ile(Me), Aze, Pip, Apr, Php and Bep, or is absent;
and wherein the parentheses () associated with the two cysteine (C, Cys) residues at sequence positions 2 and 7 indicate the presence of an intramolecular disulfide bridge between the two Cys residues in question;
and,
Formula D1 is the following amino acid sequence as set forth in (SEQ ID NO:6): Arg-Cys-X3-Thr-Ala-Thr-Cys-Ala-Thr-X10-Arg-Leu-Ala-X14-Phe-Leu-X17-Arg-X19-X20-Phe-Gly(Me)-Ala-Ile(Me)-Leu-Ser-Ser-Thr-X31-Val-Gly-Ser-X35-Thr-X37 (SEQ ID NO: 6) wherein X represents an amino acid and
wherein
- X3 is selected from the group consisting of Asn, Gly, Pro and Gln;
- X10 is selected from the group consisting of Gln, Asp and Glu;
- X14 is selected from the group consisting of Asp, His, Asn and Aad;
- X17 is selected from the group consisting of His, Asn, Gln, Glu, Thr, Val, Lys and Aad;
- X19-X20 is selected from the group consisting of Ser-Ser, Val-Val, Ser-Val and Val-Ser, or is absent;
- X31 is selected from the group consisting of Asp, Glu and Asn;
- X35 is selected from the group consisting of Asp, Glu, Asn, Ser, Phe, Orn, Aad, Gly and Thr; and
- X37 is selected from the group consisting of Pro, Apr and Hyp;
and wherein the compound has at least one residue selected from:
- X3 is Gln;
- X14 is His, Asn or Aad;
- X17 is Asn, Gln, Glu, Thr or Aad;
- X19-X20 is Val-Ser or Ser-Val; and
- X35 is Ser, Phe, Orn, Aad, Gly or Thr;
and,
Formula E1 is the following amino acid sequence as set forth in (SEQ ID NO:7): X1-X2-X3-X4-X5-X6-X7-Ala-Thr-X10-Arg-Leu-Ala-X14-Phe-Leu-X17-Arg-X19-X20-Phe- Gly(Me)-Ala-Ile(Me)-X27-Ser-Ser-Thr-Glu-X32-Gly-Ser-X35-Thr-X37 (SEQ ID NO: 7) wherein X represents an amino acid and
wherein
- X1 is selected from the group consisting of Arg, Lys and Glu;
- X3 is selected from the group consisting of Gly, Gln and Pro;
- X4 is selected from the group consisting of Thr and Glu;
- X5 is selected from the group consisting of Ala and Leu;
- X6 is selected from the group consisting of Thr and Ser;
- X10 is selected from the group consisting of Glu and Gln;
- X14 is selected from the group consisting of Aad, His, Asp, Asn and Arg;
- X17 is selected from the group consisting of Gln, His and Thr;
- X19-X20 is selected from Ser-Ser, Thr-Thr, Ala-Thr, Ala-Ala, Gly-Thr, Gly-Gly and Ala-Asn or is absent;
- X27 is selected from the group consisting of Leu and Pro;
- X32 is selected from the group consisting of Val and Thr;
- X35 is selected from the group consisting of Asn and Ser;
- X37 is selected from the group consisting of Hyp and Pro; and
X2 and X7 are amino acid residues whose side chains together form a lactam bridge; or a pharmaceutically acceptable salt or solvate thereof.

13. Composition according to any of the claims 1 to 12, wherein short-acting glucagon RA is chosen amongst human glucagon and dasiglucagon.

14. Composition according to any of claims 1 to 13, wherein it further comprises a short-acting Glucose dependant Insulinotropic Polypeptide ("GIP" also known as Gastric Inhibitoy Polypeptide) Polypeptide RA.
Composition according to any of claims 1 to 14, wherein it further comprises a a short-acting Peptide Tyrosine Tyrosine (or "PYY") analog.

15. Composition according to any of claims 1 to 15, wherein it further comprises a copolyamino acid bearing carboxylate charges and hydrophobic radicals Hy.

16. Composition according to any of claims 1 to 15, for use in a method of treating obesity, so as to diminish the food intake, **characterised in that** the composition is administered in a therapeutical scheme that comprises modulation of the delivered dose.

17. Composition according to any of claims 1 to 15, for use in a method of treating obesity, so as to diminish the food intake or increase energy expenditure, **characterised in that** the composition is administered in a therapeutical scheme that comprises stop and restart periods.

18. Composition according to any of claims 1 to 15, for use in a method of treating obesity, so as to diminish the food intake or increase energy expenditure, **characterised in that** the composition is administered in a therapeutical scheme that comprises modulation periods that allow a modulation of the rate of delivery intraday.

19. Administering device comprising:
- a composition (1) comprising at least two short-acting peptides chosen in at least two of the following groups, GLP-1 RA, Amylin RA and Glucagon RA, according to any of the preceding claims,
- an administering apparatus (2) for delivering the composition (1) to a patient, said administering apparatus (2) including:
- a reservoir (22) containing the composition (1),
- a drive mechanism (23) for pumping the composition (1) contained in the reservoir (22),
- a control unit (24) for controlling the drive mechanism (23) according to an injection profile defining at least one rate for the administering of the composition (1) to the patient,
- an injection set (26) configured to be attached to an infusion site on the patient for the subcutaneous administering of the composition (1) to the patient.
